# EUROPEAN PATENT SPECIFICATION

(11) **EP 4 380 964 B1**
(45) Date of publication and mention of the grant of the patent: **11.11.2026**
(21) Application number: 23732887.7
(22) Date of filing: 12.06.2023
(51) Int. Cl.: C07K 14/705, C07K 14/47, A61P 35/00, A61K 39/00, A61K 40/19, A61K 40/30, A61K 40/33

(54) **ENGINEERED IMMUNE CELLS**
GENTECHNISCH HERGESTELLTE IMMUNZELLEN
CELLULES IMMUNITAIRES MODIFIÉES

(30) Priority: 13.06.2022 GB 202208605
(43) Date of publication of application: 12.06.2024
(73) Proprietor: UNIKUM Therapeutics ApS, N 2200 København (DK)
(72) Inventor: BAK, Rasmus Otkjær, N 2200 København (DK); LAUSTSEN, Anders, N 2200 København (DK)
(74) Representative: J A Kemp LLP
(86) International application number: PCT/EP2023/065690
(87) International publication number: WO 2023/242138

(56) References cited:
- EP-A1- 3 741 861
- WO-A1-2020/028743
- WO-A1-2022/063818
- XIA MAO ET AL: "CXCL10 encoding synNotch T cells enhance anti-tumor immune responses without systemic side effect", BIOCHEMICAL AND BIOPHYSICAL RESEARCH COMMUNICATIONS, ELSEVIER, AMSTERDAM NL, vol. 534, 17 November 2020 (2020-11-17), pages 765 - 772, XP086429274, ISSN: 0006-291X, [retrieved on 20201117], DOI: 10.1016/J.BBRC.2020.11.002
- ANPING LI ET AL: "Activating cGAS-STING pathway for the optimal effect of cancer immunotherapy", JOURNAL OF HEMATOLOGY & ONCOLOGY, vol. 12, no. 1, 1 April 2019 (2019-04-01), XP055730404, DOI: 10.1186/s13045-019-0721-x

## Description

### Field of the Invention

The present invention relates to engineered immune cells and their use in methods for treating disease, in particular cancer and autoimmune, inflammatory and infectious diseases.

### Background of the Invention

Immune cells expressing synthetic receptors represent an established and successful approach for treating various diseases, including cancer, autoimmune diseases, inflammatory diseases, and infections. An example of such a cell therapy is chimeric antigen receptor T-cell (CAR-T) therapy. Other cells have been proposed for use with CARs. Natural killer cells are a type of cytotoxic lymphocyte that naturally attack virus-infected cells and tumour cells and can be engineered with CARs to form CAR-NK cells. WO2017019848 describes use of other phagocytic cells capable of cellular cytotoxicity, in particular macrophages.

In CAR-T therapy, a T-cell population is obtained from a patient or donor and is engineered to express a chimeric antigen receptor (CAR). The extracellular domain of a typical CAR consists of the VH and VL domains - single-chain fragment variable (scFv) - from the antigen binding sites of a monoclonal antibody that recognises a tumour associated antigen. The scFv is linked to a flexible transmembrane domain followed by an intracellular signalling domain with, for example, a tyrosine-based activation motif such as that from CD3z, and optionally additional activation domains from co-stimulatory molecules such as CD28 and CD137 (41BB), which serve to activate the T-cells and enhance their survival and proliferation. CAR T-cells are administered to the patient and the CAR recognises and binds tumour cells or other disease-causing cells. Binding of cancer cells leads to activation of cytolytic mechanisms in the T-cells, which specifically kill the bound target cells. Various preclinical and early-phase clinical trials highlight the efficacy of CAR T cells to treat cancer patients with solid tumours and hematopoietic malignancies, and to treat autoimmune diseases, inflammatory diseases, and infections.

Other receptors are also used in a similar manner to CARs. For example, synthetic receptors, such as synthetic intramembrane proteolysis receptors (SNIPRs), can be expressed in cells to activate a response element in a cell upon recognition of a specific antigen. Zhu et al., 2022, Cell 185, 1431-1443 describes the design of synthetic receptors from modular domains including extracellular regulatory domains, transmembrane domains, intracellular juxtamembrane domains, and transcriptional regulators. Xia et al., 2020, Biochemical and Biophysical Research Communications, 534(17):765-772 describes a modified T cell carrying a synNotch-based receptor system that can selectively lead to CXCL10 expression.

There remains a need in the art for improved methods for treating cancer and other diseases.

### Summary of the Invention

The inventors have developed an engineered immune cell, that utilises the versatility of synthetic receptors to target specific cells, in combination with a potent activator of immune responses - STING (stimulator of interferon genes), to enhance or alter immunological pathways, and treat disease. According to the invention, an immune cell is engineered to express on its surface a synthetic receptor comprising an extracellular antigen recognition domain that recognises an antigen on a target cell, a transmembrane domain, and an intracellular signalling domain that activates expression of a response element in the cell when the extracellular antigen recognition domain binds the target cell, wherein the cell comprises a heterologous nucleic acid comprising the response element, wherein the response element encodes a STING protein, and wherein the intracellular signalling domain is a transcription factor comprising a DNA binding domain and a transcriptional activation domain, and wherein the response element contains the cognate recognition element for the DNA binding domain, and
wherein the synthetic receptor is:
(a) a synthetic intramembrane proteolysis receptor (SNIPR), such as wherein the synthetic receptor is a synthetic RIP family receptor, optionally wherein the receptor is:
   (i) synthetic Notch receptor;
   (ii) a synthetic Robo receptor; or
   (iii) a synthetic RoboNotch receptor;
(b) a Tango receptor; or
(c) a modular extracellular signalling architecture (MESA) system. The extracellular antigen recognition domain allows the cells to specifically target disease cells and tissue to activate immune responses and treat disease in the disease microenvironment. The response element encoding a STING protein potently activates a robust immune response in the immune cells to treat disease. In combination, the immune cells, the synthetic receptor and the response element encoding a STING protein provide a powerful system for targeted stimulation of immune responses. The receptor binds specific antigens on target cells, initiating the synthetic receptor cascade, activating transgene transcription and producing a STING protein, optionally with gain-of-function mutations, which activation mediates a potent immune response in the immune cell, including the production of pro-inflammatory cytokines and type I interferons, and recruiting and activating immune cells, such as cytotoxic lymphocytes, and the sensitization of cancer cells to immune recognition. Consequently, the cells of the invention are expected to exert powerful and pervasive effects, even in disease microenvironments that are refractory to the desired immune response.

The synthetic receptors of the invention are synthetic intramembrane proteolysis receptors (SNIPRs), Tango receptors or modular extracellular signalling architecture (MESA) systems. In some embodiments, the synthetic receptors is a synthetic RIP family receptor, such as a synthetic Notch receptor (SynNotch receptor), a synthetic Robo receptor (SynRobo receptor), or a synthetic RoboNotch receptor (SynRobo receptor). In a preferred embodiment, the synthetic receptor is a SynNotch receptor. The Examples demonstrate that pDCs can express SynNotch components and can specifically bind target cells. Strikingly, activation of STING via the SynNotch response element successfully induces a type I IFN and CXCL10 response in the pDCs of the invention, but the pDCs maintain their unique characteristics with no effect on differentiation or function. The system is expected to also activate a potent and targeted immune response in other immune cells, which are also capable of expressing synthetic receptors and are capable of a STING-mediated immune response. See, for example, Su, Ting et al., 2019, Theranostics vol. 9,25 7759-7771, which demonstrates that STING agonists promote an immune response in T cells. The system is also expected to work using synthetic receptors with different transmembrane domains, e.g. Robo1, as these have similar functions to Notch1.

In some embodiments, the cell is a lymphoid cell. In some embodiments, the cell is a myeloid cell, such as a macrophage. In some embodiments, the cell is a natural killer (NK) cell, a T cell, a B cell, or a plasmacytoid dendritic cell (pDC). In a preferred embodiment, the cell is a plasmacytoid dendritic cell (pDC).

In some embodiments, the STING response element comprises a gain-of-function mutation that causes it to be constitutively active in the absence of 2'3'-cGAMP. In some embodiments, the STING protein comprises a mutation, preferably a substitution, at one or more residues corresponding to R71, S102, V147, N154, V155, G166, C206, G207, G230, H232, R238, F279, R281, R284, R293 or Q315 of SEQ ID NO:1. In some embodiments, the STING response element comprises a mutation at the residue corresponding to N154 or V155 in SEQ ID NO:1. In preferred embodiments, the STING response element comprises an N154S mutation or a V155M mutation. These gain-of-function mutations result in constitutive activation of immune responses by STING, including production of type I interferons and CXCL10 in response to expression of the STING protein caused by binding of an antigen to the extracellular antigen recognition domain, as shown in the Examples.

The invention also provides a cell of the invention, for use in a method of treating a disease in a subject comprising administering the cell of the invention.

In preferred embodiments, the cells of the invention are for use in treating cancer, an autoimmune, an inflammatory disease, or an infectious disease. The ability of immune cells such as pDCs to secrete immunomodulatory factors and alter immune-inhibiting or inflammatory environments are expected to be particularly useful for treating such diseases, where disease-causing cells reside in and are maintained by particular immune microenvironments. Also, cancer cells, autoreactive T-cells, inflammatory T-cells, and infectious pathogens present specific antigens that can be recognised by the extracellular antigen recognition domain to provide targeted therapy. In addition, the potent immune response activated by the STING response element is expected to provide therapeutic effects against such cells and pathogens.

In some embodiments, the STING protein activates an immune response. In preferred embodiments, the immune response comprises secretion of cytokines, such as pro-inflammatory cytokines.

In further preferred embodiments, the immune response comprises recruitment or activation of host immune cells, such as cytotoxic lymphocytes.

A pDC of the invention may also perform direct TRAIL-mediated killing of target cells.

In preferred embodiments, the pDC is a stem cell-derived plasmacytoid dendritic cell (SC-pDC). Such cells can be generated ex vivo from hematopoietic stem and progenitor cells (HSPCs), which can be obtained from a patient or a donor. Moreover, HSPCs can readily be generated from induced pluripotent stem cells (iPSCs), allowing for iPSC-derived SC-pDCs. The HSPCs and iPSCs may be engineered with the SynNotch receptor before differentiation to SC-pDCs.

In especially preferred embodiments, the invention provides a cell of the invention, preferably an engineered pDC, for use in a method of treating cancer in a subject comprising administering the engineered immune cell to the subject, wherein the cell preferably expresses on its surface a SynNotch receptor, wherein the response element encodes a stimulator of interferon genes (STING) protein comprising a gain-of-function mutation that causes it to be constitutively active in the absence of 2'3'-cGAMP, such as a mutation at N154 or V155, preferably an N154S mutation or a V155M mutation.

In further especially preferred embodiments, the invention provides a cell of the invention, preferably an engineered pDC, for use in a method of treating an autoimmune disease in a subject comprising administering the engineered immune cell to the subject, wherein the cell preferably expresses on its surface a SynNotch receptor, wherein the response element encodes a stimulator of interferon genes (STING) protein comprising a gain-of-function mutation that causes it to be constitutively active in the absence of 2'3'-cGAMP, such as a mutation at N154 or V155, preferably an N154S mutation or a V155M mutation.

In further especially preferred embodiments, the invention provides a cell of the invention, preferably an engineered pDC, for use in a method of treating an inflammatory disease in a subject comprising administering the engineered immune cell to the subject, wherein the cell preferably expresses on its surface a SynNotch receptor, wherein the response element encodes a stimulator of interferon genes (STING) protein comprising a gain-of-function mutation that causes it to be constitutively active in the absence of 2'3'-cGAMP, such as a mutation at N154 or V155, preferably an N154S mutation or a V155M mutation.

In further especially preferred embodiments, the invention provides a cell of the invention, preferably an engineered pDC, for use in a method of treating an infectious disease in a subject comprising administering the engineered immune cell to the subject, wherein the cell preferably expresses on its surface a SynNotch receptor, wherein the response element encodes a stimulator of interferon genes (STING) protein comprising a gain-of-function mutation that causes it to be constitutively active in the absence of 2'3'-cGAMP, such as a mutation at N154 or V155, preferably an N154S mutation or a V155M mutation.

The invention also provides the engineered immune cells, preferably engineered pDCs, of the invention for use in treating disease, such as for use in treating cancer or an inflammatory, autoimmune or infectious disease.

The invention also provides use of the engineered immune cells, preferably engineered pDCs, of the invention in the preparation of a medicament for treating disease, such as a medicament for treating cancer or an inflammatory, autoimmune or infectious disease.

In certain embodiments, the methods of the invention further comprise a step of producing the pDCs, wherein the step of producing the pDCs comprises the steps of:
(a) transfecting or transducing HSPCs with one or more vectors comprising the synthetic receptor and the response element; and
(b) differentiating the HSPCs into pDCs.

In certain embodiments, the methods of the invention further comprise a step of producing the pDCs, wherein the step of producing the pDCs comprises the steps of:
(a) differentiating HSPCs into pDCs; and
(b) transfecting or transducing the pDCs with one or more vectors comprising the synthetic receptor and the response element.

### Brief Description of the Figures

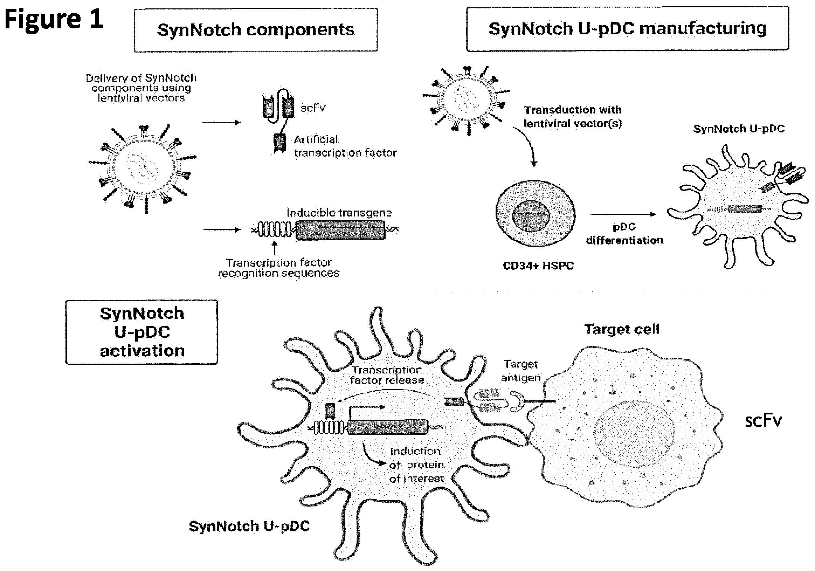

**Figure 1****.** Schematic illustration showing the production of SynNotch U-pDCs expressing the SynNotch elements. "U-pDC" is an alternative term that is also used throughout to refer to pDCs. HSPCs were pre-expanded for three days before being transduced with SynNotch constructs using lentiviral vectors carrying the SynNotch receptor and the response element. In some cases, the lentiviral vector encodes both the SynNotch receptor and response element in one cassette and in other cases the two elements are encoded by separate vectors. After the transduction, HSPCs were subsequently differentiated into U-pDCs over 16 days of culture.

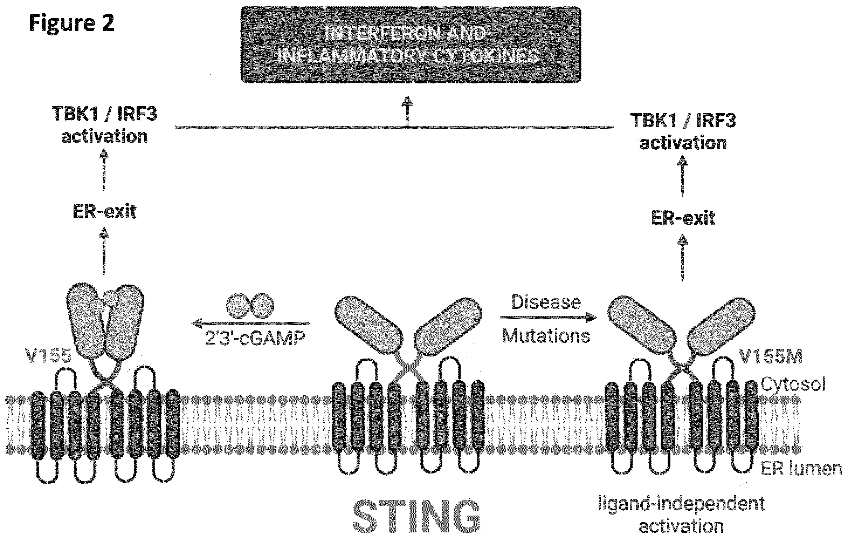

**Figure 2****.** Illustration of innate immune signaling of the STING V155M gain of function. Normal STING signaling relies on the activation of STING based on the binding of the ligand 2'3'-cGAMP. The STING V155M harbors a substitution of valine at position 155 to that of methionine, which enhances stability of the dimer and/or mimics the effect of ligand binding, which provides ligand-independent/constitutive activation of STING signaling. The STING N154S harbors a substitution of asparagine at position 154 to that of serine, which increases the stability of the dimer, resulting in a constitutive active protein.

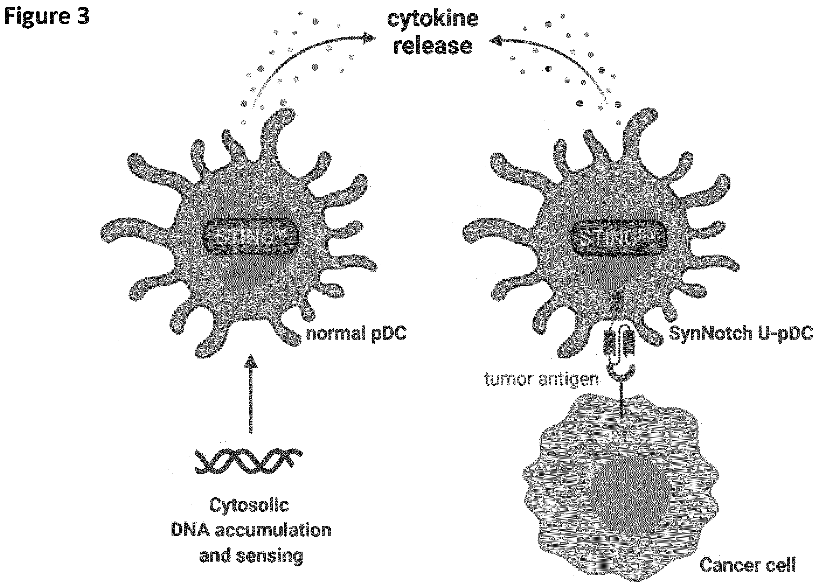

**Figure 3****.** STING signaling in normal pDCs versus the SynNotch U-pDCs carrying the STING V155M gain of function (GoF). STING signaling in unmodified pDCs relies on the activation of STING by cytosolic sensing of accumulated DNA. In comparison, binding of a tumor antigen to the SynNotch receptor to the SynNotch U-pDCs results in the transcription of the STING GoF variant, resulting in a STING-mediated cytokine response without the use of a STING agonist.

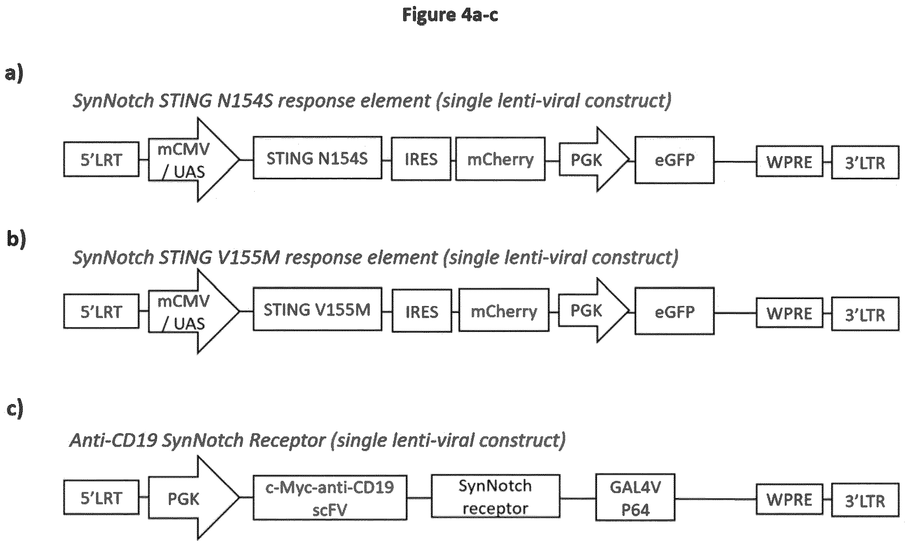

**Figure 4****.** Schematic illustration showing SynNotch STING constructs (the two elements split between two LV vectors). a-b) Schematic of a SynNotch response element in a 3rd gen. lentiviral construct. The constructs contain a STING N154S (a) or STING V155M (b) response element under the control of an inducible miniCMV promotor (mCMV) flanked by GAL4-binding UAS sequences. Connected to the response element is an mCherry gene through an IRES domain. Downstream of this is an eGFP transgene constitutively expressed by a PGK promotor. In U-pDCs the construct will allow expression of the STING gain of function variants only when the SynNotch receptor binds to a specific tumor antigen. c) Schematic illustration of a SynNotch receptor in a 3rd gen. lentiviral construct. The construct contains a SynNotch receptor with an anti-CD19 scFv on the extracellular side fused to the GAL4-VP64 transcription factor on the intracellular side. Upstream of the anti-CD19 scFv is a c-Myc tag to allow easy validation of construct expression and activation of the receptor using anti-c-Myc beads. The SynNotch receptor is constitutively expressed by a PGK promotor. In U-pDCs the construct will allow expression of the anti-CD19 scFv SynNotch receptor, which upon binding to CD19 on a target cell will facilitate transcription factor (GAL4-VP64) release, which induces the expression of the aforementioned response element.

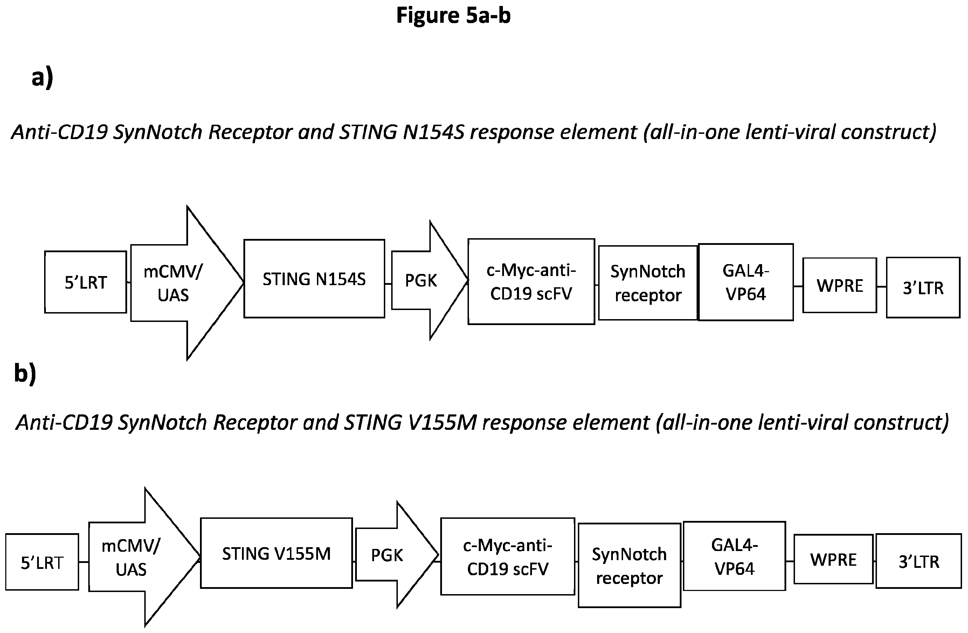

**Figure 5****.** Schematic illustration showing SynNotch STING constructs (the two elements carried by a single LV vector). a-b) Schematic of a SynNotch receptor and response element in a 3rd gen. lentiviral construct. The constructs contain a STING N154S (a) or STING V155M (b) response elements under the control of a mCMV promotor flanked by GAL4-binding UAS sequences. Downstream of this inducible transgene, a PGK promotor expresses an anti-CD19 scFv SynNotch-GAL4-VP64 receptor. Upstream of the anti-CD19 scFv is a c-Myc tag to allow easy validation of construct expression and activation of the receptor using anti-c-Myc beads. Upon transduction the construct allows a full SynNotch system to be delivered to U-pDCs by an 'all-in-one' lentiviral vector, which enables U-pDCs to express the STING gain of function variants upon binding to CD19 antigen on target cells.

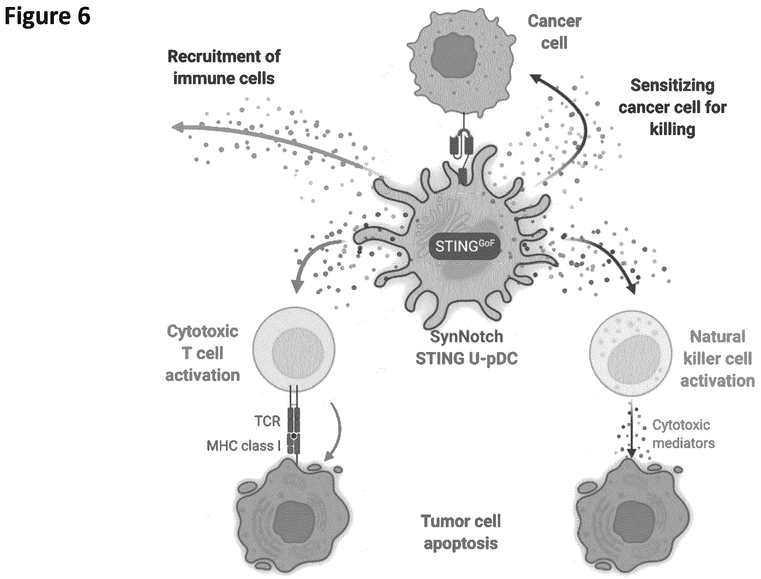

**Figure 6****.** Immune responses of activated SynNotch STING GoF U-pDCs. Binding of SynNotch STING GoF U-pDCs to a specific tumor antigen will initiate transcription of the STING GoF variant. The variant will in turn initiate a broad transcriptional program in the SynNotch U-pDCs, resulting in the production of type I IFN and pro-inflammatory proteins. These will sensitize the cancer cells to immune-mediated killing, prime surrounding bystander immune cells, such as cytotoxic lymphocytes (NK cells and CD8 T cells), and recruit other immune cells to the tumor site. Overall, this will promote specific anti-tumor responses in the tumor.

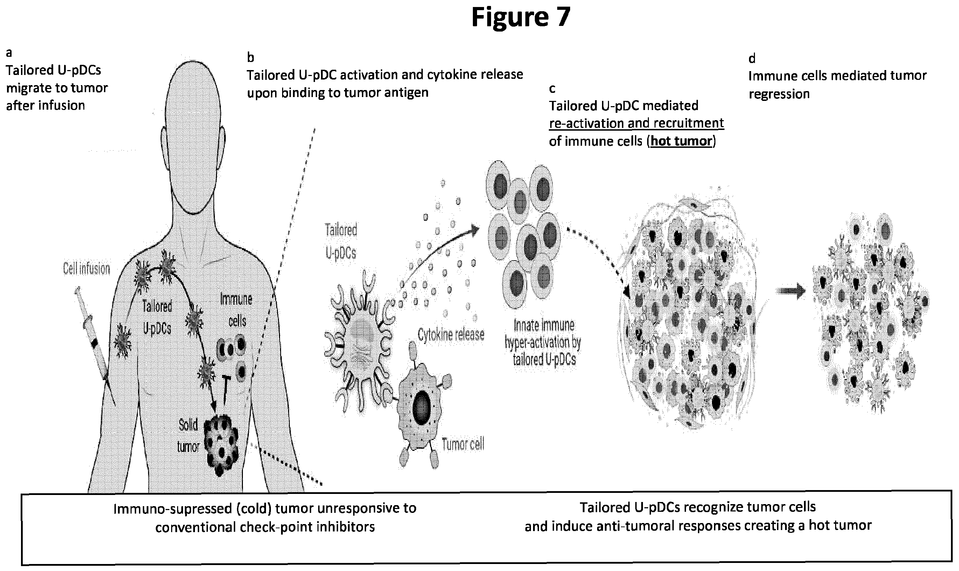

**Figure 7****.** Infusion of SynNotch STING GoF U-pDCs into a cancer patient. U-pDCs expressing the SynNotch STING GoF are injected into a patient. The U-pDCs will home to the tumor site and upon recognition of the specific tumor antigen will become activated and secrete type I IFN and pro-inflammatory cytokines. This will in turn promote recruitment of immune cells to the tumor site, such as cytotoxic lymphocytes (NK cells and CD8 T cells). Moreover, the pro-inflammatory environment will abolish the immune-inhibiting environment that the tumor cells generate, sensitizing the tumor cells, and transforming a 'cold' tumor into a 'hot' tumor to promote anti-tumor responses.

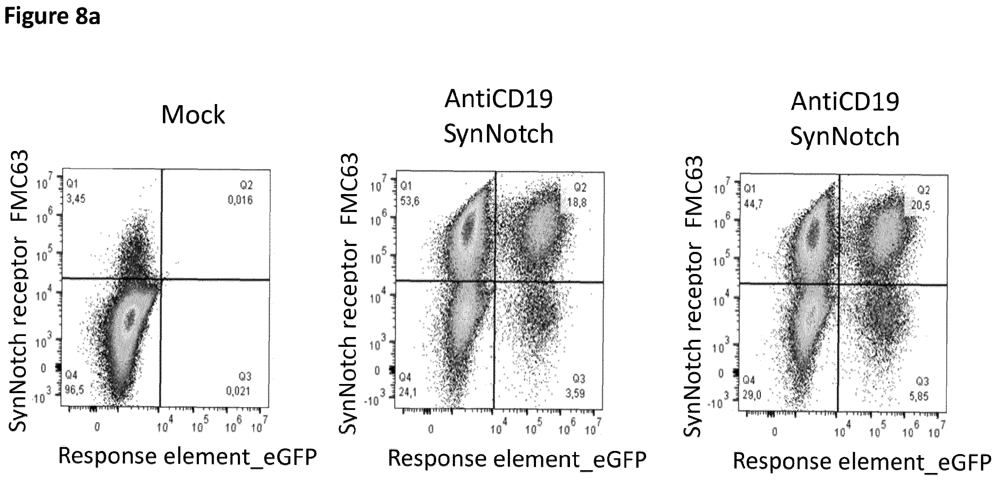

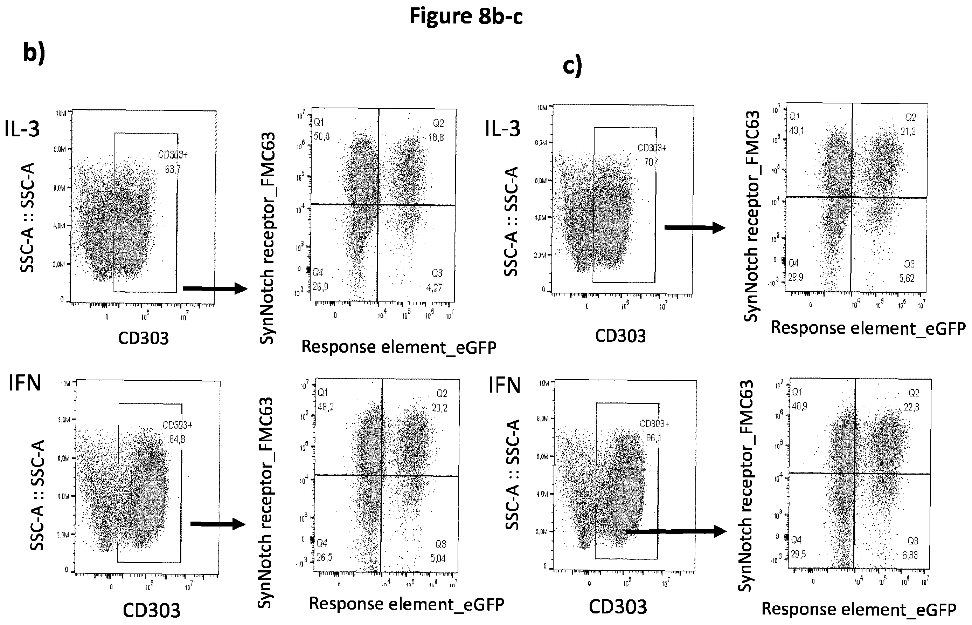

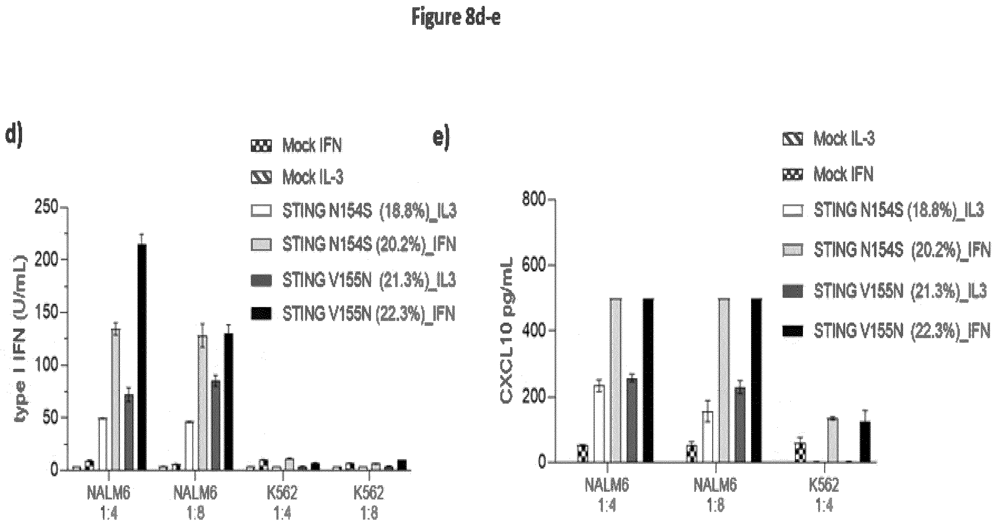

**Figure 8****.** U-pDCs expressing the SynNotch STING variants N154S or V155M effectively respond to target cells with a STING-mediated response. a) FACS plots showing LV transduction efficiency measured 6 days post transduction using flow cytometry. Here, the two components of the SynNotch system (receptor and response element) are delivered by two separate LVs. The level of antiCD19 SynNotch receptor and the SynNotch response elements were evaluated using an anti-FMC63 antibody and eGFP, respectively. b-c) Transduction efficiency measured 1-day after priming of U-pDCs (day3+16+1) on SynNotch U-pDCs either expressing antiCD19 SynNotch STING N154S (b) or antiCD19 SynNotch STING V155M (c). The level of antiCD19 SynNotch receptor and the SynNotch response elements were evaluated using an anti-FMC63 antibody and eGFP, respectively. Cells were gated based on viable cells, CD11c negative and CD303 positive. d) 5e5 non-primed (IL-3) or primed (IFN) SynNotch U-pDCs were seeded in 48-well plates and overlayed with 2e6 (1:4) or 4e6 (1:8) target cells (NALM6) or non-target cells (K562). Supernatants were harvested 20 hours later and evaluated for levels of type I IFN. e) 5e5 non-primed (IL-3) SynNotch U-pDCs were seeded in 48-well plates and overlayed with 2e6 (1:4) or 4e6 (1:8) target cells (NALM6) or non-target cells (K562). Supernatants were harvested 20 hours later and evaluated for levels of CXCL10 using a CXCL10 ELISA. Data shown are from one donor in biological triplicates.

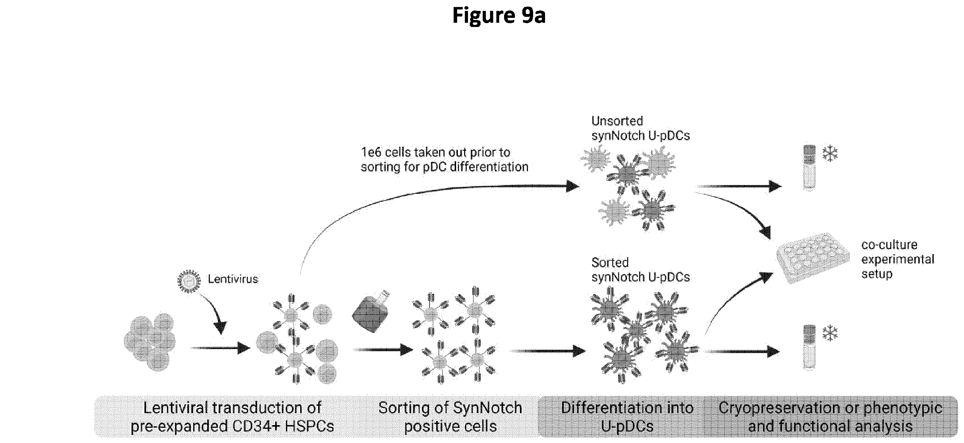

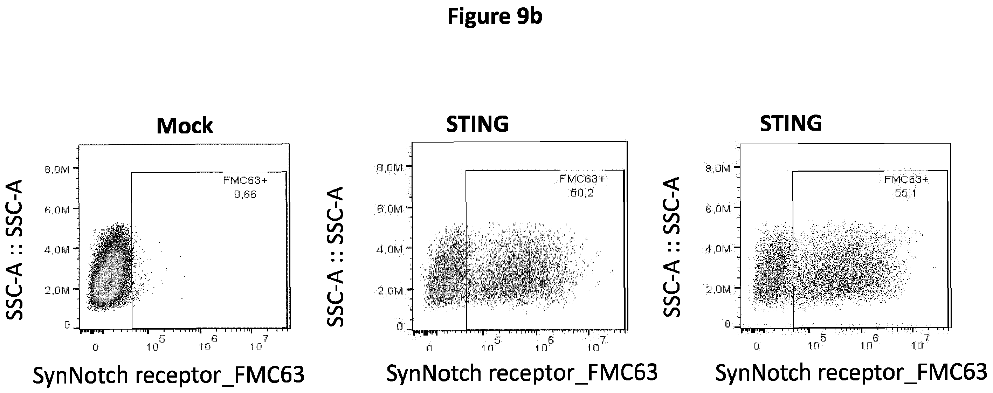

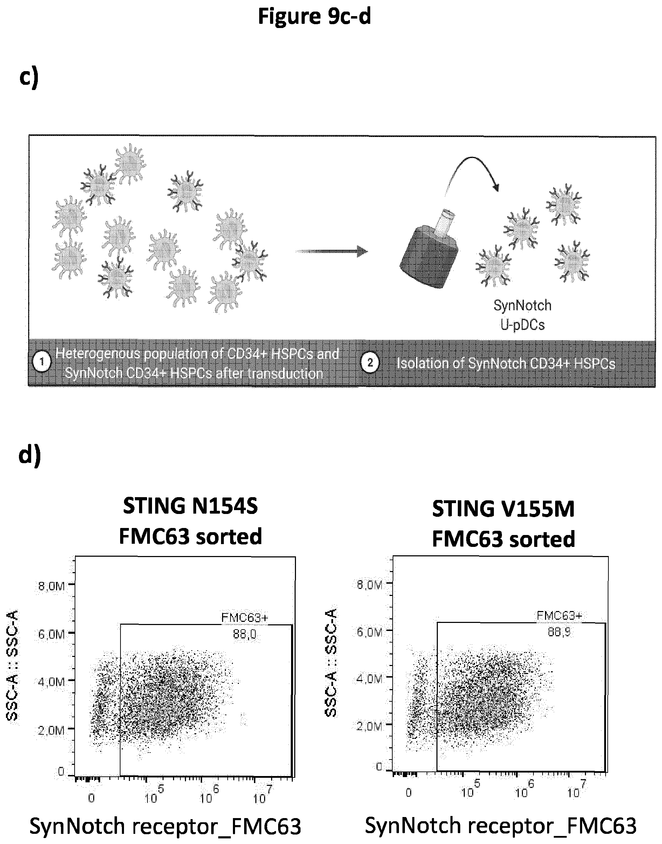

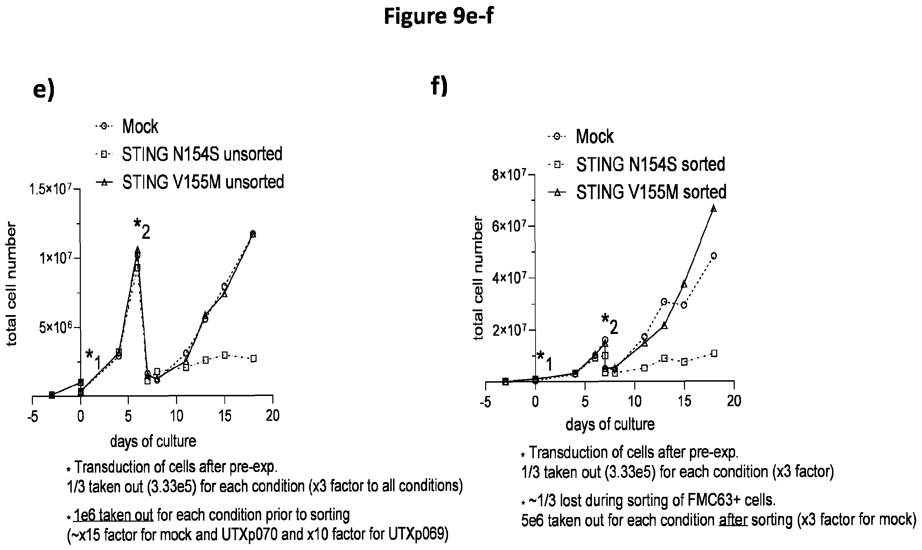

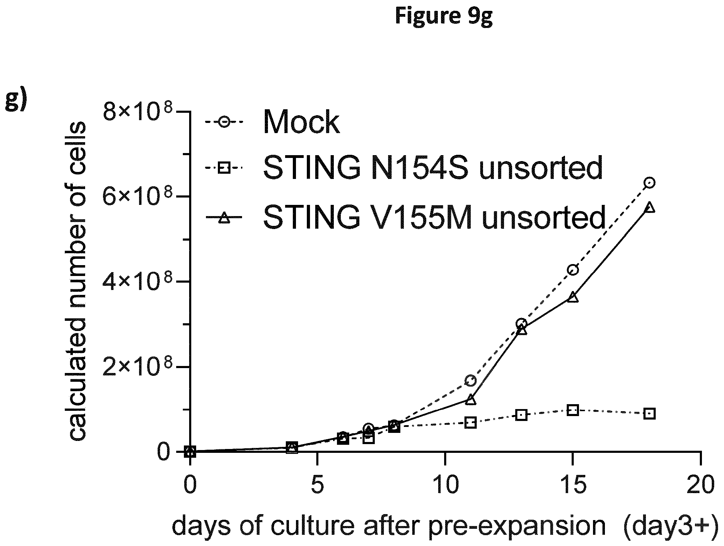

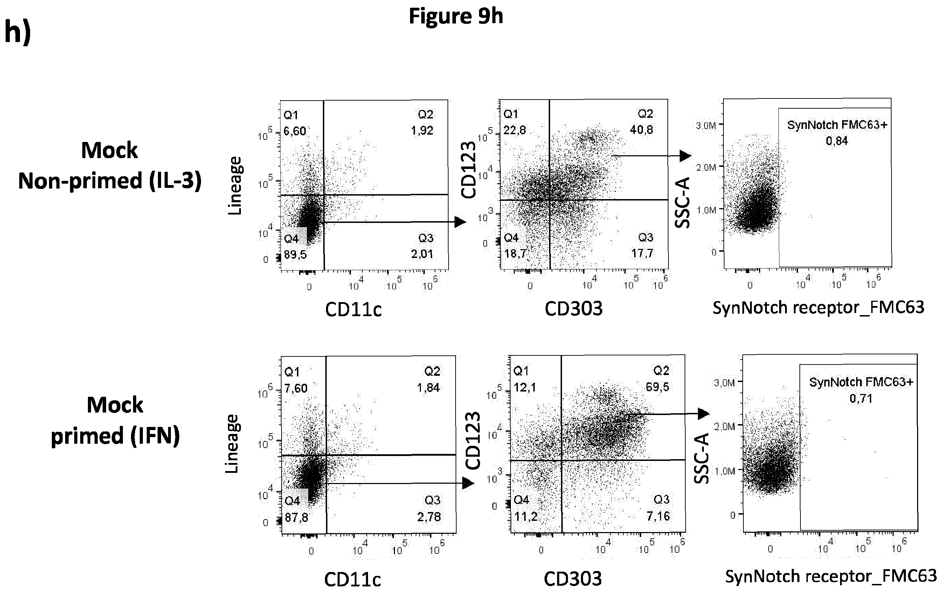

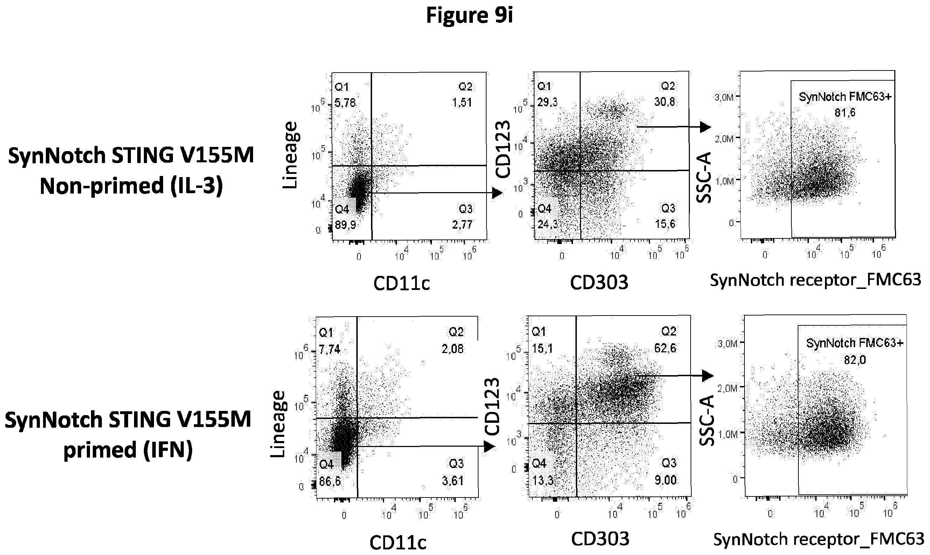

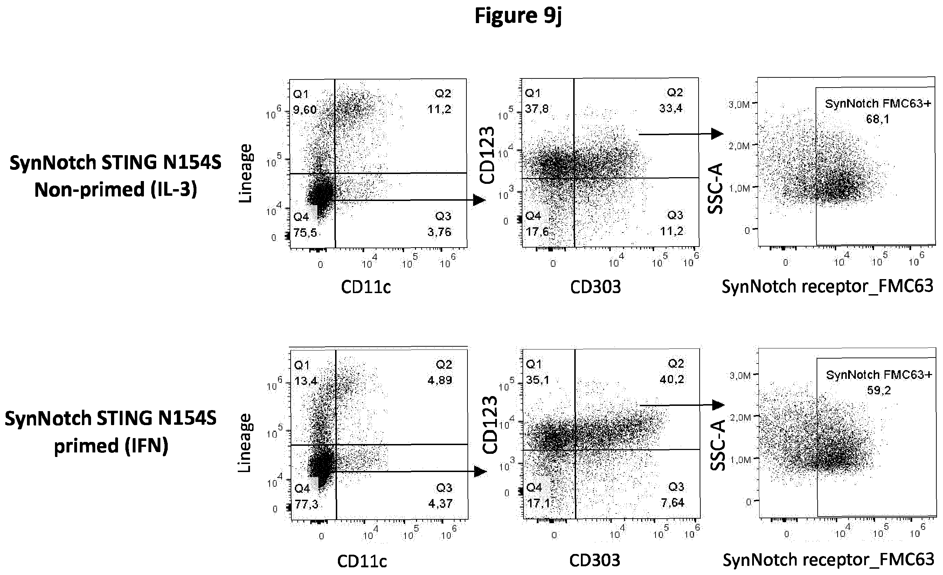

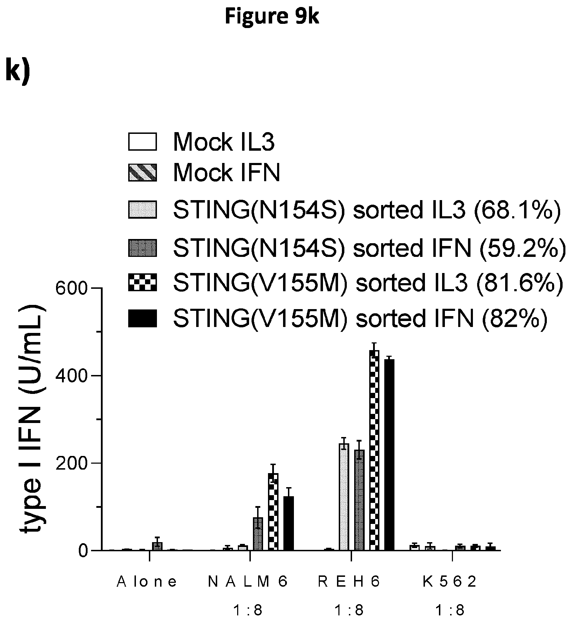

**Figure 9****.** U-pDCs expressing the SynNotch STING variants N154S or V155M effectively respond to target cells with a STING-mediated type I IFN response. a) Schematic showing experimental setup. HSPCs were initially thawed and pre-expanded at low density for 3-4 days before being transduced with lentiviral vectors at an MOI of 100. This experiment uses an 'all-in-one' lentiviral vector carrying either the STING N154S or the STING V155M response element along an SynNotch receptor targeting CD19. The day after transduction, medium was changed, and HSPCs were differentiated into U-pDCs. 6-days post transduction, transduction efficiency was analyzed. Cells were also sorted by labeling with antiFMC63-PE (FMC63=scFv) followed by positive immunomagnetic selection for PE. Prior to sorting, 1e6 HSPCs were taken out to analyze if the transduction, or the sorting of cells, affected pDC differentiation or expansion of cells. After 18-days of pDC differentiation, pDCs were taken out and primed for co-culture experiments or cryopreserved for later use. b) Transduction efficiency was measured at 6-days post transduction by flow cytometry. c) Figure illustrating immunomagnetic selection of antiCD19 SynNotch positive cells. HSPCs were stained with anti-FMC63-PE followed by positive immunomagnetic selection of PE positive cells. d) Purity of SynNotch positive cells after selection. e-f) Proliferation of HSPCs (actual numbers) during differentiation into U-pDCs. To estimate if transduction, expression of the STING construct, or sorting affected proliferation/viability/differentiation of HSPCs into U-pDCs, a similar number of mock cells were seeded prior to and after sorting of SynNotch positive cells. For unsorted cells, 1e6 cells were seeded, whereas for the sorted condition 5e6 cells were seeded. Below each graph, splitting conditions are stated. g) Similar to panel e) but calculated number of total cells assuming no cells were discarded during differentiation to U-pDCs. h-j) After 18 days of pDC differentiation, bulk U-pDCs were taken out and primed for one day with IFN, or left non-primed (IL-3). U-pDCs were subsequently evaluated for pDC markers (lineage negative, CD11c negative, CD123+, CD303+, and SynNotch receptor expression (FMC63)). The panels show primed (IFN) or non-primed (IL-3) for non-transduced (mock) U-pDCs (h), sorted SynNotch STING V155M U-pDCs (i), and sorted SynNotch STING N154S U-pDCs (j). k) 1e5 primed (IFN) or non-primed (IL-3) U-pDCs were seeded and overlayed with 8e5 target (NALM6, REH6) or non-target (K562) cells. Supernatants were collected 20 hours later, and levels of type I IFN were evaluated. Data shown are from one donor done in biological triplicates (k).

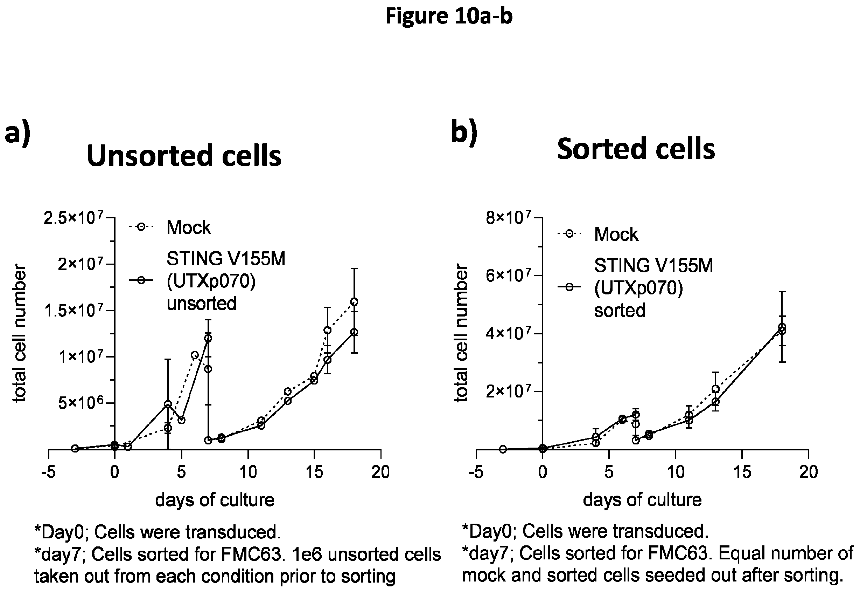

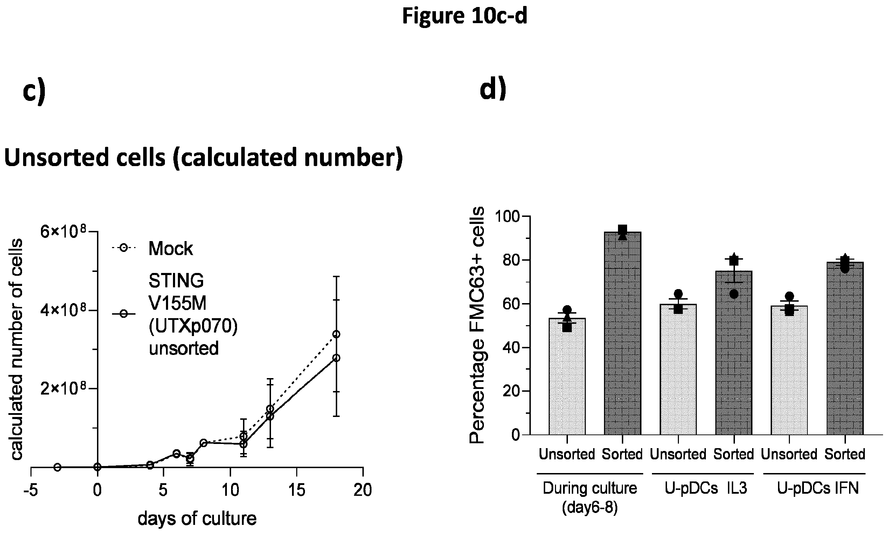

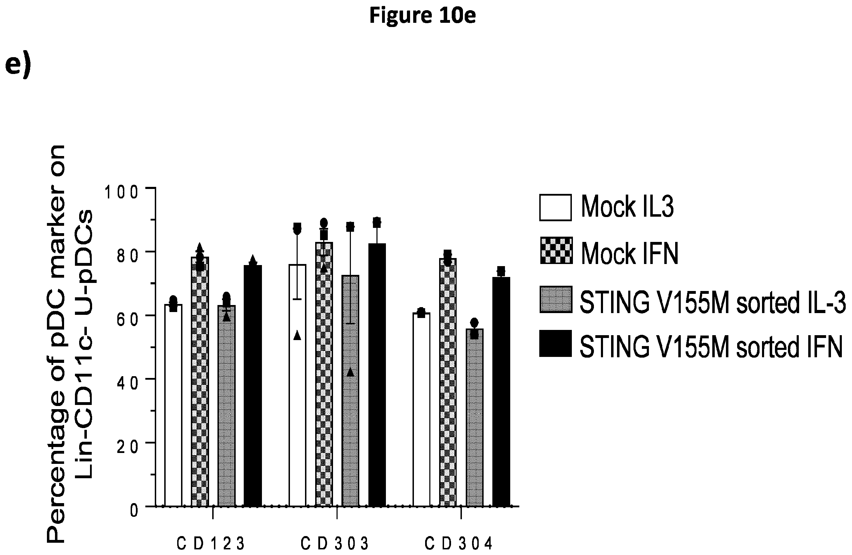

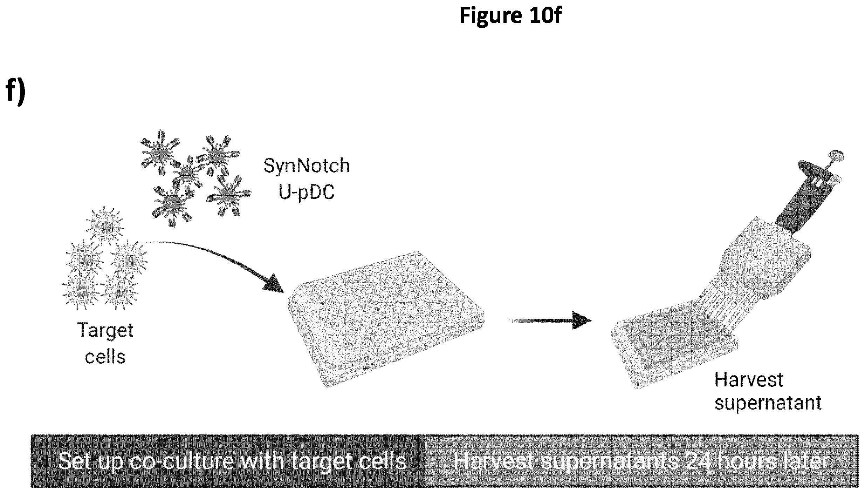

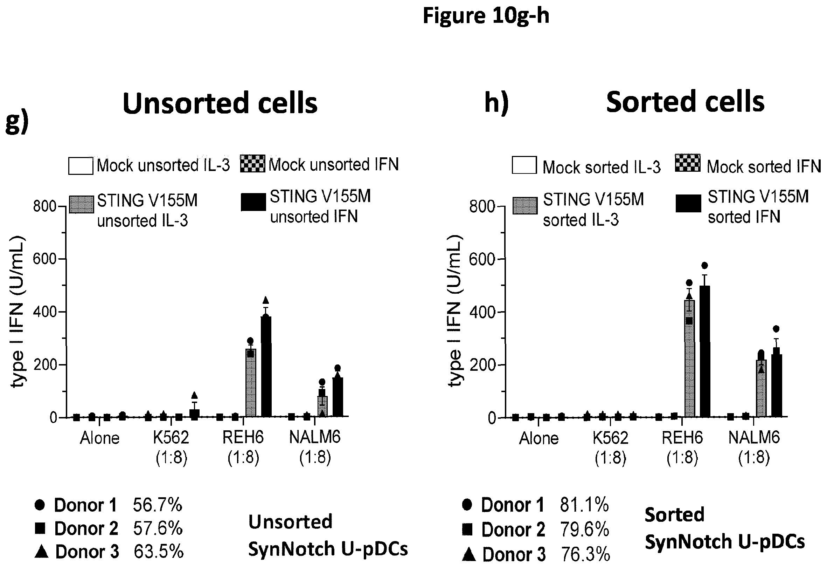

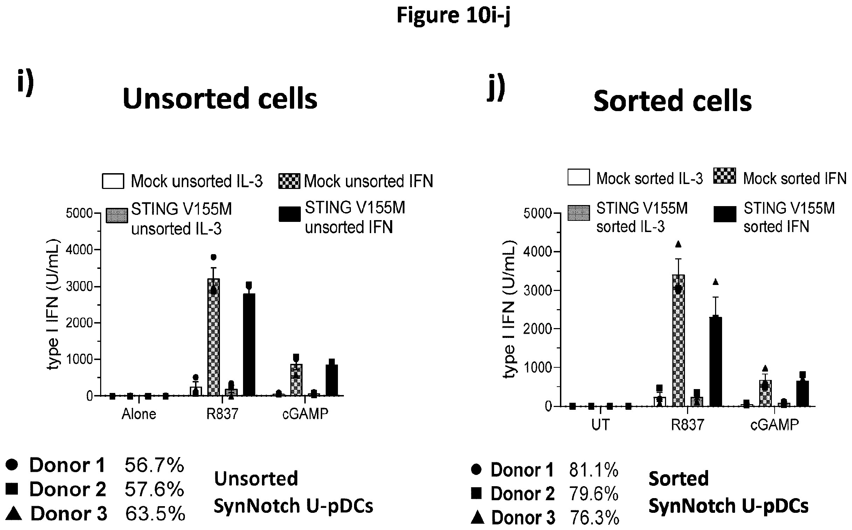

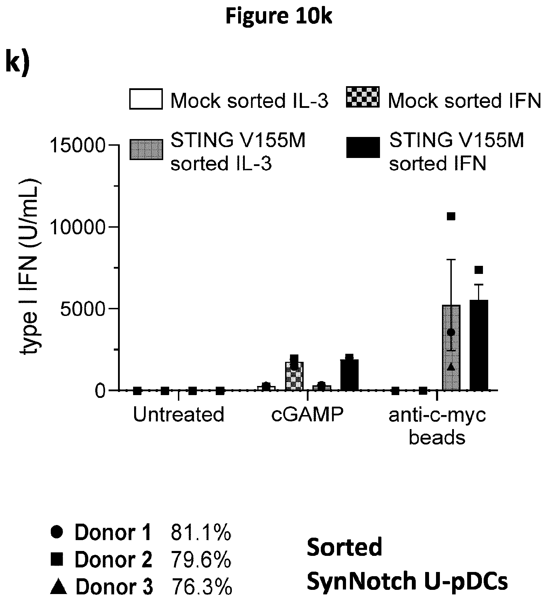

**Figure 10****.** U-pDCs expressing the SynNotch STING variant V155M effectively respond to target cells with a STING-mediated type I IFN response. HSPCs were initially thawed and pre-expanded at low density for 3-4 days before being transduced with an MOI of 100 of lentiviral vectors carrying the STING V155M SynNotch cassette. An all-in-one SynNotch construct (SynNotch receptor and response element in a single construct) was used. The day after transduction, medium was changed, and HSPCs were differentiated into U-pDCs. 6-days post transduction, transduction efficiency was analyzed. SynNotch-positive cells were also selected by labeling with antiFMC63-PE followed by immunomagnetic selection for PE. Prior to sorting, 1e6 HSPCs were taken out to analyze if the transduction, or the sorting of cells affected pDC differentiation or expansion of cells. After 18-days of pDC differentiation, pDCs were taken out and primed for co-culture experiments. a-b) Proliferation of HSPCs (actual numbers) during differentiation into U-pDCs. To estimate if transduction, expression of the STING construct, or sorting affected proliferation/viability/differentiation of HSPCs into U-pDCs, a similar number of mock cells were seeded prior to and after sorting of SynNotch positive cells. For unsorted cells, 1e6 cells were seeded, whereas for the sorted condition 5e6 cells were seeded. Below each graph, splitting conditions are stated. c) Similar to panel a) but calculated number of total cells assuming no cells were thrown out during differentiation to U-pDCs. d) Percentage of cells expressing the SynNotch receptor either 6-8 days post transduction, or after a full pDC differentiation (18 days) for non-primed (IL3) or primed (IFN) U-pDCs. e) Percentage of Lin-CD11c- U-pDCs expressing the pDC markers CD123, CD303, and CD304. f) Schematic overview showing the co-culture setup of SynNotch U-pDCs. 1e5 SynNotch U-pDCs or mock U-pDCs were seeded in a 96-well plate and overlayed with 8e5 target cells (E:T ratio of 1:8). 24 hours later, supernatants were collected and type I IFN levels were evaluated. g-h) Levels of type I IFN after co-culture with target cells (REH6 and NALM6) or non-target cells (K562) for unsorted SynNotch U-pDCs (g) or sorted SynNotch U-pDCs (h). Percentage of SynNotch positive U-pDCs for each donor is stated below each graph. I-h) 1e5 unsorted (i) or sorted (j) SynNotch U-pDCs were seeded and stimulated with the TLR7 agonist (R837, 2.5 µg/mL), or the STING agonist 2'-3'-cGAMP (4 µg/mL). Supernatants were collected 24 hours later and levels of type I IFN were evaluated using a type I IFN bioassay. k) 0.8-1e6 U-pDCs were seeded in a 24-well and stimulated with 2'3' cGAMP or anti-c-Myc magnetic beads that activate the SynNotch receptor. 24 hours later, supernatants were collected and type I IFN analyzed using a type I IFN bioassay. Data shown are from three donors performed in biological triplicates (g-k).

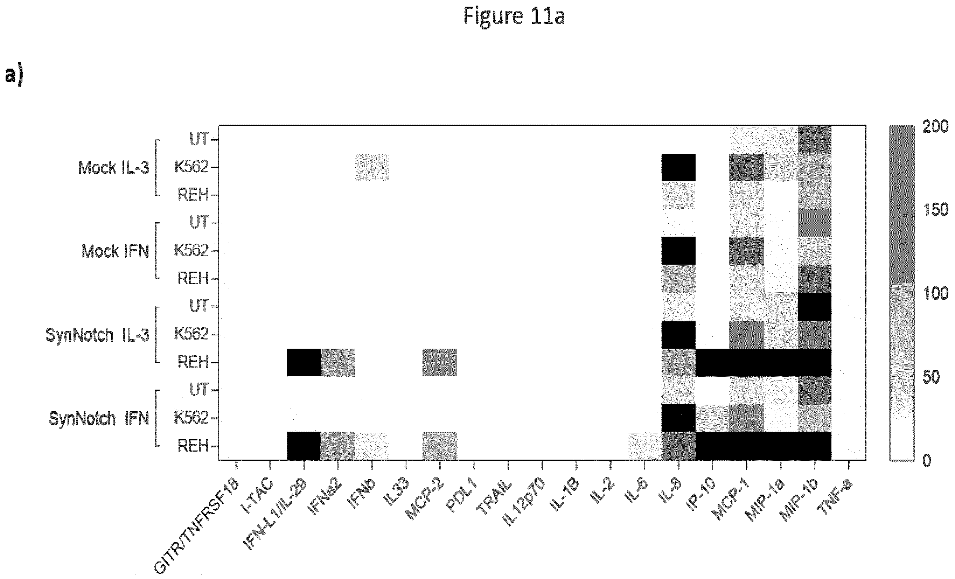

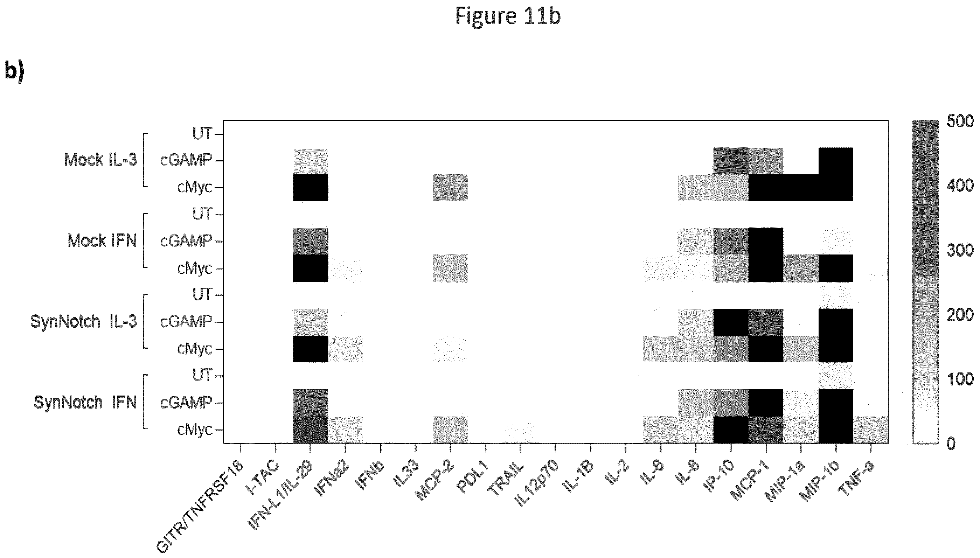

**Figure 11****.** U-pDCs expressing the SynNotch-STINGV155M induce a broad range of immunostimulatory cytokines and chemokines upon recognition of cognate antigen. a) 1e5 primed (IFN) or non-primed (IL-3) U-pDCs were seeded out and overlayed with 8e5 target (REH) or non-target (K562) cells (effector:target ratio of 1:8). Supernatants were collected 20 hours later and indicated cytokines/chemokines were evaluated using Meso-scale multiplex ELISA. Heat-map showing the compiled cytokine measurements defined in the range of pg/mL. Black squares indicate values above the maximum range detection. b) 1e6 primed (IFN) or non-primed (IL-3) U-pDCs were seeded out and stimulated with anti-c-Myc magnetic beads (15 µL per 1e6 cells), cGAMP (4 µg/mL), or left unstimulated (UT). Supernatants were collected 20 hours later and indicated cytokines/chemokines were evaluated using Meso-scale multiplex ELISA. Data are mean value +/- SEM of three donors.

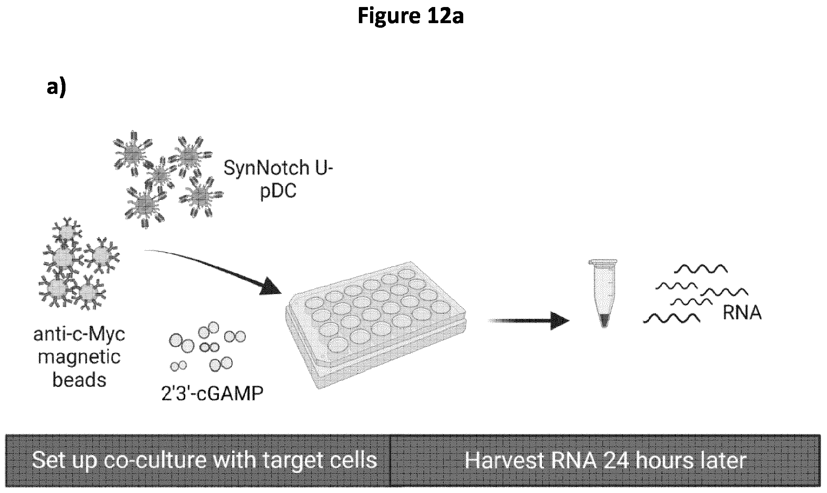

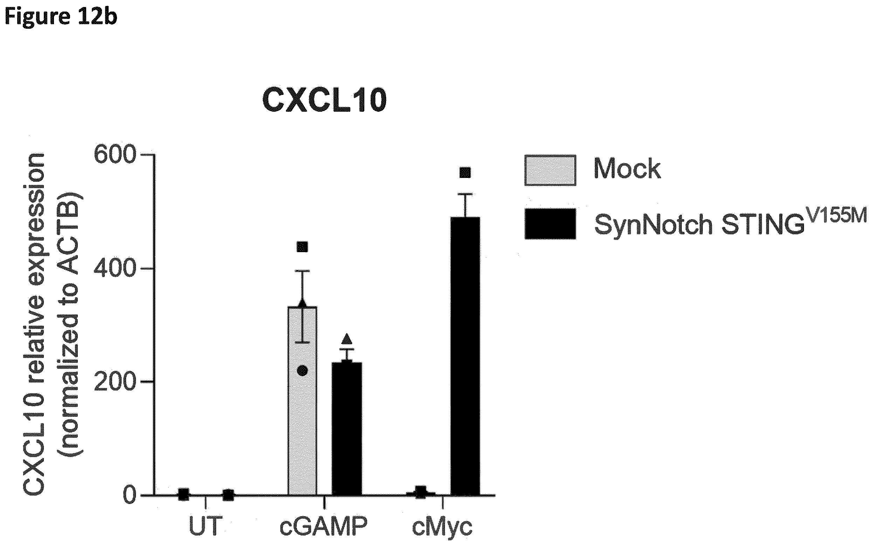

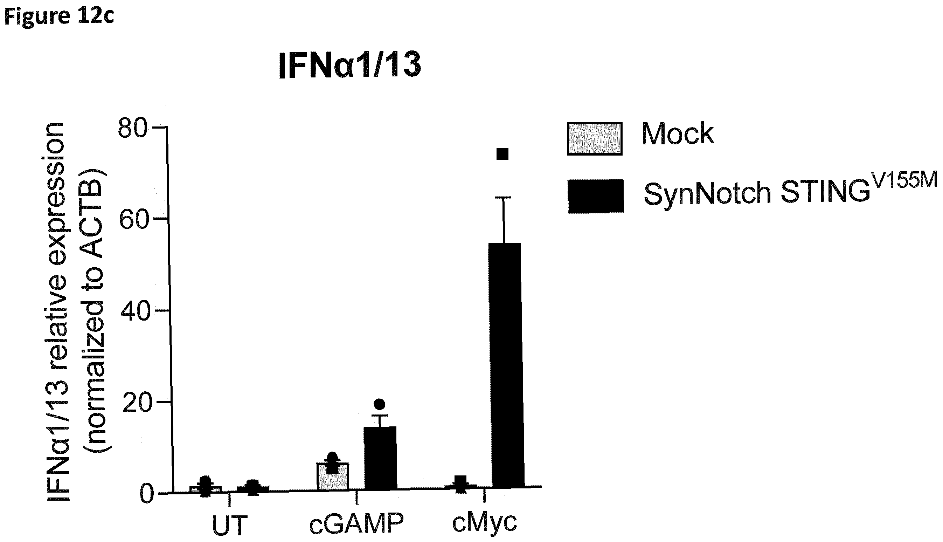

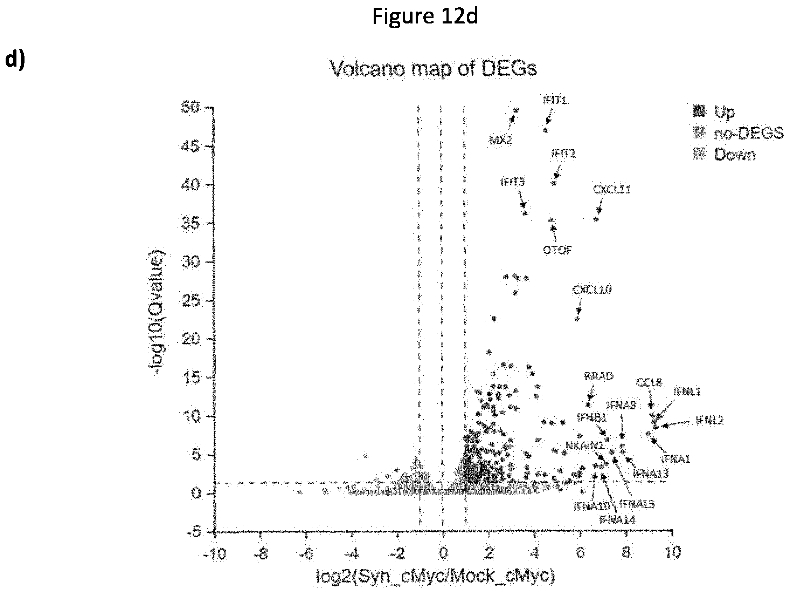

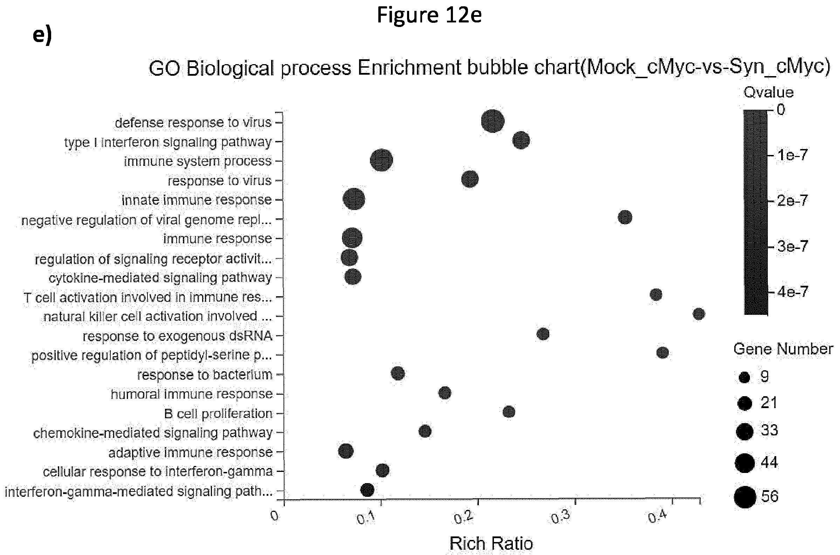

**Figure 12****.** RNA-seq profile of U-pDCs with activated SynNotch-STINGV155M shows upregulation of a broad immune response. a) Schematic showing the setup. 0.5-1e6 primed (IFN) or non-primed (IL-3) U-pDCs were seeded out and stimulated with anti-c-Myc magnetic beads (15 µL per 1e6 cells), cGAMP (4 µg/mL), or left unstimulated (UT). Following 20 hours of stimulation, RNA was extracted and subjected to qPCR and RNA-seq. b-c) Levels of CXCL10 (b) and IFNa1/13 (c) measured using qPCR. d) Volcano plot showing differentially expressed genes of Mock versus SynNotch STINGV 155M U-pDCs stimulated with anti-cMyc magnetic beads. Arrows indicate different known inflammatory genes. e) Gene ontology bubble chart displaying the 20 most enriched biological processes for the differentially expressed genes in activated SynNotch STINGV155M U-pDCs. The x-axis shows the ratio between the number of differentially expressed genes within the biological process and the number of total genes annotated in that process (rich ratio). The size of the bubble represents the number of differentially expressed genes within the process. Data are mean value +/- SEM of three donors (d-e) using technical triplicates (b-c).

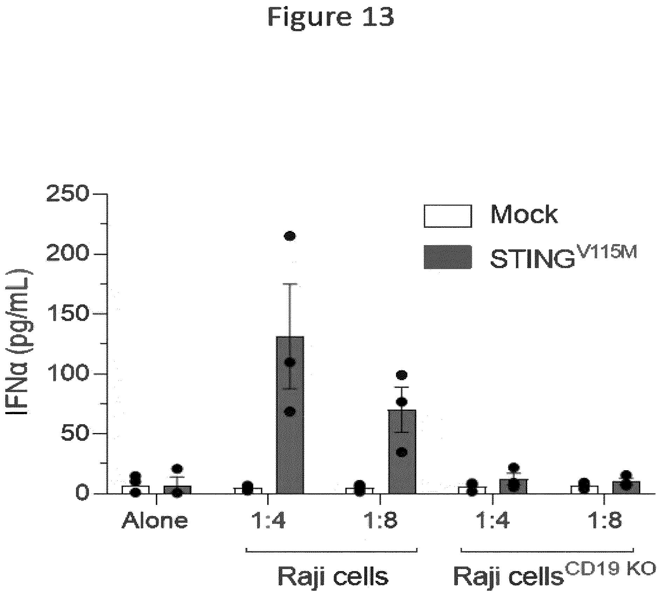

**Figure 13****.** U-pDCs expressing the SynNotch-STINGV155M are only activated upon recognition of cognate antigen. A total of 1e5 Mock or SynNotch-STINGV155M U-pDCs were co-cultured with Raji cells, or Raji cells with CRISPR-Cas9 knockout of CD19 (Raji CD19 KO). Following 20-hours of stimulation supernatants were harvested and evaluated by IFNa ELISA. Data are mean value +/- SEM of three donors.

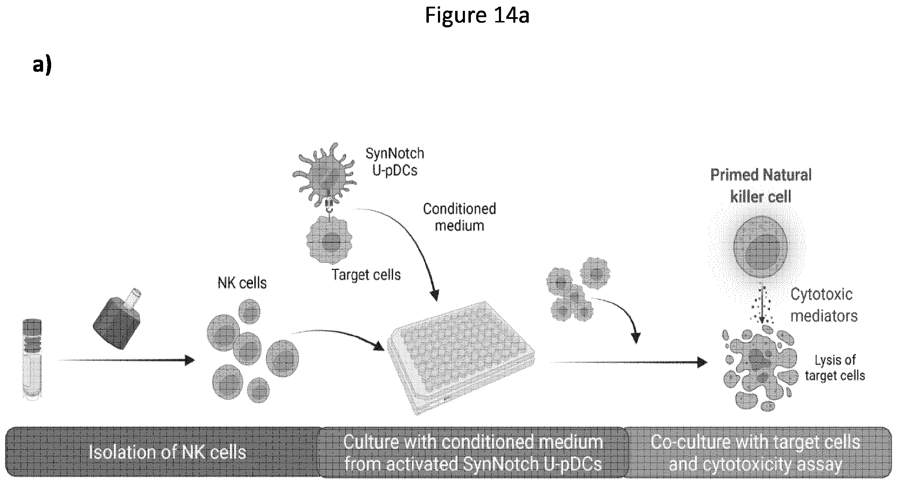

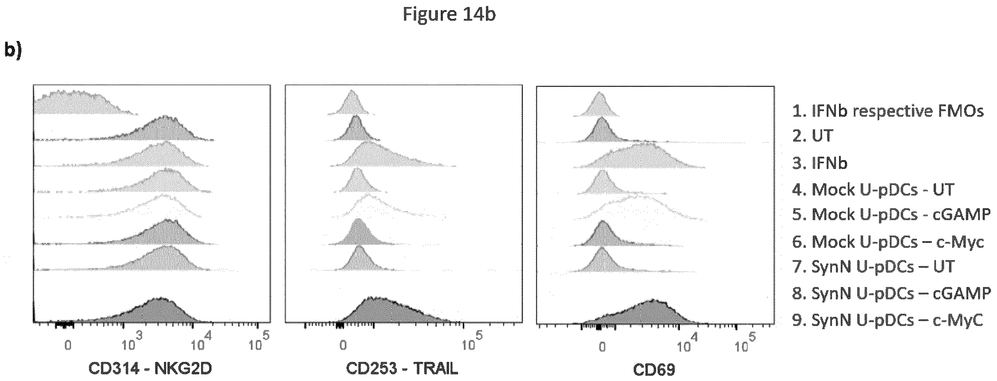

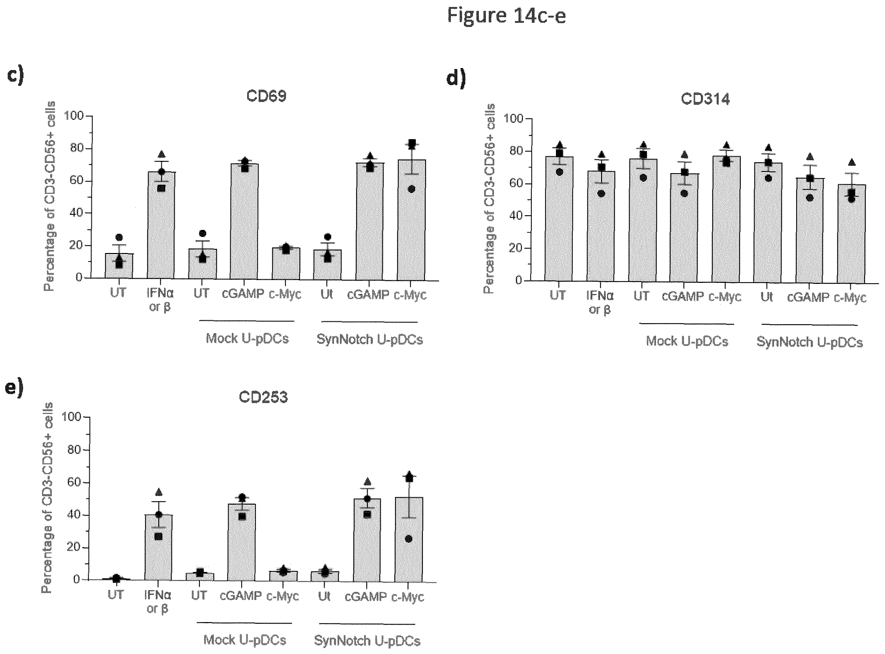

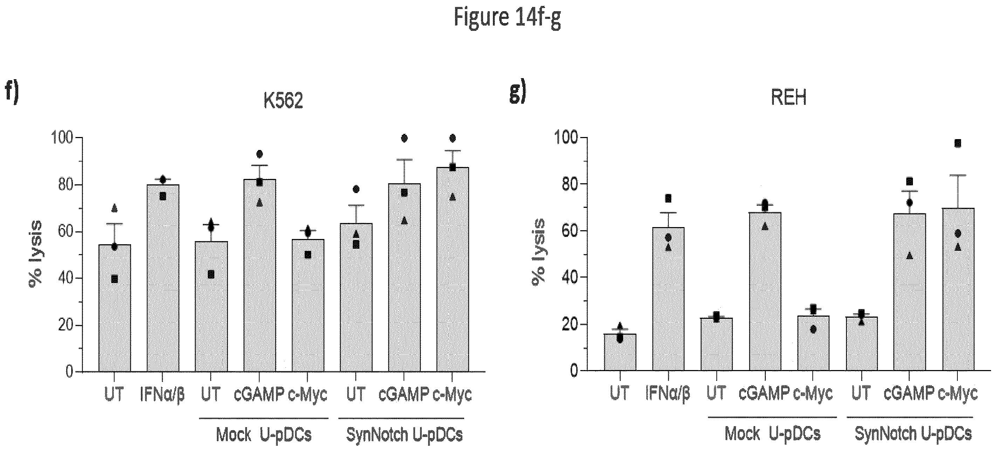

**Figure 14****.** U-pDCs expressing the SynNotch-STINGV155M activated by cognate antigen primes NK cells. a) Schematic showing the setup. NK cells were isolated from PBMCs using negative selection. Following selection, NK cells were primed for 20 hours in conditioned medium from Mock or SynNotch U-pDCs that had prior been stimulated with 2'3'-cGAMP or anti-c-Myc magnetic beads. Following priming, NK cells were analyzed for known NK activation markers (CD314, CD253, and CD69). Next a co-culture with primed NK cells and target cancer cells (genetically engineered to express eGFP and nanoluciferase) were established and lysis of target cells was estimated using flow cytometry (eGFP levels) or release of nanoluciferase in the supernatant. b) Expression of NK priming markers following culture of NK cells in conditioned medium from Mock or SynNotch U-pDCs. As a control DC medium alone (UT) or IFNa or IFNb was used (500 U/mL). Cells were gated based on viable, CD3 negative, and CD56 positive cells. Data are from one representative donor. c-e) Percentage expression of the NK priming markers CD69 (c), CD314 (d), and CD253 following culture in conditioned medium from Mock or SynNotch-STINGV155M U-pDCs. f-g) Following priming of NK cells, NK cells were overlayed with target cells K562 (f) or REH (g) expressing nanoluciferase at an E:T ratio of 2:1. Specific lysis of target cells was analyzed 20 hours later by assessing release of nanoluciferase in the supernatant. Percentage lysis is normalized to target cells lysed with tergitol. Target cell death without the effect of NK cells has been subtracted to get the specific lysis of cells. Data are mean value +/- SEM of three NK donors run in biological triplicates.

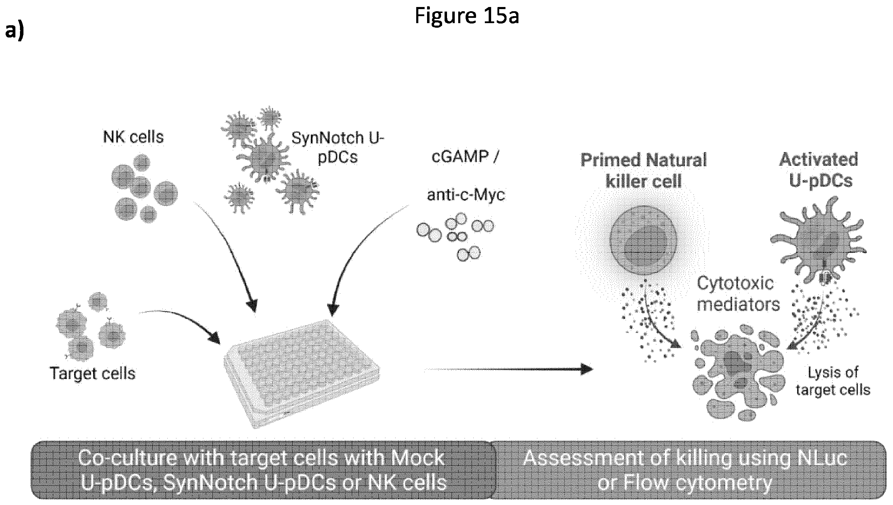

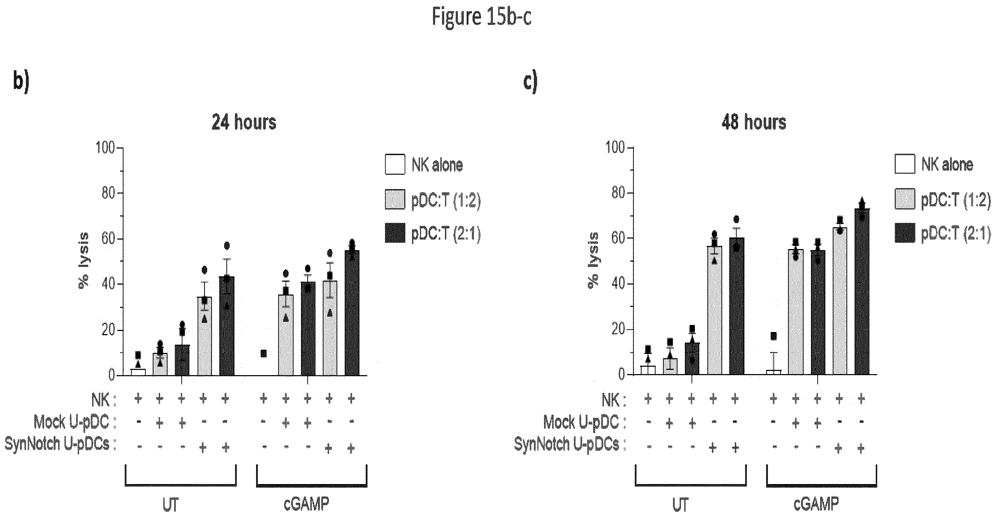

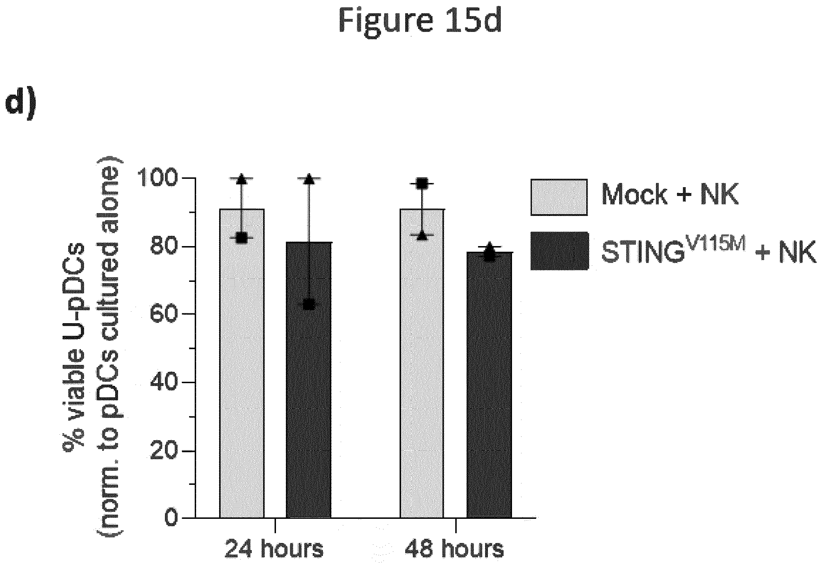

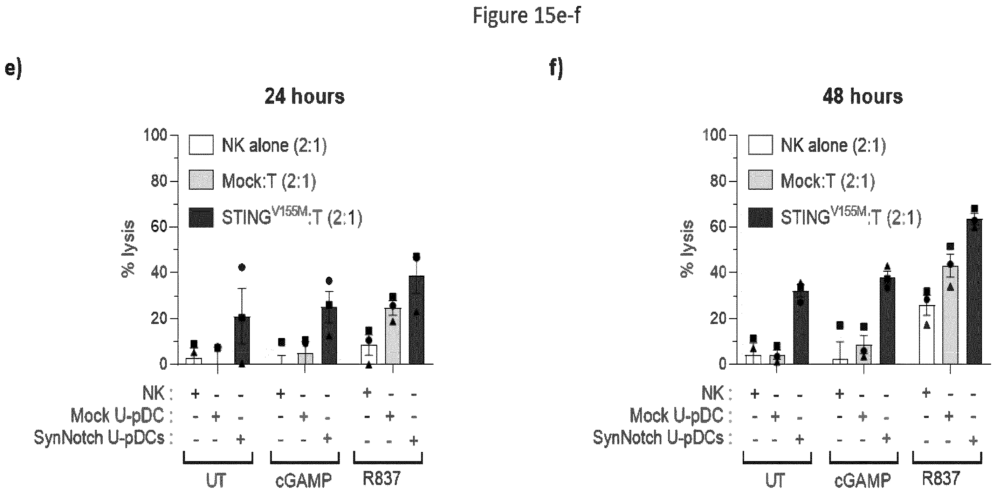

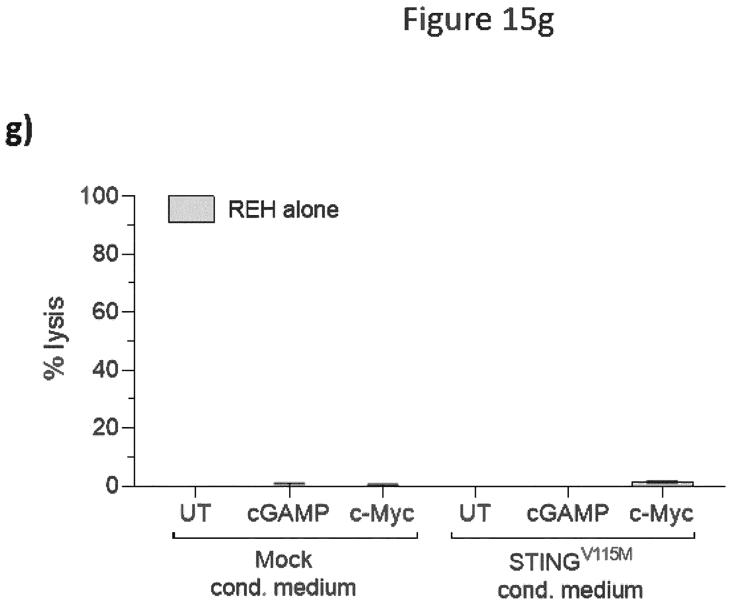

Figure 15. Activated SynNotch-STINGV155M U-pDCs directly kill cancer cells expressing cognate antigen. a) Schematic illustration of setup. In a co-culture setup U-pDCs or NK cells were either co-cultured together or alone with target cancer cells (REH expressing eGFP-nanoluciferase). An E:T ratio of 1:2 or 2:1 was used. To promote the activity of pDCs, cells were stimulated with cGAMP, or left unstimulated. Specific lysis of target cells was analyzed 24 hours or 48 hours later by counting viable target cells using flow cytometry. Percentage lysis is normalized to target cells cultured alone. b-c) Lysis of cancer cells following co-culture of NK cells and target cells with or without the addition of Mock or SynNotch STINGV155M U-pDCs. Lysis of target cells was determined after 24 hours (b) or 48 hours (c). d) Following 24 hours and 48 hours of co-culture of NK cells and U-pDCs numbers of U-pDCs were analyzed using flow cytometry to evaluate if the U-pDCs are killed by NK cells. e-f) Co-culture of NK cells, Mock U-pDCs, or SynNotch-STINGV155M U-pDCs with target cells (REH expressing eGFP-NL). Following 24-hours (e), or 48-hours (f) lysis of target cells was evaluated by counting numbers of viable target cells. g) REH cells were overlayed with conditioned medium from Mock or SynNotch-STINGV155M U-pDCs that had been stimulated for 20 hours with either cGAMP, anti-cMyc magnetic beads (which activates the SynNotch receptor), or left unstimulated. Specific lysis of REH were evaluated 20 hours later by assessing release of nanoluciferase in the supernatant. Data are mean value +/- SEM of three U-pDC and NK donors done in biological duplicates.

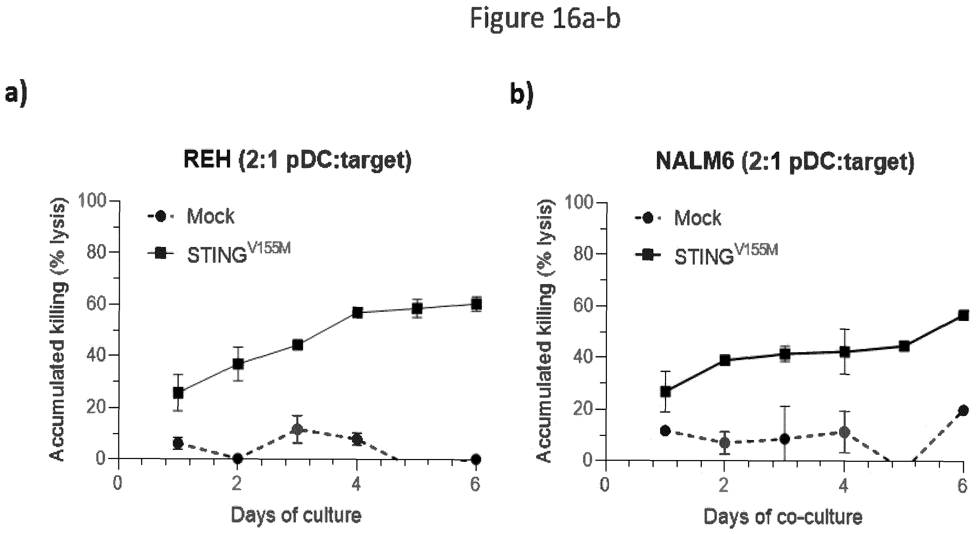

Figure 16. Killing of target cells and viability of U-pDCs during prolonged co-culture. U-pDCs were co-cultured with REH or NALM6 at an E:T ratio of 2:1. During co-culture cells were continuously taken out, and numbers of U-pDCs or target cells were evaluated using flow cytometry. a-b) Accumulated killing of REH cells (a), or NALM6 cells at an E:T ratio of 2: 1 (b) during 6-days of co-culture with Mock or SynNotch-STINGV155M U-pDCs. Data are mean value +/- SEM of three U-pDC donors in biological duplicates.

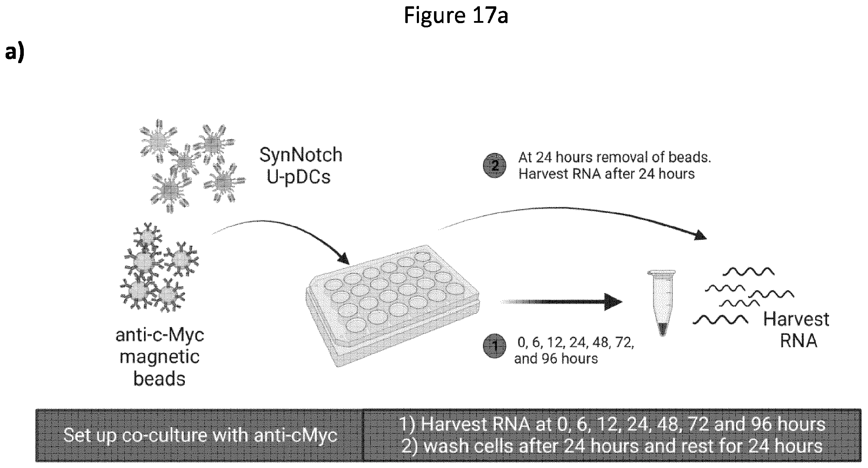

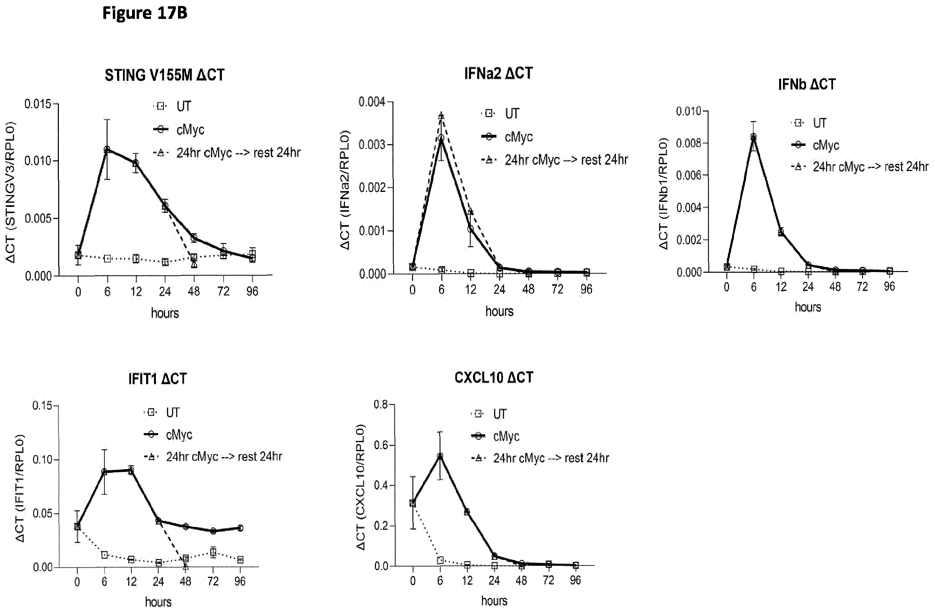

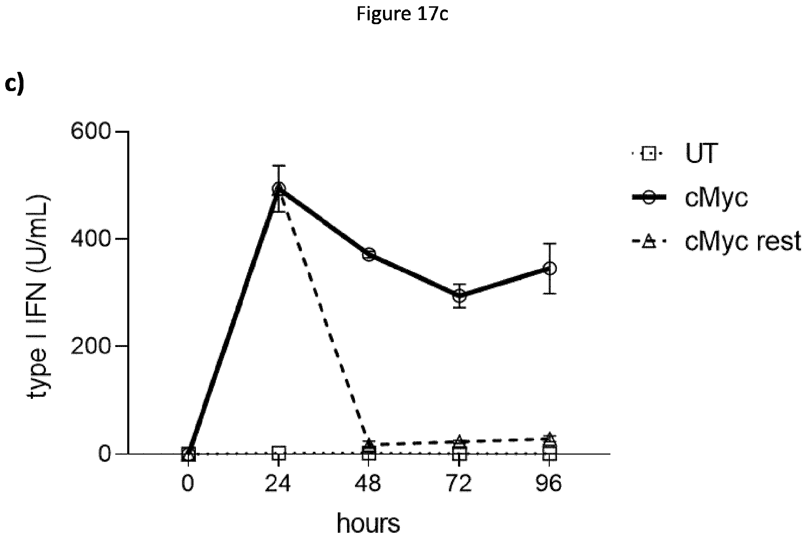

**Figure 17****.** Kinetic response of SynNotch STINGV155M activation in U-pDCs. a) Schematic illustration of setup. SynNotch-STINGV155M U-pDCs were seeded out and stimulated with anti-cMyc magnetic beads. Following 6, 12, 24, 48, 72 and 96 hours of stimulation RNA or supernatant was harvested. In a second setup, anti-cMyc magnetic beads were removed from cells, and cells were left for 24-hours before supernatant and RNA were harvested. b) Levels of STINGV155M, IFNa2, IFNb, IFIT1 and CXCL10 following stimulation or rest-phase. c) Levels of type I IFN during culture. Data are mean value +/- SEM of two U-pDC donors run in technical triplicates.

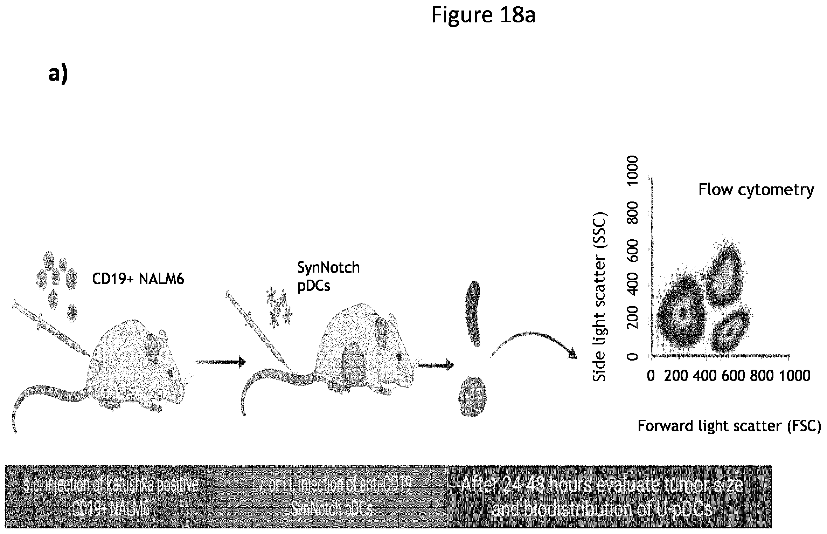

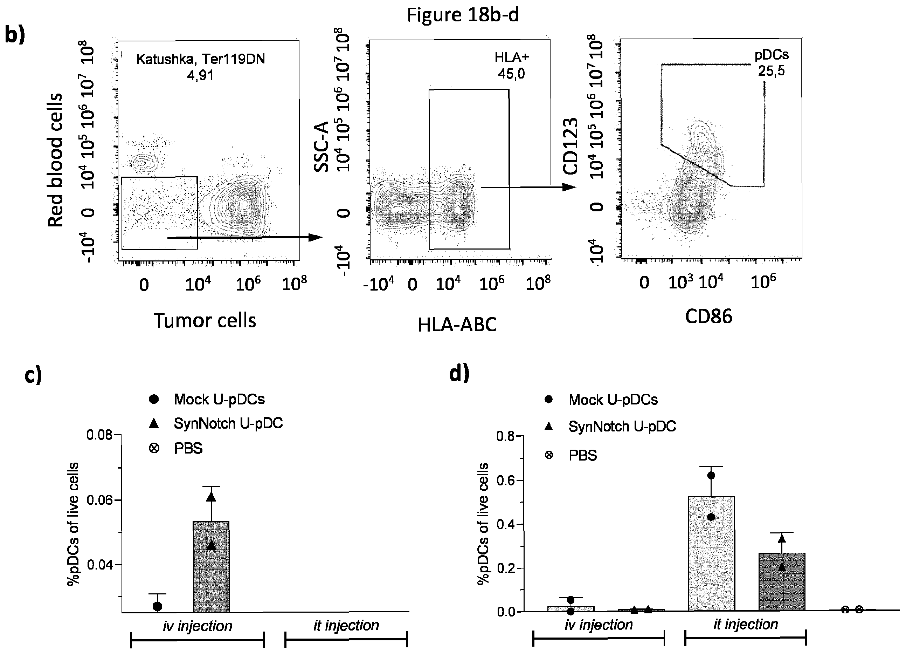

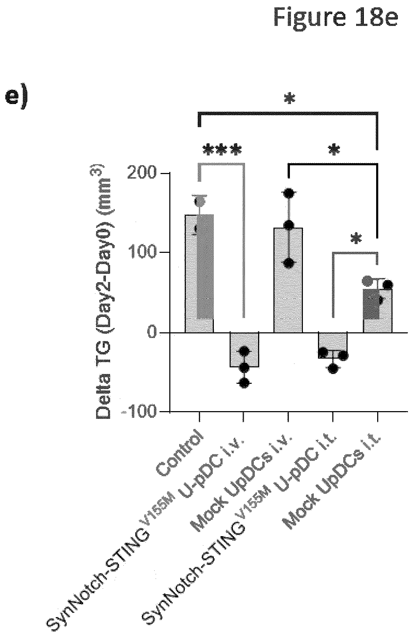

**Figure 18****.** SynNotch-STINGV155M U-pDCs induce immune responses and tumor regression in an in vivo model. a) Schematic illustration of setup. NXG mice (Janvier) were injected subcutaneously (s.c.) with 5e6 katushka-positive NALM6 cells. When mice had developed a mean tumor size of 120 mm3 ± 50 mm3, 1e7 Mock or SynNotch-STINGV155M U-pDCs were injected intravenously (i.v.) by the tail vein or intratumorally (i.t.). b-d) 48-hours after i.v. or i.t. administration of Mock or SynNotch-STINGV155M U-pDCs, spleen and tumor tissue was harvested and processed, and the proportion of U-pDCs were evaluated by flow cytometry. b) shows a gating strategy to evaluate infiltration of U-pDCs. Cells were initially gated based on FSC and SSC. Next, red blood cells (Ter119 positive), and katushka positive NALM6 tumor cells were excluded. Cells double negative for Katushka and Ter119 were next gated on HLA-ABC, and cells expressing CD123 and CD86 were evaluated. c-d) Proportion of U-pDCs from two mice evaluated from the spleen (c) or the tumor (d) 48-hours following injection. e) The tumor growth volume (n=3 mice per group) measured 48-hours post i.v. or i.t. injection with Mock or SynNotch-STINGV155M U-pDCs. Data are presented as mean +/- SEM. Comparison of groups (e) was done by One-way ANOVA followed by the Bonferroni multiple comparisons test.

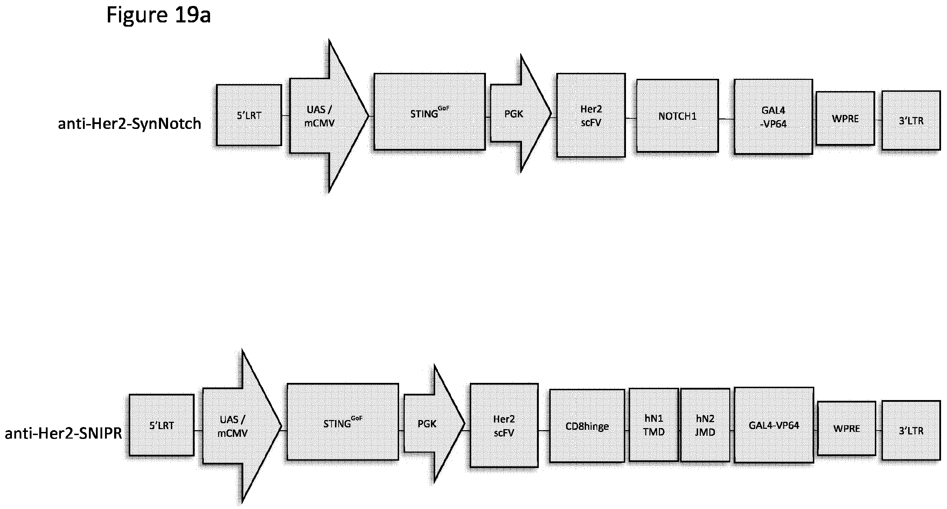

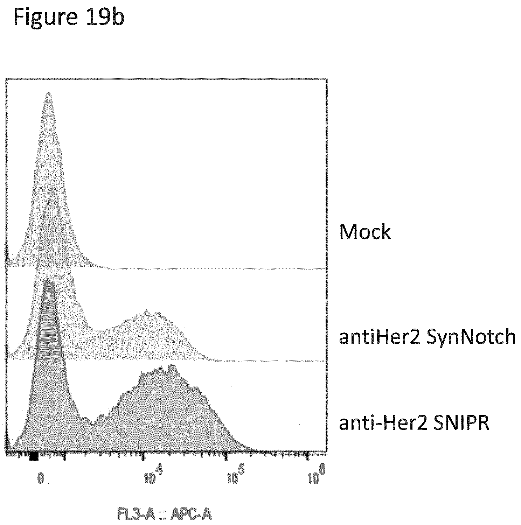

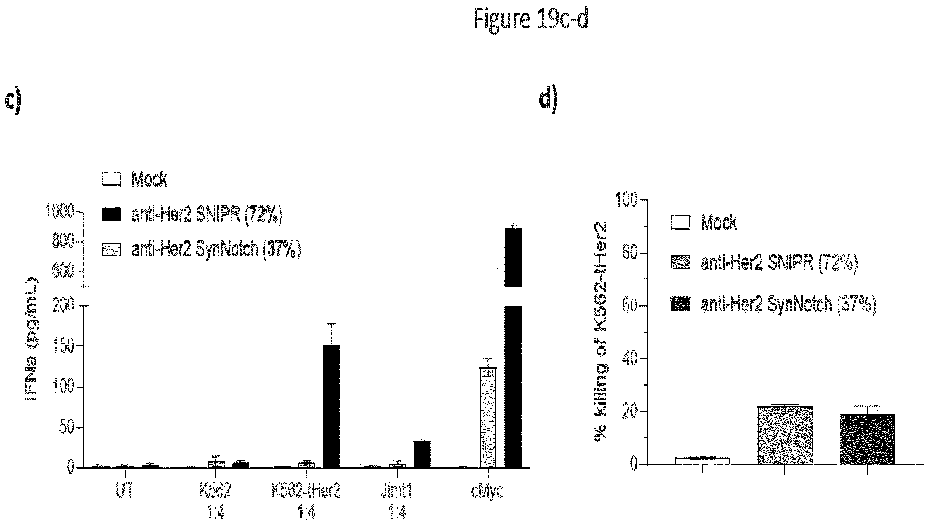

**Figure 19****.** Expression of the next-generation SNIPR System in U-pDCs. a) Schematic illustration showing the anti-Her2 SynNotch and anti-Her2 SNIPR harboring the STINGV155M response element. The constructs contain the STING V155M response element under the control of an inducible miniCMV promotor (mCMV) flanked by GAL4-binding UAS seuqences. A PGK controls the expression of the anti-Her2 SynNotch, or anti-Her2 SNIPR respectively, which are connected to a Gal4-VP64. Upstream of the anti-Her2 scFv is a c-Myc tag to allow for easy validation of construct expression and activation using anti-cMyc magnetic beads. b) Expression of the anti-Her2 SynNotch or anti-Her2 SNIPR in pDCs. c) A total of 1e5 Mock, anti-Her2 SynNotch, or anti-Her2 SNIPR pDCs were co-cultured with K562, K562 expressing a truncated variant of Her2 (K562-Her2), or the breast cancer cell line Her2 at an E:T ratio of 1:4. As a control cells were activated with anti-cMyc magnetic beads. Following 20-hours of stimulation supernatants were harvested and evaluated by IFNa ELISA.. d) Mock, anti-Her2 SynNotch, or anti-Her2 SNIPR pDCs were co-cultured with JIMT1 cells expressing eGFP-NL at a E:T of 2:1. Viability of JIMT1 cells were evaluated 48-hours later by release of nanoluciferase. Data are mean value +/- SEM of two donors.

### Detailed Description of the Invention

### Synthetic Receptors

The invention provides engineered immune cells that express on their surface a synthetic receptor comprising an extracellular antigen recognition domain that recognises an antigen on a target cell, a transmembrane domain, and an intracellular signalling domain that activates expression of a response element in the cell when the extracellular antigen recognition domain binds the target cell, wherein the cell comprises a heterologous nucleic acid comprising the response element, wherein the response element encodes a STING protein, and wherein the intracellular signalling domain is a transcription factor comprising a DNA binding domain and a transcriptional activation domain, and wherein the response element contains the cognate recognition element for the DNA binding domain, and
wherein the synthetic receptor is:
(a) a synthetic intramembrane proteolysis receptor (SNIPR), such as wherein the synthetic receptor is a synthetic RIP family receptor, optionally wherein the receptor is:
   (i) synthetic Notch receptor;
   (ii) a synthetic Robo receptor; or
   (iii) a synthetic RoboNotch receptor;
(b) a Tango receptor; or
(c) a modular extracellular signalling architecture (MESA) system. The synthetic receptors allow the cells to recognise and bind specific target cells and to activate immune responses upon binding.

In some embodiments, the cell is a lymphoid cell. In some embodiments, the cell is a myeloid cell, such as a macrophage. In some embodiments, the cell is a natural killer (NK) cell, a T cell, a B cell, or a plasmacytoid dendritic cell (pDC). In a preferred embodiment, the cell is a plasmacytoid dendritic cell (pDC).

In some embodiments, the synthetic receptor is a synthetic intramembrane proteolysis receptor (SNIPR).

SNIPRs comprise an extracellular antigen recognition domain, which may be an scFv with specificity for a particular cell surface antigen, such as a tumour antigen, a transmembrane domain, which controls proteolysis and cleavage, and an intracellular signalling domain, which may be a transcription factor. SNIPRs comprise a transmembrane domain from the regulated intracellular proteolysis (RIP) family of proteins. For example, a SNIPR may comprise a transmembrane domain selected from the group consisting of Notch1, Notch2 and Robo1. In preferred embodiments, the SNIPR is a SynNotch receptor. Binding of the antigen recognition domain releases the transcription factor, which can target a response element that encodes STING either wildtype or with a selective mutation, such as a gain-of-function mutation. Recognition elements for the transcription factor drive expression of STING protein, thereby supporting constitutive activation of an immune response. As shown in the Examples, binding of an immune cell of the invention to its target cell activates expression of STING, which induces a targeted and potent immune response useful for treating disease, including production of type I and III IFNs, CXCL10, MCP-1, MCP-2, MIP-1a, MIP-1b. STING and preferred STING proteins are discussed in the subsequent section. In some embodiments, the SNIPR may further comprise a juxtamembrane domain, such as a human NOTCH2 juxtamembrane domain.

In some embodiments, the immune cells comprise a heterologous nucleic acid encoding the synthetic receptor. The immune cells comprise a heterologous nucleic acid encoding the response element. In some embodiments the synthetic receptor and response element are encoded by a single heterologous nucleic acid. In some embodiments, the heterologous nucleic acid or acids is or are selected from the group consisting of a viral construct, a plasmid, a cosmid, an mRNA. In some embodiments, the viral construct is an AAV construct, an adenoviral construct, a lentiviral construct, or a retroviral construct.

In some embodiments, the heterologous nucleic acid is integrated into the genome of the engineered cell. In some embodiments, the heterologous nucleic acid is not integrated into the genome of the engineered cell. In some embodiments, the heterologous nucleic acid is introduced by a transposase, retrotransposase, episomal plasmid, mRNA, or random integration. Preferably, the heterologous nucleic acid is introduced with a gene editing system such as TALEN, zinc finger or, most preferably, CRISPR/Cas9.

The term heterologous as used herein has its normal meaning. In particular, the heterologous nucleic acid is a nucleic acid that has been introduced into the cell and that is not present in an unmodified cell in the same configuration or location. The heterologous nucleic acid may be constructed using endogenous (preferably human) sequences.

Each domain may be heterogeneous, that is, comprised of sequences derived from different protein chains.

The extracellular antigen recognition domain recognises an antigen on a target cell and can be designed or selected according to the desired therapeutic use or target cell. In some embodiments, the antigen recognition domain is an antibody or fragment thereof, e.g., a Fab, Fab', Fv, F(ab')2, dAb, or preferably, one or more single-domain antibodies ("nanobodies") or one or more single chain antibody fragments ("scFv"). A scFv is a single chain antibody fragment having the variable regions of the heavy and light chains of an antibody linked together. A single-domain antibody, also known as a nanobody, is an antibody fragment consisting of a single monomeric variable antibody domain. scFvs and nanobodies are useful in synthetic receptors because they may be engineered to be expressed as part of a single chain along with the other synthetic receptor components.

The extracellular antigen recognition domain will generally recognise a cell surface antigen. In certain embodiments, for example when used for treating cancer, the extracellular antigen recognition domain will recognise and specifically bind a tumour associated antigen, which is an antigen that is expressed exclusively on tumour cells or that is expressed at a higher level by tumour cells relative to healthy cells. In certain embodiments, for example when used for treating an autoimmune disease or an inflammatory disease, the extracellular antigen recognition domain will recognise and specifically bind an antigen that is expressed on the surface of the pathogenic autoreactive immune cells such as B-cells or macrophages underlying the autoimmune or inflammatory disease. Suitable antigens that may be bound for targeting B-cells or macrophages include CD20 and CD19. In certain embodiments, for example when used for treating an infectious disease, the extracellular antigen recognition domain will recognize and specifically bind a pathogen antigen.

In certain embodiments, the extracellular antigen recognition is bispecific or multispecific, with specificity to more than one target of interest.

The extracellular antigen recognition domain recognises an antigen on a target cell. The antigen may therefore be a target antigen. In certain embodiments, the domain specifically binds the antigen and does not exhibit significant binding to any other antigens, or exhibits significantly stronger binding to the target antigen than any other antigen. In preferred embodiments, expression of the response element only occurs when the extracellular antigen recognition domain binds its specific antigen.

In preferred embodiments, the antigen recognised by the extracellular antigen recognition domain is a tumour antigen, preferably a tumour-associated surface antigen. In certain embodiments, the tumour antigen is selected from the group consisting of 5T4, alphafetoprotein (AFP), B7-1 (CD80), B7-2 (CD86), BCMA, B-human chorionic gonadotropin, CA-125, carcinoembryonic antigen (CEA), carcinoembryonic antigen (CEA), CD123, CD133, CD138, CD19, CD20, CD22, CD23, CD24, CD25, CD30, CD33, CD34, CD4, CD40, CD44, CD56, CD70, CD8, CLL-1, c-Met, CMV-specific antigen, CS-1, CSPG4, CTLA-4, DLL3, disialoganglioside GD2, ductal -epithelial mucine, EBV-specific antigen, EGFR variant III (EGFRvIII), ELF2M, endoglin, ephrin B2, epidermal growth factor receptor (EGFR), epithelial cell adhesion molecule (EpCAM), epithelial tumour antigen, ErbB2 (HER2/neu), fibroblast associated protein (fap), FLT3, folate binding protein, GD2, GD3, glioma-associated antigen, glycosphingolipids, gp36, HBV- specific antigen, HCV-specific antigen, HER1, HER2, HER2-HER3 in combination, HERV-K, high molecular weight-melanoma associated antigen (HMW-MAA), HIV-1 envelope glycoprotein gp4l, HPV-specific antigen, human telomerase reverse transcriptase, IGFI receptor, IGF-II, IL-11Ralpha, IL-13R-a2, Influenza Virus-specific antigen, CD38, insulin growth factor (IGF1)-1, intestinal carboxyl esterase, kappa chain, LAGA-la, lambda chain, Lassa Virus-specific antigen, lectin-reactive AFP, lineage-specific or tissue specific antigen such as CD3, MAGE, MAGE-A1, major histocompatibility complex (MHC) molecule, major histocompatibility complex (MHC) molecule presenting a tumour-specific peptide epitope, M-CSF, melanoma-associated antigen, mesothelin, MN-CA IX, MUC-l, mut hsp70-2, mutated p53, mutated p53, mutated ras, neutrophil elastase, NKG2D, Nkp30, NY-ESO-1, p53, PAP, prostase, prostate specific antigen (PSA), prostate-carcinoma tumour antigen- 1 (PCTA-l), prostate-specific antigen protein, STEAP1, STEAP2, PSMA, RAGE-l, ROR1, RU1, RU2 (AS), surface adhesion molecule, survivin, telomerase, TAG-72, the extra domain A (EDA) and extra domain B (EDB) of fibronectin and the A1 domain of tenascin-C (TnC Al), thyroglobulin, tumour stromal antigens, vascular endothelial growth factor receptor-2 (VEGFR2), virus-specific surface antigen such as an HIV-specific antigen (such as HIV gpl20), as well as any derivate or variant of these surface markers.

In preferred embodiments, the extracellular antigen recognition domain recognises an antigen selected from the group consisting of BCMA, CD19, CLL1, CS1, STEAP1, STEAP2, CD70, and CD20. In some embodiments, the extracellular antigen recognition domain specifically targets CD19.

In other preferred embodiments, the extracellular antigen recognition domain recognises HER2. In some embodiments, the extracellular antigen recognition domain specifically targets HER2.

In preferred embodiments, the antigen recognised by the extracellular antigen recognition domain is a tumour antigen, preferably a tumour-associated surface antigen. In certain embodiments, the tumour antigen is selected from the group consisting of CD19 , CD20, CD22, CD30, CD33, CD38, CD123, CD138, CS-1, B-cell maturation antigen (BCMA), MAGEA3, MAGEA3/A6, KRAS, CLL1, MUC-1, HER2, EpCam, GD2, GPA7, PSCA, EGFR, EGFRvIII, ROR1, mesothelin, CD33/IL3Ra, c-Met, CD37, PSMA, Glycolipid F77, GD-2, gp100, NY-ESO-1 TCR, FRalpha, CD24, CD44, CD133, CD166, CA-125, HE4, Oval, estrogen receptor, progesterone receptor, uPA, PAI-1, MICA, MICB, ULBP1, ULBP2, ULBP3, ULBP4, ULBP5 or ULBP6, or a combination thereof.

In certain embodiments, the immune cell is useful for treating an infectious disease and the infectious disease is a viral or fungal infection, such as infection of Influenza, Yellow Fever virus, West Nile virus, Hantavirus, Ebola virus, Rotavirus, Norovirus, Rabies virus, Tick-borne encephalitis virus (TBEV), rhinoviruses, Coronavirus, RSV, Measles, Parainfluenza, Zikavirus, Dengue virus, HIV, HBV, HCV, human cytomegalovirus (CMV), Epstein-Barr virus (EBV), *Aspergillus spp.,* such as *Aspergillus fumigatus, Candida spp*., such as *C*. *albicans, Mucorales, Cryptococcus, spp.,* such as *Cryptococcus neoformans* or *Cryptococcus gattii*, or *Pneumocystis jirovecii*. The cells of the invention are expected to be particularly useful for treating chronic infections. In such embodiments, the antigen recognised by the extracellular antigen recognition domain is a pathogen antigen. The immune cell will then target the pathogen via the antigen and clear the infectious agent. In certain embodiments, the antigen is selected from: Yellow Fever virus NS1 protein, West Nile virus NS1 protein, Hantavirus N protein, Ebola virus N protein, Rotavirus VP6, Norovirus VP1, rabies virus N protein, TBEV envelope glycoprotein, rhinovirus VP proteins, *Influenza* hemagglutinin antigens, *Influenza* neuraminidase antigens, coronavirus spike protein, RSV F protein, MeV N protein, *Parainfluenza* hemagglutinin antigens, *Parainfluenza* neuraminidase antigens, ZIKV E, NS1, NS3, NS4B, and NS5 proteins, Dengue virus C protein, M protein, E protein, and NS1 protein, gp120, gp41, Env, HBV surface antigen, HBV-surface proteins S and L, HCV E2 glycoprotein, CMV glycoprotein B, fungal beta glucan.

The intracellular signalling domain activates expression of a response element which activates expression of STING in the cell when the extracellular antigen recognition domain binds the target cell. The expression of STING activates an inflammatory immune response. In some embodiments, the expression of STING results in the induction of Interferon Stimulated Genes (ISGs) in the cell. In some embodiments, the ISGs induced following the expression of STING comprise IFIT1, IFIT2, MX2, CXCL11, CXCL10, IFNL1, IFNL2, IFNL3, IFNB1, IFNA8 and IFNA10.

As shown in the examples, induction of STING expression and resulting expression of ISGs is rapid. In some embodiments, STING expression is induced within 12 hours of binding the antigen on the target cell, such as within 10 hours, 8 hours, 6 hours or 5 hours. Preferably, STING expression is induced within 6 hours. In preferred embodiments, maximal STING expression is induced within 12 hours, such as within 10 hours, 8 hours, 6 hours or 5 hours. Preferably, maximal STING expression is induced within 6 hours. In some embodiments, expression of ISGs is induced within 12 hours of binding the antigen on the target cell, such as within 10 hours, 8 hours, 6 hours or 5 hours. Preferably, expression of ISGs is induced within 6 hours. In preferred embodiments, maximal expression of ISGs is induced within 12 hours, such as within 10 hours, 8 hours, 6 hours or 5 hours. Preferably, maximal expression of ISGs is induced within 6 hours.

In preferred embodiments, the immune response activated in the cell is the secretion of cytokines. The cytokines may include pro-inflammatory cytokines. For example, pro-inflammatory cytokines may promote an inflammatory response, which may be useful in cancer therapy. Examples of pro-inflammatory cytokines include, but are not limited to, IL-1a, IL-1b, IL-6, IL-13, IL-17a, tumour necrosis factor (TNF)-alpha, TNF-beta, fibroblast growth factor (FGF) 2, granulocyte macrophage colony-stimulating factor (GM-CSF), soluble intercellular adhesion molecule 1 (sICAM-1), soluble vascular adhesion molecule 1 (sVCAM-1), vascular endothelial growth factor (VEGF), VEGF-C, VEGF-D, and placental growth factor (PLGF). Examples of effectors include, but are not limited to, granzyme A, granzyme B, soluble Fas ligand (sFasL), and perforin. Examples of acute phase-proteins include, but are not limited to, C -reactive protein (CRP) and serum amyloid A (SAA). Preferred cytokines expressed following activation of the intracellular signalling domain and expression of STING include IFNα, IFNβ, IFNλ, TNFα, IL-6, CXCL9, CXCL10, CCL2, CCL3, CCL4, CCL8 and/or CXCL11. Most preferred cytokines are CXCL10 and type-I interferons. Further preferred cytokines are MCP-1, MCP-2, MIP-1a, MIP-1b.

As shown in the examples, secretion of cytokines is rapid. In some embodiments, secretion of cytokines occurs within 12 hours of binding the antigen on the target cell, such as within 10 hours, 8 hours, 6 hours or 5 hours. Preferably, secretion of cytokines occurs within 6 hours. In preferred embodiments, maximal secretion of cytokines occurs within 12 hours, such as within 10 hours, 8 hours, 6 hours or 5 hours. Preferably, maximal secretion of cytokines occurs within 6 hours.

The response element encodes a STING protein. In some embodiments, the STING protein activates an immune response. In some embodiments, the immune response comprises secretion of cytokines or comprises recruitment or activation of host immune cells, such cytotoxic lymphocytes (e.g. NK cells and CD8 T cells). In some embodiments, the cytokines are pro-inflammatory cytokines. In some embodiments, the cytokines comprise CXCL10, MCP-1, MCP-2, MIP-1a and/or MIP-1b. In some embodiments, the immune response comprises production of Type I IFN.

In some embodiments, the immune response comprises the activation of NK cells. Activation may induce CD69 and TRAIL on the NK cells. In some embodiments, activation of the NK cells increases their cytotoxic activity. In preferred embodiments, activation of the NK cells stimulates the NK cells to lyse target cells expressing the antigen.

In certain embodiments, the immune response that is activated by STING is the recruitment of immune cells, such as or cytotoxic lymphocytes (e.g. NK cells and CD8 T cells).

In some embodiments, the immune response is the direct lysis of target cells by the immune cell.

The intracellular signalling domain is preferably a transcription factor. The transcription factor may comprise a DNA binding domain and a transcriptional activation domain. The DNA binding domain may be selected from the group consisting of Gal4, Pax6, zinc fingers (ZFs), synthetic zinc fingers (synZFs), transcription activator like effectors (TALEs), TetR, HNF1 alpha, vHNF1 beta. The response element will contain the cognate recognition element for the DNA binding domain. Appropriate recognition elements are well established, including UAS elements, which are bound by Gal4, and HNF 1-binding site, which is bound by HNF1 alpha, etc.

The DNA binding domain may be derived from the group consisting of Gal4, Pax6, zinc fingers (ZFs), synthetic zinc fingers (synZFs), transcription activator like effectors (TALEs), TetR, HNF1 alpha, vHNF1 beta. The transcription activation domain may be selected from the group consisting of VP64, VP16, WWTR1, CREB3, NF-κB, p65, Rta, HSF1, and RelA. The transcription activation domain may be derived from the group consisting of VP64, VP16, WWTR1, CREB3, NF-κB, p65, Rta, HSF1, and RelA. The intracellular signalling domain may be selected from the group consisting of Gal4-VP64, Gal4-VP16, TetR-VP64, LacI-VP64, HNF1-WWTR1, HNF1-CREB3, Pax6-p65, ZF-p65, synZF-p65, or HNF1-p65. The response element comprises a promoter that is activated by the intracellular signalling domain. Preferably, the response element comprises an inducible promoter that is activated by the intracellular signalling domain. For example, a suitable promoter is an inducible miniCMV promoter, optionally comprising UAS sequences that are bound by GAL-4. The promoter is operably linked to a gene that encodes a STING protein. The response element and STING gene may be integrated in the genome or present on a vector such as a plasmid. The response element and gene may be introduced by any appropriate technique, such as by transposase, retrotransposase, episomal plasmid or random integration. Preferably, the response element is introduced with a gene editing system such as TALEN, zinc finger or, most preferably, CRISPR/Cas9. Preferred synthetic receptors comprise extracellular antigen recognition domains described above. Preferred synthetic receptors comprise intracellular signalling domains, such as transcription factors, that activate expression of STING.

In some embodiments, the synthetic receptor comprises a Notch domain encoded by a Notch gene. A preferred Notch gene is NOTCH1. Alternative Notch genes include NOTCH2, NOTCH3 and NOTCH4. In other embodiments, the synthetic receptor comprises a Robo1 domain.

In certain embodiments, the synthetic receptor system is multiplexed with multiple transcription factor domains and/or response elements. In certain embodiments, the immune cell expresses on its surface a synthetic receptor, optionally a SynNotch receptor, comprising an extracellular antigen recognition domain that recognises an antigen on a target cell, a transmembrane domain, and two or more intracellular signalling domains that activate expression of a two or more response elements in the cell when the extracellular antigen recognition domain binds the target cell, wherein at least one response element encodes a stimulator of interferon genes (STING) protein, and wherein at least one response element encodes an additional gene, preferably a gene that activates an immune response. In certain embodiments, the pDC expresses on its surface two or more synthetic receptors, optionally SynNotch receptors, wherein at least one activates expression of STING, and at least one activates expression of an additional gene, preferably a gene that activates an immune response. In certain such embodiments, the additional gene encodes TRAIL, a checkpoint inhibitor, such as PDL1, IL-12 or Type I IFN. In preferred embodiments, expression of PD-L1 or IL10 is activated. In preferred embodiments, expression of IL-12 is activated.

The synthetic receptor comprises a transmembrane domain linking the extracellular antigen recognition domain and the intracellular signalling domain. The transmembrane domain may be derived from the transmembrane domain of 4-1BB/CD137, an alpha chain of a T cell receptor, a beta chain of a T cell receptor, a gamma chain of a T cell receptor, a delta chain of a T cell receptor, CD3 epsilon, CD3 delta, CD3 gamma, CD3 zeta, CD4, CD5, CD8 alpha, CD9, CD16, CD19, CD22, CD33, CD34, CD37, CD45, CD64, CD80, CD86, CD 134, CD137, CD154, or a zeta chain of a T cell receptor, or any combination thereof. The transmembrane domain may be derived from a member of the regulated intramembrane proteolysis (RIP) family, for example the transmembrane domain may be derived from Notch1, Notch2, Notch3, Notch4, Robo1. In a preferred embodiment, the transmembrane domain is derived from Notch1.

In certain embodiments, the synthetic receptor comprises a hinge or spacer, in particular to allow free movement of the extracellular antigen recognition domain. Suitable hinge or spacers include regions of IgG1, IgG2, IgG3, IgG4, IgA, IgD, IgE, and IgM, or a fragment thereof. In some embodiments, the hinge or spacer is from CD8 alpha. Optionally, short linkers may form linkages between any or some of the extracellular, transmembrane, and intracellular domains of the construct. In some embodiments, the linker may be derived from repeats of glycine-glycine-glycine-glycine-serine. The linkers may also be used as a peptide tag. The linker peptide sequence may be of any appropriate length to connect one or more proteins of interest and is preferably designed to be sufficiently flexible so as to allow the proper folding and/or function and/or activity of one or both of the peptides it connects.

In preferred embodiments, the immune cell, preferably a pDC, comprises a nucleic acid that encodes the synNotch receptor and the nucleic acid encodes:
a) a promoter, such as PGK,
b) an extracellular antigen recognition domain that is an anti-CD19 scFv, such as an anti-CD19 scFv,
c) NOTCH1
d) a Gal4-VP64 transcription factor, and
e) optionally a Woodchuck Hepatitis Virus (WHV) Posttranscriptional Regulatory Element (WPRE);
and the engineered cell also comprises a response element encoding:
a) a promoter responsive to the transcription factor in the synNotch receptor such as the minimal CMV promoter preceded by 5x UAS elements, which are target sequences for Gal4, and
b) STING, preferably comprising a gain-of-function mutation that causes it to be constitutively active in the absence of 2'3'-cGAMP, and optionally:
c) an IRES,
d) a marker, such as mCherry,
e) a promoter, such as PGK, followed by a reporter, such as eGFP, and
f) a Woodchuck Hepatitis Virus (WHV) Posttranscriptional Regulatory Element (WPRE)
g) a suicide switch, e.g. iCas9.

In other preferred embodiments, the immune cell, preferably a pDC, comprises a single nucleic acid which encodes both the synNotch receptor and the response element, wherein the nucleic acid encodes:
a) a promoter responsive to the transcription factor in the synNotch receptor such as the minimal CMV promoter preceded by 5x UAS elements, which are target sequences for Gal4,
b) STING, preferably comprising a gain-of-function mutation that causes it to be constitutively active in the absence of 2'3, preferably a V155M mutation,
c) a second promoter, such as PGK,
d) an extracellular antigen recognition domain, such as a scFv, preferably an anti-HER2 scFv,
e) NOTCH1,
f) a Gal4-VP64 transcription factor, and
g) optionally a Woodchuck Hepatitis Virus (WHV) Posttranscriptional Regulatory Element (WPRE).

In other preferred embodiments, the immune cell, preferably a pDC, comprises a single nucleic acid which encodes both the SNIPR and response element, wherein the nucleic acid encodes:
(a) a promoter, responsive to the transcription factor in the SNIPR, such as the minimal CMV promoter preceded by 5x UAS elements, which are target sequences for Gal4, and
(b) STING, preferably comprising a gain-of-function mutation that causes it to be constitutively active in the absence of 2'3'-cGAMP
(c) a second promoter, such as PGK,
(d) an extracellular antigen recognition domain, such as a scFv, preferably an anti-HER2 scFv,
(e) a CD8 hinge region,
(f) a transmembrane domain, preferably a human NOTCH 1 transmembrane domain (hN1 TMD),
(g) a juxtamembrane domain, preferably a human NOTCH2 juxtamembrane domain
(h) a Gal4-VP64 transcription factor, and
(i) optionally a Woodchuck Hepatitis Virus (WHV) Posttranscriptional Regulatory Element (WPRE).

Exemplary constructs are set forth in the Examples, and in particular in Example 14 and Figure 19a.

The Examples demonstrate that such constructs can be expressed by pDCs, which maintain their functionality and type I IFN response, and can be used to successfully recognise target cells, bind and subsequently become activated to produce IFN-alpha and pro-inflammatory factors. The constructs could be expressed in any immune cell, such as T cells, B cells, NK cells, and macrophages. In certain embodiments the synthetic receptor and the response element are encoded by separate heterologous nucleic acids. In certain embodiments, the synthetic receptor and the response element are encoded by the same heterologous nucleic acid, which can simplify manufacture of the engineered immune cell, as demonstrated in the Examples.

### STING protein

The examples demonstrate that the stimulation of interferon genes (STING) protein is effective to activate a potent and targeted immune response in engineered immune cells in combination with a synthetic receptor system. STING is known to play a role in promoting expression of interferons in an immune response and the STING pathway can be activated in T cells, macrophages, B cells, NK cells, and other leukocytes to produce type I IFNs (Su, Ting et al., 2019, Theranostics vol. 9,25 7759-7771), so activation of STING is expected to be effective in different immune cells.

The response element encodes a stimulator of interferon genes (STING) protein. STING is an ER-associated membrane protein that is critical for innate immune sensing of pathogens and STING is a key immune response regulator. STING-mediated activation of the IFN-I pathway through the TBK1/IRF3 signaling axis involves both cyclic-dinucleotide binding and its translocation from the ER to vesicles. Activation of STING is known to induce production of type I interferons and inflammatory cytokines. By activating STING on binding to a target cell, the immune cells of the invention may induce a targeted and potent immune response to treat disease. For example, activation of STING upon binding to a target cell may result in cytotoxic T cell activation and or NK cell activation, which may mediate target cell apoptosis, in particular tumor cell apoptosis. Upon activation of STING, the pDC of the invention may recruit cytotoxic T cell and NK cells to the target cell and the disease microenvironment. Upon activation of STING, the cell of the invention may also mediate direct killing of the target cell, for example via cytotoxic activity or TRAIL-mediated killing.

An exemplary STING protein sequence is SEQ ID NO: 1 (Uniprot Q86WV6)

10 20 30 40 50

60 70 80 90 100

110 120 130 140 150

160 170 180 190 200

210 220 230 240 250

260 270 280 290 300

310 320 330 340 350

360 370


An exemplary nucleotide sequence encoding a STING protein sequence is SEQ ID NO:2 (NM_198282.4)

1 gttcattttt cactcctccc tcctaggtca cacttttcag aaaaagaatc tgcatcctgg
61 aaaccagaag aaaaatatga gacggggaat catcgtgtga tgtgtgtgct gcctttggct
121 gagtgtgtgg agtcctgctc aggtgttagg tacagtgtgt ttgatcgtgg tggcttgagg
181 ggaacccgct gttcagagct gtgactgcgg ctgcactcag agaagctgcc cttggctgct
241 cgtagcgccg ggccttctct cctcgtcatc atccagagca gccagtgtcc gggaggcaga
301 agatgcccca ctccagcctg catccatcca tcccgtgtcc caggggtcac ggggcccaga
361 aggcagcctt ggttctgctg agtgcctgcc tggtgaccct ttgggggcta ggagagccac
421 cagagcacac tctccggtac ctggtgctcc acctagcctc cctgcagctg ggactgctgt
481 taaacggggt ctgcagcctg gctgaggagc tgcgccacat ccactccagg taccgggca
541 gctactggag gactgtgcgg gcctgcctgg gctgccccct ccgccgtggg gccctgttgc
601 tgctgtccat ctatttctac tactccctcc caaatgcggt cggcccgccc ttcacttgga
661 tgcttgccct cctgggcctc tcgcaggcac tgaacatcct cctgggcctc aagggcctgg
721 ccccagctga gatctctgca gtgtgtgaaa aagggaattt caacgtggcc catgggctgg
781 catggtcata ttacatcgga tatctgcggc tgatcctgcc agagctccag gcccggattc
841 gaacttacaa tcagcattac aacaacctgc tacggggtgc agtgagccag cggctgtata

901 ttctcctccc attggactgt ggggtgcctg ataacctgag tatggctgac cccaacattc
961 gcttcctgga taaactgccc cagcagaccg gtgaccatgc tggcatcaag gatcgggttt
1021 acagcaacag catctatgag cttctggaga acgggcagcg ggcgggcacc tgtgtcctgg
1081 agtacgccac ccccttgcag actttgtttg ccatgtcaca atacagtcaa gctggcttta
1141 gccgggagga taggcttgag caggccaaac tcttctgccg gacacttgag gacatcctgg
1201 cagatgcccc tgagtctcag aacaactgcc gcctcattgc ctaccaggaa cctgcagatg
1261 acagcagctt ctcgctgtcc caggaggttc tccggcacct gcggcaggag gaaaaggaag
1321 aggttactgt gggcagcttg aagacctcag cggtgcccag tacctccacg atgtcccaag
1381 agcctgagct cctcatcagt ggaatggaaa agcccctccc tctccgcacg gatttctctt
1441 gagacccagg gtcaccaggc cagagcctcc agtggtctcc aagcctctgg actgggggct
1501 ctcttcagtg gctgaatgtc cagcagagct atttccttcc acagggggcc ttgcagggaa
1561 gggtccagga cttgacatct taagatgcgt cttgtcccct tgggccagtc atttcccctc
1621 tctgagcctc ggtgtcttca acctgtgaaa tgggatcata atcactgcct tacctccctc
1681 acggttgttg tgaggactga gtgtgtggaa gtttttcata aactttggat gctagtgtac
1741 ttagggggtg tgccaggtgt ctttcatggg gccttccaga cccactcccc acccttctcc
1801 ccttcctttg cccggggacg ccgaactctc tcaatggtat caacaggctc cttcgccctc
1861 tggctcctgg tcatgttcca ttattgggga gccccagcag aagaatggag aggaggagga
1921 ggctgagttt ggggtattga atcccccggc tcccaccctg cagcatcaag gttgctatgg
1981 actctcctgc cgggcaactc ttgcgtaatc atgactatct ctaggattct ggcaccactt
2041 ccttccctgg ccccttaagc ctagctgtgt atcggcaccc ccaccccact agagtactcc
2101 ctctcacttg cggtttcctt atactccacc cctttctcaa cggtcctttt ttaaagcaca
2161 tctcagatta

In preferred embodiments, the STING response element comprises a gain-of-function mutation that causes it to be constitutively active in the absence of 2'3'-cGAMP. As demonstrated in the Examples, use of such a mutant STING protein provides robust activation of immune responses. In preferred embodiments, the STING protein comprises a mutation, preferably a substitution, at one or more residues corresponding to R71, S102, V147, N154, V155, G166, C206, G207, G230, H232, R238, F279, R281, R284, R293, or Q315 of SEQ ID NO:1. Proteins with mutations at these residues may be constitutively active (as described in WO2020028743 Tse et al. Mol Ther. 2021 Jul 7;29(7):2227-2238) and so may be particularly useful in pDCs of the present invention. Preferred mutations at these residues are R71H, V147L, N154S, V155M, V155R, G166E, G230A, H232R, R293Q, R281M, R284M, R238M, and R293M. In more preferred embodiments, the STING proteins comprises a mutation at one or more residues selected from V147, N154 and V155. In most preferred embodiments, the STING protein comprises a mutation at residue N154 and/or V155. The preferred mutation at N154 is N154S. The preferred mutations at V155 are V155R and, more preferably V155M.

The STING protein encoded by the response element may comprise a sequence with at least 75%, 80%, 85%, 90%, 95%, 96%, 97%, 98% or 99% sequence identity to SEQ ID NO:1, across the length of SEQ ID NO:1, optionally with one or more of the mutations described in the preceding paragraph. In preferred embodiments, the STING protein comprises SEQ ID NO:1, preferably with one or more of the mutations described in the preceding paragraph. The STING protein may be truncated.

The response element encoding the STING protein may comprise a sequence with at least 75%, 80%, 85%, 90%, 95%, 96%, 97%, 98% or 99% sequence identity to SEQ ID NO:2, across the length of SEQ ID NO:2, optionally with one or more substitutions to introduce the mutations described in the paragraph above. In preferred embodiments, the response element encoding the STING protein comprises SEQ ID NO:2, preferably with one or more substitutions to introduce the mutations described in the paragraph above. The response element encoding the STING protein may be codon-optimised.

An exemplary sequence encoding STING N154 is provided as SEQ ID NO:3

### Sequence for STING N154S:

Atgccccacagctctctgcaccccagcatcccttgccccagaggccacggcgcccagaaggccgccctggtgctgctgagcgcttg
tctggtcacactgtggggactcggagaacctcctgagcacaccctgcggtacctggtgctgcatctggcatctctgcagctgggcctg
ctgctgaatggcgtgtgcagcctggccgaggaactgcggcacatccactctagatatagaggctcttattggagaaccgtgcgggcct
gcctgggatgtcctctgcggagaggcgctctgctgctctctatctacttctactactccctgcccaatgccgtcggccctccattcac
ctggatgctggctctgctgggcctgagccaggccctgaacatcctgctgggcctcaagggtctggctcctgctgaaatcagcgccgtg
tgcgagaagggcaacttcagcgtggcccacggcctggcctggtcctactacatcggctacctgaggctgattctgcctgagctgcaag
ccagaatccggacatacaaccagcactacaacaatctgctgcggggagctgtgtcccagcgcctgtacatcctgctgcctctggattg
cggcgttcctgacaacctgagcatggccgatcctaacatcagattcctggacaagctgccccagcagacaggcgaccacgccggca
tcaaggacagagtgtacagcaacagcatctacgagctgctggaaaacggccagagggccggcacctgtgtgctggaatacgccac
acctctgcagaccctgtttgccatgtcccaatacagccaagccggatttagcagagaggatagactggaacaggccaagctgttctgc
cggaccctggaggacatccttgctgacgcccctgagagccagaacaactgcagactgatcgcctaccaggagccagccgacgaca
gcagcttcagcctgtctcaggaggtgctgagacacctgagacaggaagagaaagaggaagtgaccgtgggcagcctgaagacctc
tgccgtgccctccaccagcaccatgagccaggagcccgagctgcttattagcggcatggaaaaacctctgccactgagaacagatttc
agctga

An exemplary sequence encoding STING V155M is provided as SEQ ID NO:4

### Sequence STING V155M

atgccccacagctcccttcatcctagcatcccctgccccagaggccacggcgcccagaaggccgccctggtcctgctgtctgcctgc
ctggtgaccctgtggggcctgggcgagcctccagagcacaccctgcggtacctggtgctgcacctggcatctcttcagctgggcctgt
tgcttaatggcgtgtgcagcctggccgaggaactgagacacatccactctagataccggggcagctactggcggacagttagagctt
gtctgggctgccctctgcgcagaggcgccctgctgctcctgagcatctacttctactacagcctgccaaatgccgtgggacctcctttca
cctggatgctggccctgctgggactgagccaggctctgaatatcctgctcggcctgaagggcctggctcctgctgagatcagcgccgt
gtgtgaaaaaggcaacttcaacatggcccacggcctggcctggtcctactatatcggatatctgcggctgattctgcccgagctgcaag
ccagaatccggacctacaaccagcactacaacaacctgctgagaggagctgtgtcccagcggctgtacatcctgctgcctctggactg

cggcgtgcccgacaacctgagcatggccgatcctaacatcagattcctggataagctgcctcagcagacaggcgaccacgccggca
tcaaggacagagtgtacagcaacagcatctacgagctgctggaaaacggtcagagagccggaacatgcgtgctggagtacgccac
acctctgcagaccctgttcgccatgagccaatactctcaggccggctttagccgggaagatagactggaacaggccaagctgttttgta
gaaccctcgaggacatcctggctgatgcccctgagagccagaacaactgcagactgatcgcctaccaggagcctgctgacgacagc
tcattcagcctgagccaagaggtgctgaggcacctgagacaggaggagaaggaagaagtgacagtcggctctctgaagaccagcg
ccgtgccatctaccagtaccatgtcccaggagcccgagctgctgatcagcggcatggaaaaacctctgcccctgcggaccgacttca
gctga

The response element encoding the STING protein may comprise a sequence with at least 75%, 80%, 85%, 90%, 95%, 96%, 97%, 98% or 99% sequence identity to SEQ ID NO:3 or 4, across the length of SEQ ID NO:3 or 4. In preferred embodiments, the response element encoding the STING protein comprises SEQ ID NO:3 or 4. In certain embodiments, the response element comprises a sequence with at least 75%, 80%, 85%, 90%, 95%, 96%, 97%, 98% or 99% sequence identity to SEQ ID NO:3 or 4, across the length of SEQ ID NO:3 or 4, which encodes a STING protein that additionally comprises one or more substitutions to introduce the mutations described in the paragraphs above and/or that does not comprise the N154S or V155M mutations. The codon optimisation exemplified in to SEQ ID NO:3 and 4 will be also useful for sequences encoding other substitutions.

### Plasmacytoid dendritic cells (pDCs) for treating disease

The examples demonstrate that the STING protein is particularly effective to activate a potent and targeted immune response in engineered plasmacytoid dendritic cells (pDCs) in combination with a synthetic receptor system. pDCs are a rare type of immune cell that are considered to be key in linking the innate and adaptive immune systems. They are known to secrete large quantities of type 1 interferon (IFNs) in response to a viral infection and are key effectors in cellular immunity with the ability to not only initiate immune responses but also to induce tolerance to exogenous and endogenous antigens. Plasmacytoid dendritic cells have been proposed for use in vaccines, where they present antigens and are delivered to lymph nodes to activate T-cells (Charles et al., 2020, Oncology, 9(1)). WO2018/206577 provides methods for generating populations of pDCs. WO2022/063818 describes engineered pDCs that express CAR or SynNotch constructs that activate particular immune responses in the treatment of disease.

The engineered cells for use in the invention may be plasmacytoid dendritic cells (pDCs). Plasmacytoid dendritic cells (pDCs) have a multifaceted role in the immune system, which makes them extremely adaptable for the targeted treatments of the invention. pDCs are key effectors in cellular immunity with the ability to not only initiate immune responses but also to induce tolerance to exogenous and endogenous antigens (Swiecki, and Colonna, Nat Rev Immunol, 2015. 15(8)). pDCs are distinct from conventional DCs as their final stage of development occurs within the bone marrow; their antigens are taken up by receptor-mediated endocytosis; they express high levels of interferon regulatory factor 7; and they primarily sense pathogens through toll-like receptor (TLR) 7 and 9 (Swiecki and Colonna, Nat Rev Immunol, 2015. 15(8); and Tangand Cattral, Cell Mol Life Sci, 2016). Through these pattern-recognition receptors, pathogen nucleic acids can activate pDCs to produce high levels of type I interferon (IFN). Thus, activated pDCs link the innate and adaptive immune system together via cytokine production combined with antigen-presenting cell (APC) activity. Furthermore, pDC functionality is also essential to achieve an antiviral state during infections, provide vital adjuvant activity in the context of vaccination, and for promoting immunogenic anti-tumour responses upon activation (Swiecki, and Colonna, Nat Rev Immunol, 2015. 15(8); Tovey, et al. Biol Chem, 2008. 389(5); and Rajagopal, et al. Blood, 2010. 115(10): p. 1949-57). A delicate balance must be maintained, however, as hyper-activation of pDCs has been associated with the pathogenesis of several diseases, including viral infections, autoimmune diseases and tumourigenesis (Swiecki and Colonna, Nat Rev Immunol, 2015. 15(8); and Tang and Cattral, Cell Mol Life Sci, 2016).

Preferably, the engineered pDCs express TRAIL. In another embodiment said pDCs express CD123, CD303, CD304, CD4 and/or HLA-DR. In yet another embodiment said pDCs express IFN type I, IFN type III and/or proinflammatory cytokines. In a further embodiment said pDCs express IRF7, TLR7 and/or TLR9. In one embodiment said pDCs express CD40, CD80, CD83 and/or CD86. For example, said pDCs express TRAIL, CD123, CD303, CD304, CD4, HLA-DR, IFN type I, IFN type III, IRF7, TLR7 and/or TLR9. In preferred embodiments, the pDCs of the invention express CD123, CD303 and CD304 and are negative for lineage markers and CD11c, as shown in the Examples.

In one preferred embodiment of the present invention, the pDCs express TNF-related apoptosis-inducing ligand (TRAIL). TRAIL, which is also designated CD253, is a ligand involved in killing cancer cells by interacting with TRAIL receptors (death receptor 5, DR5) on the surface of cancer cells and thereby triggering apoptosis.

The engineered pDCs of the invention are preferably matured cells having a surface phenotype that strongly resembles blood pDCs. In a preferred embodiment, said pDCs express CD123, CD303, CD304, CD4 and/or HLA-DR.

In another preferred embodiment said pDCs express IFN type I, IFN type III and/or proinflammatory cytokines.

The pDCs may in one preferred embodiment express Toll-like receptors, such as for example Toll-like receptor 7 (TLR7) and/or Toll-like receptor 9 (TLR9).

In yet another preferred embodiment said pDCs express Interferon regulatory factor 7 (IRF7).

In a preferred embodiment said pDCs secretes IL-6.

In preferred embodiments, the engineered plasmacytoid dendritic cell is capable of a type I IFN response.

In another preferred embodiment said pDCs express Cluster of differentiation 80 (CD80), which is a protein found on Dendritic cells, activated B cells and monocytes that provides a costimulatory signal necessary for T cell activation and survival.

The pDCs may in a preferred embodiment also express proteins characteristic for antigen presenting cells such as for example Cluster of Differentiation 86 (CD86) and/or Cluster of Differentiation 40 (CD40). CD86 is a protein expressed on antigen-presenting cells that provides costimulatory signals necessary for T cell activation and survival, whereas CD40 is a costimulatory protein found on antigen presenting cells and is required for their activation.

Preferably, said pDCs express CD40, CD80, CD83 and/or CD86. In another preferred embodiment said pDCs express interleukin 6 (IL-6).

In preferred embodiments, the engineered pDCs of the invention are stem cell-derived plasmacytoid dendritic cell.

In certain embodiments, pDCs are autologous. Such treatments may minimise any risk of rejection of the transferred cells. In alternative embodiments, the cells are allogenic, such as isolated from healthy donors. Such treatments can potentially be prepared more quickly and offered "off the shelf". In certain embodiments, the cells are or have been cryopreserved. Moreover, the cells may be xenogeneic.

### Methods for treating disease

The invention provides the engineered cell of the invention for use in a method of treating a disease in a subject comprising administering an engineered cell to the subject. Therefore, the invention provides a new adoptive cell therapy. Adoptive cell therapy is the transfer of *ex vivo* grown cells, most commonly immune-derived cells, into a host with the goal of transferring the immunologic functionality and characteristics of the transferred cells. Adoptive cell therapy is well established for treating cancer and autoimmune, inflammatory and infectious diseases, albeit using different transferred immune cells with different immune-regulating effects and activities.

The methods of treatment may comprise (i) collecting autologous hematopoietic stem progenitor cells (HSPCs), either from the subject to be treated or a healthy donor; (ii) preparing engineered pDCs, for example using a method discussed below; (iii) optionally administering to the subject lymphodepleting chemotherapy; and (iv) administering to the subject the engineered pDCs.

The methods may comprise administering cells expressing more than one exogenous construct. Individual cells may express more than one construct, or the population of cells administered may comprise a plurality of different cells.

The engineered cells may be further modified to express immune-modulatory proteins, such as cytokines (e.g., IL-2, IL-12 or IL-15), which may stimulate T-cell activation and recruitment, and may thus aid in combating the tumour microenvironment. Thus, the cells may comprise a population of cells expressing the exogenous construct and further expressing an immune modulatory protein such as, for example, IL-2, IL-12, or IL-15.

The engineered cells may be isolated from a subject and used fresh, or frozen for later use, in conjunction with (e.g., before, simultaneously or following) lymphodepletion.

The engineered cells may be administered to the subject by dose fractionation, wherein a first percentage of a total dose is administered on a first day of treatment, a second percentage of the total dose is administered on a subsequent day of treatment, and optionally, a third percentage of the total dose is administered on a yet subsequent day of treatment.

An exemplary total dose comprises 10³ to 10¹¹ cells/kg body weight of the subject, such as 10³ to 10¹⁰ cells/kg body weight, or 10³ to 10⁹ cells/kg body weight of the subject, or 10³ to 10⁸ cells/kg body weight of the subject, or 10³ to 10⁷ cells/kg body weight of the subject, or 10³ to 10⁶ cells/kg body weight of the subject, or 10³ to 10⁵ cells/kg body weight of the subject. Moreover, an exemplary total dose comprises 10⁴ to 10¹¹ cells/kg body weight of the subject, such as 10⁵ to 10¹¹ cells/kg body weight, or 10⁶ to 10¹¹ cells/kg body weight of the subject, or 10⁷ to 10¹¹ cells/kg body weight of the subject.

An exemplary total dose may be administered based on a patient body surface area rather than the body weight. As such, the total dose may include 10³ to 10¹³ cells per m².

The methods may comprise lymphodepletion. Lymphodepletion may be achieved by any appropriate means. Lymphodepletion may be performed prior to administration of the engineered cells, or subsequent to. Lymphodepletion may be performed both before and after administration of the engineered cells.

The methods may comprise administration of one or more additional therapeutic agents. Exemplary therapeutic agents include a chemotherapeutic agent, an anti-inflammatory agent, an immunosuppressive, an immunomodulatory agent, or a combination thereof.

Therapeutic agents may be administered according to any standard dose regime known in the field. Exemplary chemotherapeutic agents include anti-mitotic agent, such as taxanes, for instance docetaxel, and paclitaxel, and vinca alkaloids, for instance vindesine, vincristine, vinblastine, and vinorelbine. Exemplary chemotherapeutic agents include a topoisomerase inhibitor, such as topotecan. Exemplary chemotherapeutic agents include a growth factor inhibitor, a tyrosine kinase inhibitor, a histone deacetylase inhibitor, a P38a MAP kinase inhibitor, inhibitors of angiogenesis, neovascularization, and/or other vascularization, a colony stimulating factor, an erythropoietic agent, an anti-anergic agents, an immunosuppressive and/or immunomodulatory agent, a virus, viral proteins, immune checkpoint inhibitors, BCR inhibitors (e.g., BTK, P13K, etc.), immune-metabolic agents (e.g., IDO, arginase, glutaminase inhibitors, etc.), and the like. The one or more therapeutic agents may comprise an antimyeloma agent. Exemplary antimyeloma agents include dexamethasone, melphalan, doxorubicin, bortezomib, lenalidomide, prednisone, carmustine, etoposide, cisplatin, vincristine, cyclophosphamide, and thalidomide, several of which are indicated above as chemotherapeutic agents, anti-inflammatory agents, or immunosuppressive agents.

Treatment of a disease, according to the invention, refers to a biological effect that may present as a decrease in disease burden, disease incidence or disease severity. For example, in relation to cancer treatment, this may manifest as a reduction in tumour volume, a decrease in the number of tumour cells, a decrease in tumour cell proliferation, a decrease in the number of metastases, an increase in overall or progression-free survival, an increase in life expectancy, or amelioration of various physiological symptoms associated with the tumour.

The engineered cells of the invention may be administered by any appropriate route. Generally, the cells will be administered by intravenous infusion,

### Methods for treating cancer

In preferred embodiments of the invention, the cells of the invention are for use in treating cancer. The ability of immune cells such as pDCs to secrete immunomodulatory factors and alter immune-inhibiting tumour microenvironments, and the potent effects of STING, are expected to be particularly useful for treating cancer. Also, cancer cells present specific antigens that can be recognised by the extracellular antigen recognition domain to provide targeted therapy at tumour sites, avoiding healthy cells.

In preferred embodiments, the cancer is a solid tumour. The cells of the invention may be particular effective at targeting and altering the microenvironment of solid tumours. Accordingly, in certain embodiments, the cancer is a solid cancer, such as a cancer selected from the group consisting of: bone cancer, breast cancer, pancreatic cancer, skin cancer, cancer of the head or neck, cutaneous or intraocular malignant melanoma, uterine cancer, ovarian cancer, prostate cancer, rectal cancer, cancer of the anal region, colon cancer, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, pediatric tumors, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, glioblastoma, pituitary adenoma, Kaposi's sarcoma, epidermoid cancer and squamous cell cancer.

In certain embodiments, the cells of the invention are for use in treating acute lymphoblastic leukemia (ALL) (including non T cell ALL), acute myeloid leukemia, B cell prolymphocytic leukemia, B-cell acute lymphoid leukemia ("BALL"), blastic plasmacytoid dendritic cell neoplasm, Burkitt s lymphoma, chronic lymphocytic leukemia (CLL), chronic myelogenous leukemia (CML), chronic myeloid leukemia, chronic or acute leukemia, diffuse large B cell lymphoma (DLBCL), follicular lymphoma (FL), hairy cell leukemia, Hodgkin's Disease, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome, non-Hodgkin's lymphoma (NHL), plasma cell proliferative disorder (including asymptomatic myeloma (smoldering multiple myeloma or indolent myeloma), plasmablastic lymphoma, plasmacytoid dendritic cell neoplasm, plasmacytomas (including plasma cell dyscrasia; solitary myeloma; solitary plasmacytoma; extramedullary plasmacytoma; and multiple plasmacytoma), POEMS syndrome (also known as Crow-Fukase syndrome; Takatsuki disease; and PEP syndrome), primary mediastinal large B cell lymphoma (PMBC), small cell- or a large cell-follicular lymphoma, splenic marginal zone lymphoma (SMZL), systemic amyloid light chain amyloidosis, T-cell acute lymphoid leukemia ("TALL"), T-cell lymphoma, transformed follicular lymphoma, or Waldenstrom macroglobulinemia, or a combination thereof. In some embodiments, the cancer is a myeloma. In one particular embodiment, the cancer is multiple myeloma. In some embodiments, the cancer is leukemia. In some embodiments, the cancer is acute myeloid leukemia. In some embodiments, the cancer is relapsed or refractory large B-cell lymphoma, diffuse large B-cell lymphoma (DLBCL) not otherwise specified, primary mediastinal large B-cell lymphoma, high grade B-cell lymphoma, or DLBCL arising from follicular lymphoma.

The extracellular antigen recognition domain in the exogenous construct is selected to bind an antigen expressed on the surface of the cancer to be treated. Exemplary extracellular antigen recognition domains are described above. In preferred embodiments, the subject has a HER-2 positive cancer, and the synthetic receptor is an anti-HER SNIPR.

The methods further may comprise administering a chemotherapeutic. The chemotherapeutic selected may be a lymphodepleting (preconditioning) chemotherapeutic, and is preferably administered before the cells of the invention. Such administration of a chemotherapeutic may improve survival of the transplanted cells.

In preferred embodiments, the cell of the invention is for use in treating cancer and the cell is an engineered stem cell-derived plasmacytoid dendritic cell.

In the therapeutic methods using the engineered cells of the invention, engineered cells are administered to a subject already suffering from cancer, in an amount sufficient to cure, alleviate or partially arrest the cancer or one or more of its symptoms. Such therapeutic treatment may result in remission, stabilisation, reduction in metastasis or elimination of the cancer. An amount adequate to accomplish this is defined as "therapeutically effective amount". The subject may have been identified as suffering from cancer and being suitable for an adoptive cell transfer immunotherapy by any suitable means.

### Methods for treating autoimmune and inflammatory diseases

In preferred embodiments of the invention, the cells of the invention are for use in treating an autoimmune or inflammatory disease. The ability of immune cells such as pDCs to secrete immunomodulatory factors and alter inflammatory tissues or sites of autoimmune attack are expected to be particularly useful for treating autoimmune and inflammatory diseases.

Targeting engineered pDCs to inflamed tissue and sites of inflammatory disease is expected to be effective because depletion of pDCs (in a murine asthma model) leads to T cell-mediated hyper-responsiveness, resulting in breakdown of tolerance (see Jan de Heer, 2004, J Exp Med, 200(1):89-98). pDCs are also important for long-term graft survival after allogeneic stem cell transplantation (see Peric et al. Biol Blood Marrow Transplant, 2015, 21(8), Gongalves et al. Biol Blood Marrow Transplant, 21(7), and Waller et al. J Clin Oncol, 2014, 32(22)). Also, high expression of PD-L1/CD86 in pDCs correlate with increased T-regs in liver transplant tolerance (see Tokita et al., Transplantation. 2008, 85(3):369-77). Also, adoptive transfer (pre-operative) of donor pDCs highly promotes heart transplant survival (see PMID: Björck et al. J Heart Lung Transplant, 2005, 24(8) and Abe et al., Am J Transplant, 2005, 5(8)).

In certain embodiments, the autoimmune disease is selected from the list consisting of: type 1 diabetes, thyroid autoimmune diseases (e.g. Hashimoto's and Graves'), Addison's adrenal insufficiency, oophoritis, orchitis, lymphocytic hypophysitis, autoimmune hypoparathyroidism, autoimmune hypoparathyroidism, Goodpasture's disease, autoimmune myocarditis, membranous nephropathy, autoimmune hepatitis, ulcerative colitis, Crohn's disease, multiple sclerosis, myasthenia gravis, neuromyelitis optica.

In certain embodiments, the inflammatory disease is selected from the list consisting of: cystic fibrosis, chronic inflammatory intestinal diseases like, for example, ulcerative colitis or Crohn's disease, vasculitis, in particular Kawasaki disease, chronic bronchitis, inflammatory arthritis diseases like, for example, psoriatic arthritis, osteoarthritis, rheumatoid arthritis, and systemic onset juvenile rheumatoid arthritis (SOJRA, Still's disease), graft-versus-host disease, asthma, psoriasis, systemic lupus erythematosus and allograft rejection.

In certain embodiments, the autoimmune or inflammatory disease is transplant rejection or graft-versus-host-disease (GVHD).

The extracellular antigen recognition domain in the exogenous construct is selected to target an antigen expressed on the surface of immune cells that cause autoimmune or inflammatory disease.

In preferred embodiments, the cell of the invention is for use in treating an autoimmune or inflammatory disease and the cell is an engineered stem cell-derived plasmacytoid dendritic cell that expresses a synthetic receptor comprising an extracellular antigen recognition domain that recognises an antigen on a target cell, a transmembrane domain, and an intracellular signalling domain that activates expression of a response element in the cell when the extracellular antigen recognition domain binds the target cell, wherein the response element encodes a stimulator of interferon genes (STING) protein.

In the therapeutic methods using the cells of the invention, engineered cells are administered to a subject already suffering from an autoimmune disease, in an amount sufficient to cure, alleviate or reduce the frequency of one or more symptoms. An amount adequate to accomplish this is defined as "therapeutically effective amount". The subject may have been identified as suffering from an autoimmune disease and being suitable for an adoptive cell transfer immunotherapy by any suitable means.

### Methods for treating infectious diseases

In preferred embodiments of the invention, the cells of the invention are for use in treating an infectious disease. The ability of immune cells such as pDCs to secrete immunomodulatory factors and alter immune-inhibiting disease microenvironments are expected to be particularly useful for treating infectious diseases.

The cells of the invention are expected to be particularly useful for treating chronic infections. In preferred embodiments, the infectious disease is a chronic viral or fungal infection, such as infection of Influenza, Yellow Fever virus, West Nile virus, Hantavirus, Ebola virus, Rotavirus, Norovirus, Rabies virus, Tick-borne encephalitis virus (TBEV), rhinoviruses, Coronavirus, RSV, Measles, Parainfluenza, Zikavirus, Dengue virus, HIV, HBV, HCV, human cytomegalovirus (CMV), Epstein-Barr virus (EBV), *Aspergillus spp.,* such as *Aspergillus fumigatus, Candida spp*., such as *C*. *albicans, Mucorales, Cryptococcus, spp.,* such as *Cryptococcus neoformans* or *Cryptococcus gattii*, or *Pneumocystis jirovecii*.

In methods of treating infectious disease, the cell will generally express a synthetic receptor comprising an extracellular antigen-binding domain which recognises a pathogen antigen. The cell will target the pathogen via the antigen and activate an immune response to clear the infectious agent. In certain embodiments, the antigen is selected from: Yellow Fever virus NS1 protein, West Nile virus NS1 protein, Hantavirus N protein, Ebola virus N protein, Rotavirus VP6, Norovirus VP1, rabies virus N protein, TBEV envelope glycoprotein, rhinovirus VP proteins, *Influenza* hemagglutinin antigens, *Influenza* neuraminidase antigens, coronavirus spike protein, RSV F protein, MeV N protein, *Parainfluenza* hemagglutinin antigens, *Parainfluenza* neuraminidase antigens, ZIKV E, NS1, NS3, NS4B, and NS5 proteins, Dengue virus C protein, M protein, E protein, and NS1 protein, gp120, gp41, Env, HBV surface antigen, HBV-surface proteins S and L, HCV E2 glycoprotein, CMV glycoprotein B, fungal beta glucan.

In the therapeutic methods using the cells of the invention, engineered cells are administered to a subject already suffering from an infectious disease, in an amount sufficient to cure, alleviate or reduce the frequency of one or more symptoms and/or in an amount sufficient to reduce infection load. An amount adequate to accomplish this is defined as "therapeutically effective amount". The subject may have been identified as suffering from an infectious disease and being suitable for an immunotherapy by any suitable means.

### Methods for generating cells of the invention

The engineered cells may be generated by any appropriate method. Exemplary methods for generating pDCs in significant amounts are provided in WO2018/206577. The invention also provides methods of generating engineered plasmacytoid dendritic cells.

The methods of the invention may further comprise a step of producing the pDCs.

In certain embodiments, the method for producing an engineered plasmacytoid dendritic cell (pDCs) comprises:
- providing hematopoietic stem progenitor cells (HSPCs)
- transfecting or transducing said HSPCs with a vector encoding a synthetic receptor as described herein and a vector encoding a response element as described herein, optionally wherein a single vector encodes both elements,
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs.

In certain embodiments, the method for producing an engineered plasmacytoid dendritic cell (pDCs) comprises:
- providing hematopoietic stem progenitor cells (HSPCs),
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs,
- transfecting or transducing said pDCs with a vector encoding a synthetic receptor as described herein and a vector encoding a response element as described herein, optionally wherein a single vector encodes both elements.

Accordingly, in certain embodiments, the method for producing an engineered plasmacytoid dendritic cell (pDCs) comprises:
- providing hematopoietic stem progenitor cells (HSPCs),
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs, and
- transfecting or transducing said HSPCs prior to differentiation, or transfecting or transducing said pDCs subsequent to differentiation, with a vector encoding a synthetic receptor as described herein and a vector encoding a response element as described herein, optionally wherein a single vector encodes both elements.

Transfecting or transducing the cells can be achieved by any appropriate technique.

The vector may be an appropriate vector, such as selected from the group consisting of a viral construct, an mRNA, a plasmid or a cosmid.

In some embodiments the vector is a viral construct. In some embodiments, the viral construct is an AAV construct, an adenoviral construct, a lentiviral construct, or a retroviral construct. The construct may comprise a reporter gene such as GFP, mCherry, truncated EGFR, or truncated tNGFR, or the extracellular domain of the synNotch may contain an epitope, to aid sorting of pDCs with the construct. In some embodiments, the viral construct is a lentiviral construct and transduction is performed using retronectin-coated plates or using lentiboost and protamine sulfate. In preferred embodiments of the invention, the method includes the step of transducing the HSPCs with a viral construct before the step of differentiating the HSPCs into pDCs. pDCs differentiated from HSPCs transduced with a viral construct encoding an antigen may stably express the antigen.

In some embodiments, the vector is an mRNA encoding an antigen. The mRNA may be delivered to the cell using any appropriate technique, such as electroporation or using a lipid nanoparticle (LNP). In a preferred embodiment, the step of transfecting the pDCs with an LNP comprising an mRNA encoding an antigen occurs after the step of differentiating the HSPCs into pDCs. pDCs transfected with an LNP comprising an mRNA encoding an antigen may transiently express the antigen.

In certain embodiments, the heterologous nucleic acid is introduced using a site-specific DNA editing such as TALEN, zinc finger or CRISPR/Cas.

In preferred embodiments, CD34+ HSPC are transfected or transduced and then differentiated into pDCs.

In certain embodiments, the method for producing an engineered plasmacytoid dendritic cell (pDCs) comprises:
- providing hematopoietic stem progenitor cells (HSPCs)
- transfecting or transducing said HSPCs with a vector encoding a synthetic receptor as described herein and a vector encoding a response element as described herein, optionally wherein a single vector encodes both elements,
- incubating said HSPCs in a first medium comprising cytokines and growth factor whereby said HSPCs are differentiated into precursor-pDCs
- adding interferons (IFNs) to said first medium to obtain a second medium whereby said precursor- pDCs are differentiated into pDCs

In certain embodiments, the method for producing an engineered plasmacytoid dendritic cell (pDCs) comprises:
- providing hematopoietic stem progenitor cells (HSPCs)
- transfecting or transducing said HSPCs with a vector encoding a synthetic receptor as described herein and a vector encoding a response element as described herein, optionally wherein a single vector encodes both elements,
- incubating said HSPCs in a first medium comprising cytokines and growth factor whereby said HSPCs are differentiated into precursor-pDCs
- adding stem cell factor (SCF) and an aryl hydrocarbon receptor antagonist (such as stemreginin-1) in a first medium to obtain high yield of pre-cursor pDCs
- providing a second medium comprising interferons (IFNs)
- adding interferons (IFNs) to said a second medium to said first medium comprising pre-cursor pDCs, whereby said precursor- pDCs are transformed into to obtain a high yield of fully activated and differentiated pDCs a second medium
whereby said precursor- pDCs are differentiated into pDCs

In some embodiments, the invention uses pDCs extracted from blood or bone marrow. Any appropriate technique may be used for isolating pDCs from blood or bone marrow. Such cells may be transfected with a vector comprising an expression cassette comprising a transgene encoding the at least one antigen or receptor using any appropriate method.

In some embodiments, the invention uses pDCs derived from induced pluripotent stem cells (iPSCs) or embryonic stem cells (ESCs). Any appropriate technique may be used for differentiating iPSCs and ESCs into HSPCs and into pDCs. Exemplary protocols are provided herein for differentiating HSPCs into pDCs. The production of DCs from ESCs and iPSCs are disclosed in, for example, Li et al. World J Stem Cells. 2014 Jan 26; 6(1): 1-10 and the production of HSPCs from ESCs and iPSCs are disclosed in, for example, Tan et al., Proc Natl Acad Sci U S A. 2018; 115(9):2180-2185, Tursky et al. Stem Cell Reports. 2020;15(3):735-748 and Demirci et al. Stem Cell Res Ther. 2020;11(1):493. Similar processes may be used to generate pDCs.

The pDCs comprise one or more heterologous nucleic acids encoding the response element and optionally the synthetic receptor. In some embodiments, the heterologous nucleic acid is integrated into the genome of the engineered cell. In some embodiments, the heterologous nucleic acid is not integrated into the genome of the engineered cell. In some embodiments, the heterologous nucleic acid is introduced by a transposase, retrotransposase, episomal plasmid, mRNA, or random integration. In certain embodiments, the heterologous nucleic acid is introduced with a gene editing system such as TALEN, zinc finger or CRISPR/Cas9.

In preferred embodiments said second medium comprises IFN-γ and/or IFN-β. In another embodiment said second medium further comprises IL-3. Preferably, said second medium comprises IL-3, IFN-γ and IFN-β.

The precursor-pDCs may for example be incubated for at least 24 hours in said second medium. Said precursor-pDCs may be incubated for 24 to 72 hours in said second medium. Preferably, said precursor pDCs are incubated for around 24 hours, such as 20-28, 22-26 or 24 hours.

In one embodiment said first medium comprises Flt3 ligand, thrombopoietin and/or interleukin-3. In another embodiment said first medium further comprises stem cell factor and StemRegenin 1. In another embodiment said first medium further comprises stem cell factor and UM 171. In another embodiment said first medium further comprises RPMI medium supplemented with fetal calf serum (FCS). In another embodiment said first medium comprises serum-free medium (SFEM). Preferably, said first medium comprises Flt3 ligand, thrombopoietin, SCF, interleukin-3 and StemRegenin 1.

The HSPCs may for example be incubated for 21 days in said first medium.

In one embodiment the method as described herein, further comprises a step of immunomagnetic negative selection to enrich for differentiated pDCs.

Before differentiation of HSCs into precursor-pDCs, the HSCs may be cultured in a culture medium not comprising differentiation factors. The culture medium may be supplemented with conventional cell culture components such as serum, such as for example fetal calf serum, b-mercaptoethanol, antibiotics, such as penicillin and/or streptomycin, nutrients, and/or nonessential amino acids. Conventional cell culture components can also be substituted for conventional serum-free medium supplemented with conventional penicillin and/or streptomycin.

"Hematopoietic stem cells" (HSCs) as used herein are multipotent stem cells that are capable of giving rise to all blood cell types including myeloid lineages and lymphoid lineages. Myeloid lineages may for example include monocytes and macrophages, neutrophils, basophils, eosinophils, erythrocytes, megakaryocytes/platelets and dendritic cells, whereas lymphoid lineages may include T-cells, B-cells and NK-cells.

In a preferred embodiment HSCs are Hematopoietic stem and progenitor cells (HSPCs) that are positive for the marker CD34. HSCs or HSPCs are found in the bone marrow of humans, such as in the pelvis, femur, and sternum. They are also found in umbilical cord blood and in peripheral blood.

Stem and progenitor cells can be taken from the pelvis, at the iliac crest, using a needle and syringe. The cells can be removed as liquid for example to perform a smear to look at the cell morphology or they can be removed via a core biopsy for example to maintain the architecture or relationship of the cells to each other and to the bone.

The HSCs or HSPCs may also be harvested from peripheral blood. To harvest HSCs or HSPCs from the circulating peripheral blood, blood donors can be injected with a cytokine that induces cells to leave the bone marrow and circulate in the blood vessels. The cytokine may for example be selected from the group consisting of granulocyte-colony stimulating factor (G-CSF), GM-CSF granulocyte-macrophage colony-stimulating factor (GM-CSF) and cyclophosphamide. They are usually given as an injection into the fatty tissue under the skin every day for about 4-6 days.

The HSCs or HSPCs may also be harvested or purified from bone marrow. Stem cells are 10-100 times more concentrated in bone marrow than in peripheral blood. The hip (pelvic) bone contains the largest amount of active marrow in the body and large numbers of stem cells. Harvesting stem cells from the bone marrow is usually done in the operating room.

HSCs or HSPCs may also be purified from human umbilical cord blood (UCB). In this method, blood is collected from the umbilical cord shortly after a baby is born.

The first medium is a differentiation medium, wherein HSCs are differentiated into precursor-pDCs. Thus, the first medium comprises differentiation factors.

Before differentiation of HSCs into precursor-pDCs, the HSCs may be cultured in a culture medium not comprising differentiation factors. The culture medium may be supplemented with conventional cell culture components such as serum, such as for example fetal calf serum, b-mercaptoethanol, antibiotics, such as penicillin and/or streptomycin, nutrients, and/or nonessential amino acids. Conventional cell culture components can also be substituted for conventional serum-free medium supplemented with conventional penicillin and/or streptomycin.

To initiate differentiation of HSCs into precursor-pDCs, differentiation factors, such as Flt3 ligand, thrombopoietin and/or at least one interleukin selected from interleukin-3, IFN-b and PGE2 are added to the medium. SCF and/or an aryl hydrocarbon receptor antagonist (such as stemreginin-1) can also be used.

Thus, in a preferred embodiment said first medium comprises Flt3 ligand, thrombopoietin and/or at least one interleukin selected from interleukin-3, IFN-b and PGE2. More preferably, said first medium comprises Flt3 ligand, thrombopoietin and/or interleukin-3. In another preferred embodiment, the first medium comprises SCF and/or an aryl hydrocarbon receptor antagonist (such as stemreginin-1).

Appropriate culture media can be prepared by the skilled person, for example using the guidance in WO 2018/206577.

The HSPCs are incubated in the first medium under conditions that are typical for human cell cultures and well known to the skilled person. Typical conditions for incubation of cell cultures are for example a temperature of 37 °C, 95% humidity and 5% CO₂.

In one embodiment the HSPCs are incubated for at least 1 day, such as at least 2 days, at least 3 days, such as for example at least 4 days, such as at least 5 days, at least 6 days, such as for example at least 7 days, such as at least 8 days, at least 9 days, such as for example at least 10 days, such as at least 12 days, at least 14 days in said first medium. In a more preferred embodiment the culture is incubated for at least 16 days, such as at least 18 days, at least 20 days or such as for example at least 21 days in said first medium. In a further preferred embodiment, the culture is incubated for at least 7 days in said first medium. In a further preferred embodiment, the culture is incubated for at least 1 day in said first medium.

The HSCs may for example be incubated for 1 week, 2 weeks, 3 weeks or 4 weeks in said first medium. In a preferred embodiment said HSPCs are incubated for 21 days in said first medium.

In one embodiment the first medium is refreshed during the incubation period. The medium may for example be refreshed every second day, every third day or every fourth day during the incubation period. The first medium is preferably refreshed with medium containing one or more components of the first medium as described herein and above. Preferably the medium is refreshed with medium comprising the cytokines.

After incubation of HSPCs in the first medium, wherein HSCs are differentiated into precursor-pDCs, IFNs are added to the first medium thereby obtaining a second medium.

Alternatively, a second medium is provided, which comprises IFNs, such as IFN type I, IFN type II and/or IFN type III.

In one embodiment said second medium comprises IFN-α, IFN-γ and/or IFN-β.

In one preferred embodiment said second medium comprises IFN-γ and/or IFN-β. Preferably, said second medium comprises IFN-γ and IFN-β.

In another preferred embodiment said second medium comprises interleukin-3 (IL-3). In the embodiment, wherein the first medium comprises IL-3, IL-3 may be added to the medium again, for example together with the interferons. It is understood that the three components can be added in any order. In a particular preferred embodiment said second medium comprises IFN-y, IFN-β and IL-3.

The precursor-pDCs are incubated in the second medium under conditions that are typical for human cell cultures and well known to the skilled person. Typical conditions for incubation of cell cultures are for example a temperature of 37 °C, 95% humidity and 5% CO₂.

In one embodiment said precursor-pDCs are incubated in said second medium for at least 1 hour, such as at least 5 hours, such as for example at least 10 hours, such as at least 15 hours or such as at least 20 hours in said second medium. In one preferred embodiment precursor-pDCs are incubated for at least 24 hours in said second medium.

In another embodiment said precursor-pDCs are incubated in said second medium for at least 1 day, at least two days, at least three days or at least 4 days.

In some embodiments, the methods of the invention include a step of priming the pDCs before administration. In some embodiments, the step of priming the pDCs comprises incubating the pDCs with type I IFN and/or type II IFN. The pDCs may be incubated with the type I IFN and/or type II IFN for at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours or at least 24 hours. In a preferred embodiment, the pDCs are incubated with the type I IFN and/or type II IFN for 24 hours. The step of priming the pDCs may increase the level of IFNα expressed by the pDCs. This may improve the ability of the pDCs to activate and/or pre-condition an adoptive cell transfer immunotherapy.

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

Unless specifically prohibited, the steps of a method disclosed herein may be performed in any appropriate order and the order in which the steps are listed should not be considered limiting.

### Examples

### Example 1

Hematopoietic stem and progenitor cells (HSPCs) were transduced with two lentiviral vectors, each encoding one of the SynNotch components; one construct encoding the anti-CD19 scFv SynNotch receptor, and one encoding the STING gain-of-function (STING N154S or STING V155M) response element (Figure 4a-c). Subsequently, cells were differentiated into U-pDCs. Figure 8a shows representative FACS plots, showing the expression of the anti-CD19 scFv SynNotch receptor (FMC63) and the STING N154S SynNotch response element, or the STING V155M SynNotch response element (eGFP) 6-days post transduction (Mock = cells that did not receive any of the two lentiviral vectors). At the end of pDC differentiation, U-pDCs were primed for one day, and a pDC phenotyping was performed on cells. Figure 8bc presents representative FACS plots showing the phenotype of primed (IFN) or non-primed (IL-3) U-pDCs transduced with the antiCD19 SynNotch receptor and SynNotch STING N154S (b), or SynNotch STING V155M (c) response element. U-pDCs were found to display a pDC phenotype, with no expression of the conventional dendritic cell marker CD11c (data not shown), and high levels of the pDC marker CD303 upon priming. U-pDCs were found to still express the SynNotch components, with ~20% of the pDCs being double positive for both constructs. This example shows that it is possible to express the SynNotch components in U-pDCs.

### Example 2

Primed (IFN) or non-primed (IL-3) U-pDCs, where 20% of the cells express the antiCD19 SynNotch and STING N154S response element, or STING V155M response element, or U-pDCs not expressing the construct (mock), were co-cultured with CD19 positive target cells (NALM6) or CD19 negative non-target cells (K562) at an effector:target ratio of 1:4 or 1:8. Supernatants were subsequently collected 24 hours later, and bioactive type I IFN (Figure 8d) or CXCL10 (Figure 8e) were evaluated using a commercially-available type I IFN bioassay (HEK-blue Type I IFN reporter bioassay), or a CXCL10 ELISA, respectively. The data in Figure 8d-e show that the SynNotch STING U-pDCs specifically recognize and respond to the target cell line, and subsequently become activated and produce type I IFN and CXCL10. Data are representative of one donor. This example shows that expression of the SynNotch system components in U-pDCs allows the U-pDCs to recognize target cells, resulting in a type I IFN and CXCL10 response.

### Example 3

Hematopoietic stem and progenitor cells (HSPCs) were transduced with one 'all-in-one' lentiviral vector encoding both the SynNotch receptor and the STING N154S response element, or STING V155M response element in (Figure 5a-b). Next, HSPCs were differentiated into U-pDCs. During pDC differentiation, the population was split in two where one population was purified for SynNotch positive cells by positive immunomagnetic selection and the other population was left unsorted. After pDC differentiation, U-pDCs were either cryopreserved or primed for experiments (Figure 9a). 6-days post transduction (before splitting the culture in to two populations) 50.2% and 55.1% of cells were found to express the SynNotch components for STING N154S and STING V155M, respectively (Figure 9b). Next, the populations were split in to two, and in one population SynNotch-positive cells were purified by staining with anti-FMC63-PE, followed by positive immunomagnetic selection for PE (Figure 9c). After the selection, 88.0% and 88.9% of cells were positive for the SynNotch components for STING N154S and STING V155M, respectively (Figure 9d). This example shows that it is possible to transduce HSPCs with an 'all-in-one' lentiviral vector that encodes both the SynNotch receptor and the SynNotch STING response element, allowing expression of both components of the SynNotch system in cells. This further allows purification of SynNotch positive cells using positive selection.

### Example 4

Subsequent to the experiments outlined in Example 3, lentiviral vector-transduced HSPCs were differentiated into pDCs. A similar proliferation was observed for cells transduced with the STING V155M construct as non-transduced cells (mock) (Figure 9e and Figure 9g). This was also evident for cells that had been immunomagnetically selected for FMC63 expression, showing that neither expression of STING V155M nor sorting of cells affected proliferation of cells during pDC differentiation (Figure 9e-g). Proliferation of cells expressing the SynNotch STING N154S was slightly reduced (Figure 9e-f). After the pDC differentiation, pDCs were primed (IFN) or left non-primed (IL-3) for 24 hrs. Next, pDCs were phenotyped based on the expression of lineage markers, CD11c, CD123, CD303, and FMC63. SynNotch STING V155M U-pDCs were found to show a similar phenotype as non-transduced U-pDCs (mock), being negative for lineage markers and CD11c, and expressing CD123 and CD303 upon IFN priming (Figure 9h-i). SynNotch STING N154S U-pDCs were found to display an affected pDC phenotype with high expression of lineage and CD11c, and low expression of CD123 and CD303 (Figure 9j). Next, SynNotch U-pDCs were co-cultured with CD19 positive target cells (NALM6 or REH6), or CD19 negative non-target cells (K562) at an effector:target ratio of 1:8. Supernatants were collected 24 hours later and type I IFN was evaluated using a commercially available type I IFN bioassay (HEK-blue Type I IFN reporter bioassay). The data show in figure 9k that the U-pDCs expressing the 'all-in-one' construct with both SynNotch system components specifically recognize the target cell lines NALM6 and REH6, and subsequently become activated and produce type I IFN (Figure 9k).

### Example 5

Hematopoietic stem and progenitor cells (HSPCs) were transduced with one 'all-in-one' lentiviral vector encoding the SynNotch receptor and the STING V155M response element (Figure 5b). Next, HSPCs were differentiated into U-pDCs. During pDC differentiation SynNotch positive cells were purified by immunomagnetic positive selection. After pDC differentiation, U-pDCs were either cryopreserved or primed for experiments. The setup was conducted using three individual donors. Transduction or sorting of SynNotch positive cells did not affect proliferation of HSPCs or differentiation into U-pDCs (Figure 10a-c). An average transduction efficiency of 54% was obtained, and after purification a purity of 93.9% SynNotch positive cells was achieved (Figure 10d). The level of SynNotch receptor expression was found to be retained during differentiation and priming of U-pDCs. Next, SynNotch U-pDCs or non-transduced U-pDCs (mock) were primed with IFN (IFN) or left non-primed (IL-3). Phenotypic analysis of the U-pDCs showed a similar phenotype, with U-pDCs being negative for lineage markers and CD11c and expressing the pDC markers CD123, CD303 and CD304 at high levels (Figure 10e).

### Example 6

Primed (IFN) or non-primed (IL-3) U-pDCs expressing the 'all-in-one' anti-CD19 SynNotch with STING V155M response element, or U-pDCs not expressing the construct (mock), were next co-cultured with CD19 positive target cells (NALM6 or REH6), or CD19 negative non-target cells (K562) at an effector:target ratio of 1:8. Supernatants were collected 24 hours later and levels of type I IFN was evaluated using a commercially available type I IFN bioassay (HEK-blue Type I IFN reporter bioassay) (Figure 10f). The data in Figure 10g-h show that SynNotch STING V155M U-pDCs specifically recognize the target cell lines NALM6 and REH6, and subsequently become activated to produce type I IFN. The response is found to be higher in U-pDCs that have undergone sorting for the SynNotch receptor during pDC differentiation versus unsorted cells (Figure 10g-h). The production of type I IFN in U-pDCs upon SynNotch activation occurs independent of priming of the U-pDCs.

### Example 7

Anti-CD19 SynNotch STING V155M U-pDCs, or U-pDCs not expressing the construct (mock), were activated with the TLR7 agonist R837, or the STING agonist 2'3'-cGAMP. 24 hours later, supernatants were collected, and type I IFN was analyzed using a commercially available type I IFN bioassay (HEK-blue Type I IFN reporter bioassay). The data in Figure 10i-j show that SynNotch STING V155M U-pDCs display a similar induction of type I IFN as the mock U-pDCs upon agonist stimulation, indicating expression of the SynNotch components in U-pDCs does not affect the differentiation or function of U-pDCs. A similar production of type I IFN is observed for SynNotch U-pDCs that have been sorted for the SynNotch receptor during U-pDC differentiation versus unsorted cells, indicating that the sorting process do not affect U-pDC differentiation, or function of U-pDCs.

### Example 8

Anti-CD19 SynNotch STING V155M U-pDCs, or U-pDCs not expressing the construct (mock), were activated with 2'3'-cGAMP or anti-c-Myc magnetic beads. 24 hours later, supernatants were collected, and type I IFN was analyzed using a commercially available type I IFN bioassay (HEK-blue Type I IFN reporter bioassay). The data in Figure 10k show that SynNotch STING V155M U-pDCs can be activated using anti-c-Myc magnetic beads, which bind the SynNotch receptor, allowing activation of the receptor without being co-cultured with target cells carrying the cognate antigen.

### Example 9

Anti-CD19 SynNotch-STING^{V155M} U-pDCs, or U-pDCs not expressing the construct (mock), were primed (IFN) or not primed (IL-3), and then activated by exposure to target cells expressing CD19, with 2'3'-cGAMP or anti-c-Myc magnetic beads. The data in Figure11 show SynNotch STING V155M pDCs induce a broad cytokine response in response to activation by target cells expressing cognate antigen, by cGAMP or by c-Myc. The cytokines induced include type I and III IFNs, as well as various chemokines, including MCP-2, CXCL10 (IP-10), MCP-1, MIP-1a, and MIP-1b (Figure 11a-b).

The broad immune response of SynNotch-STING^{V155M} U-pDCs activated by cGAMP or c-Myc is also reflected on the RNA level, as measured by RNA-seq (Figure 12a-e). This is reflected in the induction of multiple interferon-induced genes (ISGs), and different IFN subtypes, including IFIT1, IFIT2, MX2, CXCL11, CXCL10, IFNL1, IFNL2, IFNL3, IFNB1, IFNA1, IFNA8, and IFNA10 (Figure 12d). Overall, the resulting response of activation of the SynNotch STING-gain-of-function correlates with a potent induction of an immune response, which is linked with anti-viral defences, type I IFN signalling, induction of cytokine-mediated signalling pathways, and activation of various immune cells, including NK cells and B cells (Figure 12e).

### Example 10

The response of SynNotch-STING^{V155M} U-pDCs is dependent on recognizing cognate antigen. Secretion of IFNa was induced in anti-CD19 SynNotch STING^{V155M} pDCs upon exposure to CD19-positive target cells (Raji cells). CD19-knock-out Raji cells did not induce a response, suggesting that no ligand-independent activation occurred (Figure 13)

### Example 11

Activated SynNotch-STING^{V155M} U-pDCs produce factors that promote the activity of NK cells (Figure 14a-f). Conditioned medium from activated SynNotch STING gain of function pDCs induces activation markers on NK cells, including CD69 and CD253 (TRAIL) (Figure 14b-e). The capacity of NK cells to lyse the cancer cell lines K562 and REH is increased (Figure 14f-g) following priming with conditioned medium from SynNotch pDCs . The killing function of the NK cells can also be recapitulated with conditioned medium from cGAMP stimulated Mock U-pDCs, in addition to SynNotch pDCs (Figure 14f-g).

SynNotch-STING^{v155M} U-pDCs also cooperate with NK cells in killing cancer cells in a triple culture system, but also show killing capacity on their own (Figure 15). In a triple culture system comprising target cells, NK cells and pDCs, killing is highly induced for the co-culture of NK cells with the SynNotch U-pDCs, with up to 60% killing achieved after 48-hours (Figure 15b-c). This effect can also be replicated for Mock U-pDCs when stimulated with cGAMP (Figure 15b-c). Of note, the pDCs do not seem to be killed by the NK cells during the co-culture (Figure 15d). Importantly, co-culture of SynNotch U-pDCs alone with target cells (without the presence of NK cells) leads to potent killing of the target cells with up to 40% killing of the target cells during 48-hours of culture, whereas less than 10% of target cells are killed with NK cells alone. This indicates the SynNotch-STING^{V155M} U-pDCs are more potent at killing the target cells than NK cells (Figure 15e-f). Conditioned medium from TAA-activated SynNotch-STING^{V155M} U-pDCs do not lead to target cell death, indicating the killing is mediated by cell-to-cell contact (Figure 15g).

### Example 12

The killing function of the SynNotch-STING^{V155M} U-pDCs is rapid with killing already being observed after 1-day of culture, but also increases during 6-days of co-culture with the target cells REH and NALM6, with accumulated killing reaching up to 60% (Figure 16a-b).

The induction of the STING-gain-of-function variant occurs rapidly with mRNA expression topping during 6-hours after activation (Figure 17a-c). Accordingly, type I IFNs (IFNa2 and IFNb), and various ISGs (IFIT1 and CXCL10) are rapidly induced during the first 6-hours of stimulation (Figure 17b). Similarly, type I IFN is rapidly secreted from SynNotch activated pDCs, topping after 24-hours post stimulation (Figure 17c). The response seems to be rapidly turned off as the activation agent (anti-cMyc magnetic beads) is taken up by the cells, or removed from the culture using magnetic depletion (Figure 17b and c).

### Example 13

NXG mice (Janvier) were injected subcutaneously (s.c.) with 5e6 katushka-positive NALM6 cells. When mice had developed a mean tumor size of 120 mm³ ± 50 mm³, 1e7 Mock or SynNotch-STING^{v155M} U-pDCs were injected intravenously (i.v.) by the tail vein or intratumorally (i.t.) (Figure 18a). 48-hours after i.v. or i.t. administration of Mock or SynNotch-STING^{v155M} U-pDCs, the SynNotch pDCs homed to the tumor (Figure 18c) and induce rapid regression of the tumor following 48-hours after injection (Figure 18e).

### Example 14

A next-generation Synthetic Intramembrane Proteolysis Receptor (SNIPR) expressing the STING gain-of-function component can be expressed and is functional in pDCs (Figure 19a-d). The SNIPR system seems to be more efficient versus the conventional SynNotch, both when it comes to expression levels in cells (Figure 19b), response to target cells (Figure 19c), and killing capacity (Figure 19d).

## Claims

1. An engineered immune cell that expresses on its surface a synthetic receptor comprising an extracellular antigen recognition domain that recognises an antigen on a target cell, a transmembrane domain, and an intracellular signalling domain that activates expression of a response element in the cell when the extracellular antigen recognition domain binds the target cell, wherein the cell comprises a heterologous nucleic acid comprising the response element, wherein the response element encodes a stimulator of interferon genes (STING) protein, and
wherein the intracellular signalling domain is a transcription factor comprising a DNA binding domain and a transcriptional activation domain, and wherein the response element contains the cognate recognition element for the DNA binding domain, and
wherein the synthetic receptor is:
(a) a synthetic intramembrane proteolysis receptor (SNIPR), such as wherein the synthetic receptor is a synthetic RIP family receptor, optionally wherein the receptor is:
(i) synthetic Notch receptor;
(ii) a synthetic Robo receptor; or
(iii) a synthetic RoboNotch receptor;
(b) a Tango receptor; or
(c) a modular extracellular signalling architecture (MESA) system.

2. The cell of claim 1, wherein the cell is:
(a) a lymphoid cell, such as wherein the cell is a natural killer cell, a T cell, a B cell or a plasmacytoid dendritic cell (pDC); or
(b) a myeloid cell, such as wherein the cell is a macrophage.

3. The cell of claim 1 or claim 2, wherein:
(a) the DNA binding domain is selected from the group consisting of Gal4, Pax6, zinc fingers (ZFs), synthetic zinc fingers (synZFs), transcription activator like effectors (TALEs), TetR, HNF1 alpha, and vHNF1 beta; and/or
(b) the transcription activation domain is selected from the group consisting of VP64, VP16, WWTR1, CREB3, NF-κB, p65, Rta, HSF1, and RelA; and/or
(c) the intracellular signalling domain is selected from the group consisting of Gal4-VP64, Gal4-VP16, TetR-VP64, LacI-VP64, HNF1-WWTR1, HNF1-CREB3, Pax6-p65, ZF-p65, synZF-p65, and HNF1-p65.

4. The cell of any one of the preceding claims, wherein the extracellular antigen recognition domain is an antibody-derived domain, such as wherein the extracellular antigen recognition domain is an scFv domain.

5. The cell of any one of the preceding claims, wherein the STING protein comprises:
(a) a gain-of-function mutation that causes it to be constitutively active in the absence of 2'3'-cGAMP; and/or
(b) a mutation, preferably a substitution, at one or more residues corresponding to R71, S102, V147, N154, V155, G166, C206, G207, G230, H232, R238, F279, R281, R284, R293, or Q315 of SEQ ID NO:1.

6. The cell of any one of the preceding claims, wherein the cell comprises a heterologous nucleic acid encoding the synthetic receptor, optionally wherein the cell comprises a single heterologous nucleic acid encoding the synthetic receptor and the response element.

7. The cell of any of claims 2-6, wherein the pDC has undergone a step of priming the pDC, optionally wherein the step of priming the pDC comprises incubating the cells with type I IFN and/or type II IFN, such as wherein the pDC is incubated with type I IFN and/or type II IFN for 24 hours.

8. The cell of any one of the preceding claims, wherein the STING protein activates an immune response, such as wherein:
(a) the immune response comprises secretion of cytokines, optionally wherein the cytokines modulate the environment of the diseased tissue, or wherein the immune response comprises recruitment or activation of immune cells, such as wherein the cytokines are pro-inflammatory cytokines, optionally wherein the cytokines comprise CXCL10, MCP-1, MCP-2, MIP-1a and/or MIP-1b; and/or
(b) the immune response comprises production of type I IFN.

9. The cell of any one of the preceding claims, for use in a method of treating a disease in a subject, wherein the disease is:
(a) a cancer;
(b) an autoimmune disease;
(c) an inflammatory disease; or
(d) an infectious disease.

10. The cell for use according to claim 9a, wherein:
(a) the cancer is a solid cancer, such as a cancer selected from the group consisting of: bone cancer, breast cancer, pancreatic cancer, skin cancer, cancer of the head or neck, cutaneous or intraocular malignant melanoma, uterine cancer, ovarian cancer, prostate cancer, rectal cancer, cancer of the anal region, colon cancer, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, pediatric tumors, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, glioblastoma, pituitary adenoma, Kaposi's sarcoma, epidermoid cancer and squamous cell cancer; or
(b) the cancer is selected from the group consisting of acute lymphoblastic leukemia (ALL) (including non T cell ALL), acute myeloid leukemia, B cell prolymphocytic leukemia, B-cell acute lymphoid leukemia ("BALL"), blastic plasmacytoid dendritic cell neoplasm, Burkitt s lymphoma, chronic lymphocytic leukemia (CLL), chronic myelogenous leukemia (CML), chronic myeloid leukemia, chronic or acute leukemia, diffuse large B cell lymphoma (DLBCL), follicular lymphoma (FL), hairy cell leukemia, Hodgkin's Disease, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome, non-Hodgkin's lymphoma (NHL), plasma cell proliferative disorder (including asymptomatic myeloma (smoldering multiple myeloma or indolent myeloma), plasmablastic lymphoma, plasmacytoid dendritic cell neoplasm, plasmacytomas (including plasma cell dyscrasia; solitary myeloma; solitary plasmacytoma; extramedullary plasmacytoma; and multiple plasmacytoma), POEMS syndrome (also known as Crow-Fukase syndrome; Takatsuki disease; and PEP syndrome), primary mediastinal large B cell lymphoma (PMBC), small cell- or a large cell-follicular lymphoma, splenic marginal zone lymphoma (SMZL), systemic amyloid light chain amyloidosis, T-cell acute lymphoid leukemia ("TALL"), T-cell lymphoma, transformed follicular lymphoma, and Waldenstrom macroglobulinemia.

11. The cell, or cell for use, according to any preceding claim, wherein the antigen is a tumour-associated antigen, such as wherein the antigen is selected from:
(a) the group consisting of CD19, CD20, CD22, CD30, CD33, CD38, CD123, CD138, CS-1, B-cell maturation antigen (BCMA), MAGEA3, MAGEA3/A6, KRAS, CLL1, MUC-1, HER2, EpCam, GD2, GPA7, PSCA, EGFR, EGFRvIII, ROR1, mesothelin, CD33/IL3Ra, c-Met, CD37, PSMA, Glycolipid F77, GD-2, gp100, NY-ESO-1 TCR, FRalpha, CD24, CD44, CD133, CD166, CA-125, HE4, Oval, estrogen receptor, progesterone receptor, uPA, PAI-1, MICA, MICB, ULBP1, ULBP2, ULBP3, ULBP4, ULBP5 or ULBP6, or a combination thereof; or
(b) the group consisting of 5T4, alphafetoprotein (AFP), B7-1 (CD80), B7-2 (CD86), BCMA, B-human chorionic gonadotropin, CA-125, carcinoembryonic antigen (CEA), carcinoembryonic antigen (CEA), CD123, CD133, CD138, CD19, CD20, CD22, CD23, CD24, CD25, CD30, CD33, CD34, CD4, CD40, CD44, CD56, CD70, CD8, CLL-l, c-Met, CMV-specific antigen, CS-l, CSPG4, CTLA-4, DLL3, disialoganglioside GD2, ductal -epithelial mucine, EBV-specific antigen, EGFR variant III (EGFRvIII), ELF2M, endoglin, ephrin B2, epidermal growth factor receptor (EGFR), epithelial cell adhesion molecule (EpCAM), epithelial tumour antigen, ErbB2 (HER2/neu), fibroblast associated protein (fap), FLT3, folate binding protein, GD2, GD3, glioma-associated antigen, glycosphingolipids, gp36, HBV- specific antigen, HCV-specific antigen, HER1, HER2, HER2-HER3 in combination, HERV-K, high molecular weight-melanoma associated antigen (HMW-MAA), HIV-l envelope glycoprotein gp4l, HPV-specific antigen, human telomerase reverse transcriptase, IGFI receptor, IGF-II, IL-11Ralpha, IL-13R-a2, Influenza Virus-specific antigen, CD38, insulin growth factor (IGF1)-l, intestinal carboxyl esterase, kappa chain, LAGA-la, lambda chain, Lassa Virus-specific antigen, lectin-reactive AFP, lineage-specific or tissue specific antigen such as CD3, MAGE, MAGE-A1, major histocompatibility complex (MHC) molecule, major histocompatibility complex (MHC) molecule presenting a tumour-specific peptide epitope, M-CSF, melanoma-associated antigen, mesothelin, MN-CA IX, MUC-l, mut hsp70-2, mutated p53, mutated p53, mutated ras, neutrophil elastase, NKG2D, Nkp30, NY-ESO-1, p53, PAP, prostase, prostate specific antigen (PSA), prostate-carcinoma tumour antigen- 1 (PCTA-l), prostatespecific antigen protein, STEAP1, STEAP2, PSMA, RAGE-l, ROR1, RU1, RU2 (AS), surface adhesion molecule, survivin, telomerase, TAG-72, the extra domain A (EDA) and extra domain B (EDB) of fibronectin and the A1 domain of tenascin-C (TnC Al), thyroglobulin, tumour stromal antigens, vascular endothelial growth factor receptor-2 (VEGFR2), virus-specific surface antigen such as an HIV-specific antigen, or a combination thereof.

12. The cell for use according to claim 9b, wherein the autoimmune disease is selected from the group consisting of type 1 diabetes, thyroid autoimmune disease, Addison's adrenal insufficiency, oophoritis, orchitis, lymphocytic hypophysitis, autoimmune hypoparathyroidism, autoimmune hypoparathyroidism, Goodpasture's disease, autoimmune myocarditis, membranous nephropathy, autoimmune hepatitis, ulcerative colitis, Crohn's disease, multiple sclerosis, myasthenia gravis, neuromyelitis optica, encephalitis and Sjögren's syndrome.

13. The cell for use according to claim 9c, wherein the inflammatory disease is selected from the group consisting of cystic fibrosis, chronic inflammatory intestinal diseases like, for example, ulcerative colitis or Crohn's disease, vasculitis, in particular Kawasaki disease, chronic bronchitis, inflammatory arthritis diseases like, for example, psoriatic arthritis, osteoarthritis, rheumatoid arthritis, and systemic onset juvenile rheumatoid arthritis (SOJRA, Still's disease), graft-versus-host disease, asthma, psoriasis, systemic lupus erythematosus, obesity and inflammatory vascular disease and allograft rejection.

14. The cell of any of claims 1-8 or the cell for use according to any of claims 9b, 9c, 12 or 13, wherein the antigen is expressed on the surface of immune cells that cause autoimmune or inflammatory disease.

15. The cell or cell for use according to claim 9b or claim 9c, wherein the disease is transplant rejection or graft-versus-host disease (GVHD).

16. The cell for use according to claim 9d, wherein the infectious disease is a chronic viral or fungal infection, such as infection of Influenza, Yellow Fever virus, West Nile virus, Hantavirus, Ebola virus, Rotavirus, Norovirus, Rabies virus, Tick-borne encephalitis virus (TBEV), rhinoviruses, Coronavirus, RSV, Measles, Parainfluenza, Zikavirus, Dengue virus, HIV, HBV, HCV, human cytomegalovirus (CMV), Epstein-Barr virus (EBV), *Aspergillus spp.,* such as *Aspergillus fumigatus, Candida spp.,* such as *C*. *albicans, Mucorales, Cryptococcus spp.,* such as *Cryptococcus neoformans* or *Cryptococcus gattii,* or *Pneumocystis jirovecii.*

17. The cell of any of claims 1-8 or the cell for use according to claim 9d or claim 16, wherein the antigen is a pathogen antigen, such as wherein the antigen is selected from the group consisting of Yellow Fever virus NS 1 protein, West Nile virus NS 1 protein, Hantavirus N protein, Ebola virus N protein, Rotavirus VP6, Norovirus VP1, rabies virus N protein, TBEV envelope glycoprotein, rhinovirus VP proteins, *Influenza* hemagglutinin antigens, *Influenza* neuraminidase antigens, coronavirus spike protein, RSV F protein, MeV N protein, *Parainfluenza* hemagglutinin antigens, *Parainfluenza* neuraminidase antigens, ZIKV E, NS1, NS3, NS4B, and NS5 proteins, Dengue virus C protein, M protein, E protein, NS1 protein, gp120, gp41, Env, HBV surface antigen, HBV surface proteins S and L, HCV E2 glycoprotein, CMV glycoprotein B, fungal beta glucan.

18. The cell, or cell for use, according to any one of the preceding claims, wherein the cell has been extracted from blood or bone marrow, or wherein the cell is a pDC and the cell has been differentiated *in vitro* from HSPCs, optionally wherein the HSPCs have been obtained from blood or bone marrow, or wherein the HSPCs have been differentiated *in vitro* from induced pluripotent stem cells (iPSCs) or embryonic stem cells (ESCs).

19. A pharmaceutical composition comprising the engineered immune cell of any preceding claim and a pharmaceutically acceptable carrier.

20. A method of producing the cell or cell or for use according to any one of claims 2a or 3-19, comprising the steps of:
(a)
(i) transfecting or transducing HSPCs with one or more vectors encoding the synthetic receptor and the response element; and
(ii) differentiating the HSPCs into pDCs; or
(b)
(i) differentiating HSPCs into pDCs; and
(ii) transfecting or transducing the pDCs with one or more vectors encoding the synthetic receptor and the response element;
optionally wherein:
(A) the vector is a viral vector, optionally wherein the vector is a lentiviral vector or an adenoviral or adeno-associated viral vector; or
(B) wherein the vector is an mRNA, optionally encapsulated in a lipid nanoparticle.

21. The method of claim 20, wherein:
(a) the step of differentiating the HSPCs comprises incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs; and/or
(b) the method further comprises a step of purifying the pDCs; and/or
(c) the method further comprises a step of formulating the pDCs with a pharmaceutically acceptable excipient.

## Patentansprüche

1. Genetisch veränderte Immunzelle, die an ihrer Oberfläche einen synthetischen Rezeptor exprimiert, der Folgendes umfasst: eine extrazelluläre Antigenerkennungsdomäne, die ein Antigen auf einer Zielzelle erkennt, eine Transmembrandomäne und eine intrazelluläre Signaldomäne, die die Expression eines Response-Elements in der Zelle aktiviert, wenn die extrazelluläre Antigenerkennungsdomäne an die Zielzelle bindet, wobei die Zelle eine heterologe Nukleinsäure umfasst, die das Response-Element enthält, wobei das Response-Element für ein Stimulator-von-Interferon-Genen- (STING) Protein codiert, und
wobei die intrazelluläre Signaldomäne ein Transkriptionsfaktor ist, der eine DNA-Bindungsdomäne und eine Transkriptionsaktivierungsdomäne umfasst, und wobei das Response-Element das kognitive Erkennungselement für die DNA-Bindungsdomäne enthält, und
wobei der synthetische Rezeptor Folgendes ist:
(a) ein synthetischer intramembranärer Proteolyse-Rezeptor (SNIPR), wobei der synthetische Rezeptor beispielsweise ein synthetischer Rezeptor der RIP-Familie ist, optional wobei der Rezeptor Folgendes ist:
(i) ein synthetischer Notch-Rezeptor;
(ii) ein synthetischer Robo-Rezeptor; oder
(iii) ein synthetischer RoboNotch-Rezeptor;
(b) ein Tango-Rezeptor; oder
(c) ein modulares extrazelluläres Signalisierungs-Architektur- (MESA) System.

2. Zelle nach Anspruch 1, wobei die Zelle Folgendes ist:
(a) eine lymphoide Zelle ist, wobei die Zelle beispielsweise eine natürliche Killerzelle, eine T-Zelle, eine B-Zelle oder eine plasmazytoide dendritische Zelle (pDC) ist; oder
(b) eine myeloide Zelle ist, wobei die Zelle ein Makrophag ist.

3. Zelle nach Anspruch 1 oder Anspruch 2, wobei:
(a) die DNA-Bindungsdomäne ausgewählt ist aus der Gruppe bestehend aus Gal4, Pax6, Zinkfingern (ZFs), synthetischen Zinkfingern (synZFs), Transkriptionsaktivatorähnlichen Effektoren (TALEs), TetR, HNF1-alpha und vHNF1-beta; und/oder
(b) die Transkriptionsaktivierungsdomäne ausgewählt ist aus der Gruppe bestehend aus VP64, VP16, WWTR1, CREB3, NF-κB, p65, Rta, HSF1 und RelA; und/oder
(c) die intrazelluläre Signaldomäne ausgewählt ist aus der Gruppe bestehend aus Gal4-VP64, Gal4-VP16, TetR-VP64, LacI-VP64, HNF1-WWTR1, HNF1-CREB3, Pax6-p65, ZF-p65, synZF-p65 und HNF1-p65.

4. Zelle nach einem der vorhergehenden Ansprüche, wobei die extrazelluläre Antigenerkennungsdomäne eine von einem Antikörper abgeleitete Domäne ist, wobei die extrazelluläre Antigenerkennungsdomäne beispielsweise eine scFv-Domäne ist.

5. Zelle nach einem der vorhergehenden Ansprüche, wobei das STING-Protein umfasst:
(a) eine Funktionsgewinnmutation, die bewirkt, dass es in Abwesenheit von 2'3'-cGAMP konstitutiv aktiv ist; und/oder
(b) eine Mutation, vorzugsweise eine Substitution, an einem oder mehreren Aminosäureresten, die R71, S102, V147, N154, V155, G166, C206, G207, G230, H232, R238, F279, R281, R284, R293 oder Q315 von SEQ ID NO:1 entsprechen.

6. Zelle nach einem der vorhergehenden Ansprüche, wobei die Zelle eine heterologe Nukleinsäure umfasst, die den synthetischen Rezeptor codiert, optional wobei die Zelle eine einzige heterologe Nukleinsäure umfasst, die den synthetischen Rezeptor und das Response-Element codiert.

7. Zelle nach einem der Ansprüche 2 bis 6, wobei die pDC einen Schritt der Priming-Behandlung durchlaufen hat, optional wobei der Schritt der Priming-Behandlung der pDC das Inkubieren der Zellen mit Typ-I-IFN und/oder Typ-II-IFN umfasst, wobei die pDC beispielsweise 24 Stunden lang mit Typ-I-IFN und/oder Typ-II-IFN inkubiert wird.

8. Zelle nach einem der vorhergehenden Ansprüche, wobei das STING-Protein eine Immunantwort aktiviert, wobei:
(a) die Immunantwort die Sekretion von Zytokinen umfasst, wobei die Zytokine optional das Milieu des erkrankten Gewebes modulieren, oder wobei die Immunantwort die Rekrutierung oder Aktivierung von Immunzellen umfasst, wobei die Zytokine beispielsweise proinflammatorische Zytokine sind, wobei die Zytokine optional CXCL10, MCP-1, MCP-2, MIP-1a und/oder MIP-1b umfassen; und/oder
(b) die Immunantwort die Produktion von Typ-I-IFN umfasst.

9. Zelle nach einem der vorhergehenden Ansprüche zur Verwendung in einem Verfahren zur Behandlung einer Erkrankung bei einem Patienten, wobei die Erkrankung Folgendes ist:
(a) eine Krebserkrankung;
(b) eine Autoimmunerkrankung;
(c) eine entzündliche Erkrankung; oder
(d) eine Infektionskrankheit ist.

10. Zelle zur Verwendung nach Anspruch 9a, wobei:
(a) der Krebs ein solider Krebs ist, wie beispielsweise ein Krebs, ausgewählt aus der Gruppe bestehend aus: Knochenkrebs, Brustkrebs, Bauchspeicheldrüsenkrebs, Hautkrebs, Kopf- oder Halskrebs, kutanem oder intraokularem malignem Melanom, Gebärmutterkrebs, Eierstockkrebs, Prostatakrebs, Rektumkrebs, Krebs im Analbereich, Dickdarmkrebs, Magenkrebs, Hodenkrebs, Gebärmutterkrebs, Eileiterkarzinom, Endometriumkarzinom, Gebärmutterhalskarzinom, Vaginalkarzinom, Vulvakarzinom, Speiseröhrenkrebs, Dünndarmkrebs, Krebs des endokrinen Systems, Schilddrüsenkrebs, Nebenschilddrüsenkrebs, Nebennierenkrebs, Weichteilsarkom, Harnröhrenkrebs, Peniskrebs, pädiatrische Tumoren, Blasenkrebs, Nieren- oder Harnleiterkrebs, Nierenbeckenkarzinom, Neoplasma des Zentralnervensystems (ZNS), primärem ZNS-Lymphom, Tumorangiogenese, Tumor der Wirbelsäule, Hirnstammgliom, Glioblastom, Hypophysenadenom, Kaposi-Sarkom, epidermoidem Karzinom und Plattenepithelkarzinom; oder
(b) der Krebs aus der Gruppe stammt bestehend aus: akuter lymphoblastischer Leukämie (ALL) (die Nicht-T-Zell-ALL enthält), akuter myeloischer Leukämie, B-Zell-Prolymphozyten-Leukämie, akuter lymphoider B-Zell-Leukämie ("BALL"), plastischem plasmazytoidem dendritischen Zellneoplasma, Burkitt-Lymphom, chronischer lymphatischer Leukämie (CLL), chronischer myeloischer Leukämie (CML), chronischer myeloischer Leukämie, chronischer oder akuter Leukämie, diffusem großzelligem B-Zell-Lymphom (DLBCL), follikulärem Lymphom (FL), Haarzell-Leukämie, Morbus Hodgkin, malignen lymphoproliferativen Erkrankungen, MALT-Lymphom, Mantelzell-Lymphom, Marginalzonen-Lymphom, monoklonaler Gammopathie unbestimmter Signifikanz (MGUS), multiplem Myelom, Myelodysplasie und myelodysplastischem Syndrom, Non-Hodgkin-Lymphom (NHL), plasmazellulärer Proliferationsstörung (einschließlich asymptomatisches Myelom (schwelendes multiples Myelom oder indolentes Myelom), plasmablastischem Lymphom, plasmazytoidem dendritischem Zellneoplasma, Plasmazytomen (die Plasmazelldyskrasie enthalten; solitäres Myelom; solitäres Plasmazytom; extramedulläres Plasmazytom; und multiples Plasmazytom), POEMS-Syndrom (auch bekannt als Crow-Fukase-Syndrom; Takatsuki-Krankheit; und PEP-Syndrom), primärem mediastinalem großzelligem B-Zell-Lymphom (Primary Mediastinal large B Cell lymphoma, PMBC), kleinzelligem oder großzelligem follikulärem Lymphom, Milz-Randzonen-Lymphom (SMZL), systemischer Amyloidose der leichten Ketten, akuter lymphatischer T-Zell-Leukämie ('TALL"), T-Zell-Lymphom, transformiertem follikulärem Lymphom und Waldenström-Makroglobulinämie.

11. Zelle oder Zelle zur Verwendung nach einem der vorhergehenden Ansprüche, wobei das Antigen ein tumorassoziiertes Antigen ist, wobei das Antigen beispielsweise ausgewählt ist aus:
(a) der Gruppe bestehend aus CD19, CD20, CD22, CD30, CD33, CD38, CD123, CD138, CS-1, B-Zell-Reifungsantigen (BCMA), MAGEA3, MAGEA3/A6, KRAS, CLL1, MUC-1, HER2, EpCam, GD2, GPA7, PSCA, EGFR, EGFRvIII, ROR1, Mesothelin, CD33/IL3Ra, c-Met, CD37, PSMA, Glykolipid F77, GD-2, gp100, NY-ESO-1-TCR, FRalpha, CD24, CD44, CD133, CD166, CA-125, HE4, Oval, Östrogenrezeptor, Progesteronrezeptor, uPA, PAI-1, MICA, MICB, ULBP1, ULBP2, ULBP3, ULBP4, ULBP5 oder ULBP6 oder eine Kombination davon; oder
(b) der Gruppe bestehend aus 5T4, Alpha-Fetoprotein (AFP), B7-1 (CD80), B7-2 (CD86), BCMA, humanem B-Choriongonadotropin, CA-125, karzinoembryonalem Antigen (CEA), karzinoembryonalem Antigen (CEA), CD123, CD133, CD138, CD19, CD20, CD22, CD23, CD24, CD25, CD30, CD33, CD34, CD4, CD40, CD44, CD56, CD70, CD8, CLL-1, c-Met, CMV-spezifischem Antigen, CS-1, CSPG4, CTLA-4, DLL3, Disialogangliosid GD2, duktalem Epithel-Muzin, EBV-spezifischem Antigen, EGFR-Variante III (EGFRvIII), ELF2M, Endoglin, Ephrin B2, epidermalem Wachstumsfaktorrezeptor (EGFR), Epithelzell-Adhäsionsmolekül (EpCAM), epithelialem Tumorantigen, ErbB2 (HER2/neu), fibroblastenassoziiertem Protein (fap), FLT3, Folsäure-bindendem Protein, GD2, GD3, Gliom-assoziiertem Antigen, Glykosphingolipiden, gp36, HBV-spezifischem Antigen, HCVspezifischem Antigen, HER1, HER2, HER2-HER3 in Kombination, HERV-K, hochmolekularem Melanom-assoziiertem Antigen (HMW-MAA), HIV-1-Hüllglykoprotein gp41, HPV-spezifischem Antigen, humanem Telomerase-Reverse-Transkriptase, IGF-I-Rezeptor, IGF-II, IL-11R-alpha, IL-13R-a2, Influenza-Virus-spezifischem Antigen, CD38, Insulin-Wachstumsfaktor (IGFI)-1, intestinale Carboxylesterase, Kappa-Kette, LAGA-1a, Lambda-Kette, Lassa-Virus-spezifischem Antigen, Lektin-reaktivem AFP, linien- oder gewebespezifischem Antigen wie CD3, MAGE, MAGE-A1, Molekül des Haupthistokompatibilitätskomplexes (MHC), Molekül des Haupthistokompatibilitätskomplexes (MHC), das ein tumorspezifisches Peptid-Epitop präsentiert, M-CSF, Melanom-assoziiertem Antigen, Mesothelin, MN-CA IX, MUC-1, mutiertem hsp70-2, mutiertem p53, mutiertem p53, mutiertem ras, Neutrophilen-Elastase, NKG2D, Nkp30, NY-ESO-1, p53, PAP, Prostase, prostataspezifischem Antigen (PSA), Prostatakarzinom-Tumorantigen-1 (PCTA-1), prostataspezifischem Antigenprotein, STEAP1, STEAP2, PSMA, RAGE-1, ROR1, RU1, RU2 (AS), Oberflächenadhäsionsmolekül, Survivin, Telomerase, TAG-72, der Extra-Domäne A (EDA) und der Extra-Domäne B (EDB) von Fibronektin sowie der A1-Domäne von Tenascin-C (TnC A1), Thyroglobulin, Tumorstroma-Antigenen, vaskulärem endothelialem Wachstumsfaktor-Rezeptor-2 (VEGFR2), virusspezifischem Oberflächenantigen, wie beispielsweise einem HIV-spezifischen Antigen, oder einer Kombination davon.

12. Zelle zur Verwendung nach Anspruch 9b, wobei die Autoimmunerkrankung ausgewählt ist aus der Gruppe bestehend aus Typ-I-Diabetes, Schilddrüsen-Autoimmunerkrankung, Addison-Syndrom, Oophoritis, Orchitis, lymphozytärer Hypophysitis, autoimmunem Hypoparathyreoidismus, autoimmunem Hypoparathyreoidismus, Goodpasture-Syndrom, autoimmuner Myokarditis, membranöser Nephropathie, autoimmuner Hepatitis, Colitis ulcerosa, Morbus Crohn, Multipler Sklerose, Myasthenia gravis, Neuromyelitis optica, Enzephalitis und Sjögren-Syndrom.

13. Zelle zur Verwendung nach Anspruch 9c, wobei die entzündliche Erkrankung ausgewählt ist aus der Gruppe bestehend aus Mukoviszidose, chronisch-entzündlichen Darmerkrankungen wie beispielsweise Colitis ulcerosa oder Morbus Crohn, Vaskulitis, insbesondere dem Kawasaki-Syndrom, chronischer Bronchitis, entzündlichen Arthritis-Erkrankungen wie beispielsweise Psoriasis-Arthritis, Osteoarthritis, rheumatoider Arthritis und systemisch auftretender juveniler rheumatoider Arthritis (SOJRA, Morbus Still), Transplantat-gegen-Wirt-Reaktion, Asthma, Psoriasis, systemischem Lupus erythematodes, Adipositas sowie entzündlichen Gefäßerkrankungen und Allotransplantatabstoßung.

14. Zelle nach einem der Ansprüche 1-8 oder Zelle zur Verwendung nach Anspruch 9b, 9c, 12 oder 13, wobei das Antigen auf der Oberfläche von Immunzellen exprimiert wird, die Autoimmun- oder Entzündungskrankheiten verursachen.

15. Zelle oder Zelle zur Verwendung nach Anspruch 9b oder Anspruch 9c, wobei die Erkrankung eine Transplantatabstoßung oder eine Transplantat-gegen-Wirt-Reaktion (Graft-Versus-Host Disease, GVHD) ist.

16. Zelle zur Verwendung nach Anspruch 9d, wobei die Infektionskrankheit eine chronische Virus- oder Pilzinfektion ist, wie beispielsweise eine Infektion mit Influenza, Gelbfiebervirus, West-Nil-Virus, Hantavirus, Ebola-Virus, Rotavirus, Norovirus, Tollwutvirus, Zeckenenzephalitis-Virus (TBEV), Rhinoviren, Coronaviren, RSV, Masern, Parainfluenza, Zika-Virus, Dengue-Virus, HIV, HBV, HCV, humaner Cytomegalievirus (CMV), Epstein-Barr-Virus (EBV), Aspergillus spp., wie beispielsweise Aspergillus fumigatus, Candida spp., wie beispielsweise C. albicans, Mucorales, Cryptococcus spp., wie beispielsweise Cryptococcus neoformans oder Cryptococcus gattii, oder Pneumocystis jirovecii.

17. Zelle nach einem der Ansprüche 1-8 oder Zelle zur Verwendung nach Anspruch 9d oder Anspruch 16, wobei das Antigen ein Pathogenantigen ist, wobei das Antigen ausgewählt ist aus der Gruppe bestehend aus Gelbfiebervirus-NS1-Protein, West-Nil-Virus-NS1-Protein, Hantavirus-N-Protein, Ebola-Virus-N-Protein, Rotavirus-VP6, Norovirus-VPl, N-Protein des Tollwutvirus, TBEV-Hüllglykoprotein, VP-Proteinen des Rhinovirus, Hämagglutinin-Antigenen des Influenzavirus, Neuraminidase-Antigenen des Influenzavirus, Spike-Protein des Coronavirus, F-Protein des RSV, N-Protein des MeV, Hämagglutinin-Antigenen des Parainfluenzavirus, Neuraminidase-Antigenen des Parainfluenzavirus, ZIK V E-, NS1-, NS3-, NS4B- und NS5-Proteine, Dengue-Virus-C-Protein, M-Protein, E-Protein, NS1-Protein, gp120, gp41, Env, HBV-Oberflächenantigen, HBV-Oberflächenproteinen S und L, HCV-E2-Glykoprotein, CMV-Glykoprotein B, pilzliches Beta-Glucan.

18. Zelle oder Zelle zur Verwendung nach einem der vorhergehenden Ansprüche, wobei die Zelle aus Blut oder Knochenmark gewonnen wurde, oder wobei die Zelle eine pDC ist und die Zelle *in vitro* aus HSPCs differenziert wurde, optional wobei die HSPCs aus Blut oder Knochenmark gewonnen wurden, oder wobei die HSPCs *in vitro* aus induzierten pluripotenten Stammzellen (iPSCs) oder embryonalen Stammzellen (ESCs) differenziert wurden.

19. Pharmazeutische Zusammensetzung, die die gentechnisch veränderte Immunzelle nach einem der vorhergehenden Ansprüche und einen pharmazeutisch annehmbaren Träger umfasst.

20. Verfahren zur Herstellung der Zelle oder zur Verwendung nach Anspruch 2a oder 3-19, das folgende Schritte umfasst:
(a)
(i) Transfizieren oder Umwandeln von HSPCs mit einem oder mehreren Vektoren, die für den synthetischen Rezeptor und das Response-Element codieren; und
(ii) Differenzieren der HSPCs zu pDCs; oder
(b)
(i) Differenzieren von HSPCs zu pDCs; und
(ii) Transfizieren oder Umwandeln der pDCs mit einem oder mehreren Vektoren, die für den synthetischen Rezeptor und das Response-Element codieren;
optional wobei:
(A) der Vektor ein viraler Vektor ist, wobei der Vektor optional ein lentiviraler Vektor oder ein adenoviraler oder adeno-assoziierter viraler Vektor ist; oder
(B) der Vektor eine mRNA ist, die optional in ein Lipidnanopartikel eingekapselt ist.

21. Verfahren nach Anspruch 20, wobei:
(a) der Schritt der Differenzierung der HSPCs das Inkubieren der HSPCs in einem oder mehreren Medien umfasst, wobei diese Medien typischerweise ein oder mehrere Zytokine, Wachstumsfaktoren, Interferone (IFNs) und/oder Arylhydrocarbon-Rezeptor-(AHR) Antagonisten (wie beispielsweise Stemregenin-1) umfassen können, wodurch die HSPCs zu Vorläufer-pDCs und zu pDCs differenziert werden; und/oder
(b) das Verfahren ferner einen Schritt der Reinigung der pDCs umfasst; und/oder
(c) das Verfahren ferner einen Schritt der Formulierung der pDCs mit einem pharmazeutisch annehmbaren Hilfsstoff umfasst.

## Revendications

1. Cellule immunitaire modifiée qui exprime à sa surface un récepteur synthétique comprenant un domaine de reconnaissance d'antigène extracellulaire qui reconnaît un antigène sur une cellule cible, un domaine transmembranaire et un domaine de signalisation intracellulaire qui active l'expression d'un élément de réponse dans la cellule lorsque le domaine de reconnaissance d'antigène extracellulaire se lie à la cellule cible, ladite cellule comprenant un acide nucléique hétérologue comprenant l'élément de réponse, ledit élément de réponse codant pour une protéine STING (stimulateur des gènes de l'interféron), et
le domaine de signalisation intracellulaire étant un facteur de transcription comprenant un domaine de liaison à l'ADN et un domaine d'activation transcriptionnelle, et l'élément de réponse contenant l'élément de reconnaissance cognat propre au domaine de liaison à l'ADN, et
le récepteur synthétique étant :
(a) un récepteur de protéolyse intramembranaire synthétique (SNIPR), tel que le récepteur synthétique est un récepteur synthétique de la famille des RIP, le récepteur étant éventuellement :
(i) un récepteur Notch synthétique,
(ii) un récepteur Robo synthétique, ou
(iii) un récepteur RoboNotch synthétique,
(b) un récepteur Tango, ou
(c) un système d'architecture de signalisation extracellulaire modulaire (MESA).

2. Cellule selon la revendication 1, ladite cellule étant :
(a) une cellule lymphoïde, telle que la cellule est une cellule tueuse naturelle, une cellule T, une cellule B ou une cellule dendritique plasmacytoïde (pDC), ou
(b) une cellule myéloïde, telle que la cellule est un macrophage.

3. Cellule selon la revendication 1 ou 2, dans laquelle :
(a) le domaine de liaison à l'ADN est choisi dans le groupe constitué par : Gal4, Pax6, les doigts de zinc (ZF), les doigts de zinc synthétiques (synZF), les effecteurs de type activateur de transcription (TALE), TetR, HNF1 alpha et vHNF1 bêta, et/ou
(b) le domaine d'activation transcriptionnelle est choisi dans le groupe constitué par : VP64, VP16, WWTR1, CREB3, NF-κB, p65, Rta, HSF1 et RelA, et/ou
(c) le domaine de signalisation intracellulaire est choisi dans le groupe constitué par : Gal4-VP64, Gal4-VP16, TetR-VP64, LacI-VP64, HNF1-WWTR1, HNF1-CREB3, Pax6-p65, ZF-p65, synZF-p65 et HNF1-p65.

4. Cellule selon l'une quelconque des revendications précédentes, dans laquelle le domaine de reconnaissance d'antigène extracellulaire est un domaine dérivé d'un anticorps, tel que le domaine de reconnaissance d'antigène extracellulaire est un domaine scFv.

5. Cellule selon l'une quelconque des revendications précédentes, dans laquelle la protéine STING comprend :
(a) une mutation de gain de fonction qui l'amène à être constitutivement active en l'absence de 2',3'-cGAMP, et/ou
(b) une mutation, de préférence une substitution, au niveau d'un ou plusieurs résidus correspondant à R71, S102, V147, N154, V155, G166, C206, G207, G230, H232, R238, F279, R281, R284, R293 ou Q315 de la SEQ ID NO : 1.

6. Cellule selon l'une quelconque des revendications précédentes, ladite cellule comprenant un acide nucléique hétérologue codant pour le récepteur synthétique, ladite cellule comprenant éventuellement un seul acide nucléique hétérologue codant pour le récepteur synthétique et l'élément de réponse.

7. Cellule selon l'une quelconque des revendications 2 à 6, dans laquelle la pDC a subi une étape d'amorçage de la pDC, l'étape d'amorçage de la pDC comprenant éventuellement l'incubation des cellules avec de l'IFN de type I et/ou de l'IFN de type II, telle que la pDC est incubée avec de l'IFN de type I et/ou de l'IFN de type II pendant 24 heures.

8. Cellule selon l'une quelconque des revendications précédentes, dans laquelle la protéine STING active une réponse immunitaire telle que :
(a) la réponse immunitaire comprend la sécrétion de cytokines, lesdites cytokines modulant éventuellement l'environnement du tissu malade, ou la réponse immunitaire comprend le recrutement ou l'activation de cellules immunitaires, les cytokines étant des cytokines pro-inflammatoires, les cytokines comprenant éventuellement CXCL10, MCP-1, MCP-2, MIP-1a et/ou MIP-1b, et/ou
(b) la réponse immunitaire comprend la production d'IFN de type I.

9. Cellule selon l'une quelconque des revendications précédentes, destinée à être utilisée dans une méthode de traitement d'une maladie chez un sujet, ladite maladie étant :
(a) un cancer,
(b) une maladie auto-immune,
(c) une maladie inflammatoire, ou
(d) une maladie infectieuse.

10. Cellule destinée à être utilisée selon la revendication 9a, et pour laquelle :
(a) le cancer est un cancer solide, ledit cancer étant choisi dans le groupe constitué par : le cancer des os, le cancer du sein, le cancer du pancréas, le cancer de la peau, le cancer de la tête ou du cou, le mélanome malin cutané ou intraoculaire, le cancer de l'utérus, le cancer de l'ovaire, le cancer de la prostate, le cancer du rectum, le cancer de la région anale, le cancer du côlon, le cancer de l'estomac, le cancer des testicules, le cancer de l'utérus, le carcinome des trompes de Fallope, le carcinome de l'endomètre, le carcinome du col de l'utérus, le carcinome du vagin, le carcinome de la vulve, le cancer de l'œsophage, le cancer de l'intestin grêle, le cancer du système endocrinien, le cancer de la glande thyroïde, le cancer de la glande parathyroïde, le cancer de la glande surrénale, le sarcome des tissus mous, le cancer de l'urètre, le cancer du pénis, les tumeurs pédiatriques, le cancer de la vessie, le cancer du rein ou de l'uretère, le carcinome du bassinet rénal, le néoplasme du système nerveux central (SNC), le lymphome primitif du SNC, l'angiogenèse tumorale, la tumeur de l'axe spinal, le gliome du tronc cérébral, le glioblastome, l'adénome hypophysaire, le sarcome de Kaposi, le cancer épidermoïde et le carcinome épidermoïde, ou
(b) le cancer est choisi dans le groupe constitué par : la leucémie lymphoblastique aiguë (LLA) (y compris la LLA sans cellules T), la leucémie myéloïde aiguë, la leucémie prolymphocytaire à cellules B, la leucémie lymphoïde aiguë à cellules B (« LLA-B »), le néoplasme à cellules dendritiques plasmacytoïdes blastiques, le lymphome de Burkitt, la leucémie lymphoïde chronique (LLC), la leucémie myélogène chronique (LMC), la leucémie myéloïde chronique, la leucémie chronique ou aiguë, le lymphome diffus à grandes cellules B (LDGCB), le lymphome folliculaire (LF), la leucémie à tricholeucocytes, la maladie de Hodgkin, les affections lymphoprolifératives malignes, le lymphome du MALT, le lymphome des cellules du manteau, le lymphome de la zone marginale, la gammapathie monoclonale de signification indéterminée (MGUS), le myélome multiple, la myélodysplasie et le syndrome myélodysplasique, le lymphome non hodgkinien (LNH), le trouble prolifératif des plasmocytes (y compris le myélome asymptomatique (myélome multiple latent ou myélome indolent)), le lymphome plasmablastique, le néoplasme à cellules dendritiques plasmacytoïdes, les plasmocytomes (y compris la dyscrasie plasmocytaire, le myélome solitaire, le plasmocytome solitaire, le plasmocytome extramédullaire, et le plasmocytome multiple), le syndrome POEMS (également appelé syndrome de Crow-Fukase, maladie de Takatsuki, et syndrome PEP), le lymphome médiastinal primitif à grandes cellules B (LMPB), le lymphome folliculaire à petites cellules ou à grandes cellules, le lymphome de la zone marginale splénique (LZMS), l'amylose systémique à chaînes légères amyloïdes, la leucémie aiguë lymphoïde à cellules T (« LAL-T »), le lymphome à cellules T, le lymphome folliculaire transformé et la macroglobulinémie de Waldenström.

11. Cellule, ou cellule destinée à être utilisée, selon l'une quelconque des revendications précédentes, dans laquelle l'antigène est un antigène associé à une tumeur, ledit antigène étant choisi parmi :
(a) le groupe constitué par : CD19, CD20, CD22, CD30, CD33, CD38, CD123, CD138, CS-1, l'antigène de maturation des lymphocytes B (BCMA), MAGEA3, MAGEA3/A6, KRAS, CLL1, MUC-1, HER2, EpCam, GD2, GPA7, PSCA, EGFR, EGFRvIII, ROR1, mésothéline, CD33/IL3RA, c-Met, CD37, PSMA, le glycolipide F77, GD-2, gp100, le TCR NY-ESO-1, FRalpha, CD24, CD44, CD133, CD166, CA-125, HE4, Ova1, le récepteur des œstrogènes, le récepteur de la progestérone, uPA, PAI-1, MICA, MICB, ULBP1, ULBP2, ULBP3, ULBP4, ULBP5 ou ULBP6, ou une combinaison de ceux-ci, ou
(b) le groupe constitué par 5T4, l'alpha-fœtoprotéine (AFP), B7-1 (CD80), B7-2 (CD86), BCMA, la gonadotrophine chorionique humaine bêta, CA-125, l'antigène carcino-embryonnaire (ACE), l'antigène carcino-embryonnaire (ACE), CD123, CD133, CD138, CD19, CD20, CD22, CD23, CD24, CD25, CD30, CD33, CD34, CD4, CD40, CD44, CD56, CD70, CD8, CLL-1, c-Met, l'antigène spécifique du CMV, CS-1, CSPG4, CTLA-4, DLL3, le disialoganglioside GD2, la mucine épithéliale ductale, l'antigène spécifique de l'EBV, le variant III de l'EGFR (EGFRvIII), ELF2M, l'endogline, l'éphrine B2, le récepteur du facteur de croissance épidermique (EGFR), la molécule d'adhésion des cellules épithéliales (EpCAM), l'antigène tumoral épithélial, l'ErbB2 (HER2/neu), la protéine associée aux fibroblastes (fap), FLT3, la protéine de liaison aux folates, GD2, GD3, l'antigène associé au gliome, les glycosphingolipides, gp36, l'antigène spécifique du VHB, l'antigène spécifique du VHC, HER1, HER2, HER2-HER3 en combinaison, HERV-K, l'antigène associé au mélanome de haut poids moléculaire (HMW-MAA), la glycoprotéine d'enveloppe gp41 du VIH-1, l'antigène spécifique du HPV, la transcriptase inverse de la télomérase humaine, le récepteur de l'IGF1, IGF-11, IL-11Ralpha, IL-13R-a2, l'antigène spécifique du virus de la grippe, CD38, le facteur de croissance insulinique (IGF1)-1, la carboxylestérase intestinale, la chaîne kappa, LAGA-1a, la chaîne lambda, l'antigène spécifique du virus de Lassa, l'AFP réactive aux lectines, l'antigène spécifique d'une lignée ou d'un tissu tel que CD3, MAGE, MAGE-A1, la molécule du complexe majeur d'histocompatibilité (CMH), la molécule du complexe majeur d'histocompatibilité (CMH) présentant un épitope peptidique spécifique d'une tumeur, M-CSF, l'antigène associé au mélanome, la mésothéline, MN-CA IX, MUC-1, mut hsp70-2, p53 muté, p53 muté, ras muté, l'élastase neutrophile, NKG2D, Nkp30, NY-ESO-1, p53, PAP, la prostase, l'antigène prostatique spécifique (PSA), l'antigène tumoral du carcinome de la prostate-1 (PCTA-1), la protéine antigénique prostatique spécifique, STEAP1, STEAP2, PSMA, RAGE-1, ROR1, RU1, RU2 (AS), la molécule d'adhésion de surface, la survivine, la télomérase, TAG-72, le domaine supplémentaire A (EDA) et le domaine supplémentaire B (EDB) de la fibronectine et le domaine A1 de la ténascine-C (TnC A1), la thyroglobuline, les antigènes stromaux tumoraux, le récepteur 2 du facteur de croissance endothélial vasculaire (VEGFR2), un antigène de surface spécifique d'un virus tel qu'un antigène spécifique du VIH, ou une combinaison de ceux-ci.

12. Cellule destinée à être utilisée selon la revendication 9b, la maladie auto-immune étant choisie dans le groupe constitué par : le diabète de type 1, la maladie auto-immune thyroïdienne, l'insuffisance surrénalienne d'Addison, l'oophorite, l'orchite, l'hypophysite lymphocytaire, l'hypoparathyroïdie auto-immune, l'hypoparathyroïdie auto-immune, la maladie de Goodpasture, la myocardite auto-immune, la néphropathie membraneuse, l'hépatite autoimmune, la rectocolite hémorragique, la maladie de Crohn, la sclérose en plaques, la myasthénie grave, la neuromyélite optique, l'encéphalite et le syndrome de Sjögren.

13. Cellule destinée à être utilisée selon la revendication 9c, la maladie inflammatoire étant choisie dans le groupe constitué par : la mucoviscidose, les maladies inflammatoires chroniques de l'intestin telles que, par exemple, la rectocolite hémorragique ou la maladie de Crohn, la vascularite, en particulier la maladie de Kawasaki, la bronchite chronique, les maladies arthritiques inflammatoires telles que, par exemple, l'arthrite psoriasique, l'arthrose, la polyarthrite rhumatoïde, et l'arthrite rhumatoïde juvénile à début systémique (maladie de Still), la maladie du greffon contre l'hôte, l'asthme, le psoriasis, le lupus érythémateux disséminé, l'obésité et les maladies vasculaires inflammatoires ainsi que le rejet d'allogreffe.

14. Cellule selon l'une quelconque des revendications 1 à 8 ou cellule destinée à être utilisée selon l'une quelconque des revendications 9b, 9c, 12 et 13, dans laquelle l'antigène est exprimé à la surface des cellules immunitaires qui provoquent une maladie auto-immune ou inflammatoire.

15. Cellule ou cellule destinée à être utilisée selon la revendication 9b ou 9c, la maladie étant le rejet de greffe ou la maladie du greffon contre l'hôte (GVHD).

16. Cellule destinée à être utilisée selon la revendication 9d, ladite maladie infectieuse étant une infection virale ou fongique chronique, telle qu'une infection par le virus de la grippe, le virus de la fièvre jaune, le virus du Nil occidental, l'hantavirus, le virus Ebola, le rotavirus, le norovirus, le virus de la rage, le virus de l'encéphalite transmise par les tiques (TBEV), les rhinovirus, le coronavirus, le VRS, la rougeole, le virus paragrippal, le virus Zika, le virus de la dengue, le VIH, le VHB, le VHC, le cytomégalovirus humain (CMV), le virus d'Epstein-Barr (EBV), *Aspergillus spp.*, tel que *Aspergillus fumigatus*, *Candida spp*., tel que *C. albicans*, *Mucorales*, *Cryptococcus spp*., tel que *Cryptococcus neoformans* ou *Cryptococcus gattii*, ou *Pneumocystis jirovecii*.

17. Cellule selon l'une quelconque des revendications 1 à 8 ou cellule destinée à être utilisée selon la revendication 9d ou 16, dans laquelle l'antigène est un antigène pathogène, ledit antigène étant choisi dans le groupe constitué par : la protéine NS1 du virus de la fièvre jaune, la protéine NS1 du virus du Nil occidental, la protéine N de l'hantavirus, la protéine N du virus Ebola, le rotavirus VP6, le norovirus VP1, la protéine N du virus de la rage, la glycoprotéine d'enveloppe du TBEV, les protéines VP du rhinovirus, les antigènes hémagglutinines de la grippe, les antigènes neuraminidases de la grippe, la protéine de spicule du coronavirus, la protéine F du VRS, la protéine N du MeV, les antigènes hémagglutinines de *parainfluenza*, les antigènes neuraminidases de *parainfluenza*, les protéines E, NS1, NS3, NS4B et NS5 du virus ZIKV, la protéine C du virus de la dengue, la protéine M, la protéine E, la protéine NS1, gp120, gp41, Env, l'antigène de surface du VHB, les protéines de surface S et L du VHB, la glycoprotéine E2 du VHC, la glycoprotéine B du CMV et le bêta-glucane fongique.

18. Cellule, ou cellule destinée à être utilisée, selon l'une quelconque des revendications précédentes, ladite cellule ayant été extraite du sang ou de la moelle osseuse, ou ladite cellule étant une pDC et ladite cellule ayant été différenciée in vitro à partir de CSPH, les CSPH ayant éventuellement été obtenues à partir du sang ou de la moelle osseuse, ou les CSPH ayant été différenciées in vitro à partir de cellules souches pluripotentes induites (CSPi) ou de cellules souches embryonnaires (CSE).

19. Composition pharmaceutique comprenant la cellule immunitaire modifiée selon l'une quelconque des revendications précédentes et un vecteur pharmaceutiquement acceptable.

20. Procédé de production de la cellule ou de la cellule destinée à être utilisée selon l'une quelconque des revendications 2a ou 3 à 19, comprenant les étapes suivantes :
(a)
(i) la transfection ou la transduction des CSPH avec un ou plusieurs vecteurs codant pour le récepteur synthétique et l'élément de réponse, et
(ii) la différenciation des CSPH en pDC, ou
(b)
(i) la différenciation des CSPH en pDC, et
(ii) la transfection ou la transduction des pDC avec un ou plusieurs vecteurs codant pour le récepteur synthétique et l'élément de réponse,
étant entendu éventuellement que :
(A) le vecteur est un vecteur viral, le vecteur étant éventuellement un vecteur lentiviral ou un vecteur adénoviral ou adéno-associé, ou
(B) le vecteur est un ARNm, éventuellement encapsulé dans une nanoparticule lipidique.

21. Procédé selon la revendication 20, dans lequel :
(a) l'étape de différenciation des CSPH comprend l'incubation desdites CSPH dans un ou plusieurs milieux, le ou lesdits milieux pouvant typiquement comprendre une ou plusieurs cytokines, facteurs de croissance, interférons (IFN) et/ou antagonistes du récepteur des hydrocarbures aryliques (AHR) (tels que le stemregenin-1), moyennant quoi lesdites CSPH sont différenciées en précurseurs de pDC et en pDC, et/ou
(b) le procédé comprend en outre une étape de purification des pDC, et/ou
(c) le procédé comprend en outre une étape de formulation des pDC avec un excipient pharmaceutiquement acceptable.
